# EUROPEAN PATENT APPLICATION

(11) **EP 2 586 453 A1**
(43) Date of publication of application: **01.05.2013**
(21) Application number: 11798296.7
(22) Date of filing: 24.06.2011
(51) Int. Cl.: A61K 38/00, A61K 31/7105, A61K 31/713, A61K 48/00, A61P 35/00, A61P 43/00

(54) **TUMOR GROWTH CONTROLLING METHOD TARGETING GALACTOSYLCERAMIDE EXPRESSION FACTOR-1**

(30) Priority: 25.06.2010 JP 2010144763
(71) Applicant: Tokyo Metropolitan Institute of Medical Science, Tokyo 156-8506 (JP)
(72) Inventor: OGURA, Kiyoshi, Tokyo 156-8506 (JP)
(74) Representative: Woods, Geoffrey Corlett
(86) International application number: PCT/JP2011/065139
(87) International publication number: WO 2011/162419

(57) **Abstract**

The object of the present invention is to provide an anti-tumor agent having an anti-angiogenic effect and/or an anti-tumor growth effect or a method for tumor growth inhibition.

The present invention provides an anti-tumor agent characterized by having an anti-angiogenic effect and/or an anti-tumor growth effect, which contains a polypeptide of galactosylceramide expression factor-1 excluding the Q region, wherein the polypeptide comprises at least the C region or a part thereof.

## Description

### TECHNICAL FIELD

The present invention relates to an anti-tumor agent and a method for tumor growth inhibition, each of which targets galactosylceramide expression factor-1.

### BACKGROUND ART

Galactosylceramide (GalCer) is the smallest glycosphingolipid composed of one galactose residue and one ceramide residue, which is highly expressed specifically in myelin oligodendrocytes and/or renal epithelial cells and has an insulating function and a high salt concentration-resistant function.

Galactosylceramide expression factor-1 (GalCer expression factor-1 (GEF-1)), which is an expression factor of galactosylceramide (Ogura, K., et al., J. Neurochem., 71, 1827-1836, 1998), is a protein composed of 4 regions, i.e., a zinc-finger sequence (FYVE)-containing region (Z), a proline-rich region (P), a coiled-coil sequence region (C), and a proline 2-rich/glutamine-rich region (Q). Moreover, rat GEF-1 is a rat ortholog of mouse Hrs (HGF-regulated tyrosine kinase substrate) (Komada, M., et al., Mol. Cell Biol., 15, 6213-6221, 1995) and is known to be involved in vesicular transport and intracellular signaling.

The inventors of the present invention have already found that GEF-1/Hrs induces fibroblast-like morphological changes in MDCK cells, and have further found that GEF-1/Hrs is involved in the migration capacity of cells (JP 2005-247735 A). However, GEF-1/Hrs and its Q region-deficient mutants showed no anti-tumor growth effect (JP 2005-247735 A).

On the other hand, tumors are known to release angiogenic factors (e.g., VEGF and TGF-β) and thereby induce angiogenesis therein to compensate for low oxygen and low nutrients during their growth process. Thus, a strategy to inhibit this tumor-induced angiogenesis has been designed for inhibition of tumor growth. However, the relationship between GEF-1/Hrs and angiogenesis has not been known so far.

### DISCLOSURE OF THE INVENTION

The present invention has been made under these circumstances, and the problem to be solved by the present invention is to provide an anti-tumor agent having an anti-angiogenic effect and/or an anti-tumor growth effect or a method for tumor growth inhibition.

As a result of extensive and intensive efforts made to solve the above problem, the inventors of the present invention have found that GEF-1 is involved in angiogenesis and tumor growth. Moreover, the inventors of the present invention have also found that inhibition of GEF-1 expression allows inhibition of angiogenesis and tumor growth. These findings led to the completion of the present invention.

Namely, the present invention is as follows.
(1) A tumor growth inhibitor, which contains a polypeptide of galactosylceramide expression factor-1 excluding the Q region, wherein the polypeptide comprises at least the C region or a part thereof.
(2) A tumor growth inhibitor, which comprises a polypeptide shown in (a) or (b) below:
   (a) a polypeptide which consists of the amino acid sequence shown in SEQ ID NO: 8, 10 or 12; or
   (b) a polypeptide which consists of an amino acid sequence with deletion, substitution or addition of one or several amino acids in the amino acid sequence shown in SEQ ID NO: 8, 10 or 12 and which has an anti-tumor growth effect.
(3) A tumor growth inhibitor, which comprises a polypeptide shown in (a) or (b) below:
   (a) a polypeptide which comprises at least 10 consecutive amino acid residues in the amino acid sequence shown in SEQ ID NO: 8, 10 or 12; or
   (b) a polypeptide which consists of an amino acid sequence with deletion, substitution or addition of one or several amino acids in an amino acid sequence comprising at least 10 consecutive amino acid residues in the amino acid sequence shown in SEQ ID NO: 8, 10 or 12 and which has an anti-tumor growth effect.
(4) The tumor growth inhibitor according to (3) above, wherein the polypeptide shown in (a) comprises an amino acid sequence shown in any of SEQ ID NOs: 44 to 65.
(5) A tumor growth inhibitor, which contains a polynucleotide encoding a polypeptide of galactosylceramide expression factor-1 excluding the Q region, wherein the polypeptide comprises at least the C region or a part thereof.
(6) A tumor growth inhibitor, which comprises a polynucleotide encoding a polypeptide shown in (a) or (b) below:
   (a) a polypeptide which consists of the amino acid sequence shown in SEQ ID NO: 8, 10 or 12; or
   (b) a polypeptide which consists of an amino acid sequence with deletion, substitution or addition of one or several amino acids in the amino acid sequence shown in SEQ ID NO: 8, 10 or 12 and which has an anti-tumor growth effect.
(7) A tumor growth inhibitor, which comprises a polynucleotide encoding a polypeptide shown in (a) or (b) below:
   (a) a polypeptide which comprises at least 10 consecutive amino acid residues in the amino acid sequence shown in SEQ ID NO: 8, 10 or 12; or
   (b) a polypeptide which consists of an amino acid sequence with deletion, substitution or addition of one or several amino acids in an amino acid sequence comprising at least 10 consecutive amino acid residues in the amino acid sequence shown in SEQ ID NO: 8, 10 or 12 and which has an anti-tumor growth effect.
(8) A tumor growth inhibitor, which comprises a polynucleotide shown in (a) or (b) below:
   (a) a polynucleotide which consists of the nucleotide sequence shown in SEQ ID NO: 7, 9 or 11; or
   (b) a polynucleotide which is hybridizable under stringent conditions with a polynucleotide consisting of a nucleotide sequence complementary to the nucleotide sequence shown in SEQ ID NO: 7, 9 or 11 and which encodes a polypeptide having an anti-tumor growth effect.
(9) A tumor growth inhibitor, which contains an expression inhibitor of galactosylceramide expression factor-1.
(10) The tumor growth inhibitor according to (9) above, wherein the expression inhibitor of galactosylceramide expression factor-1 is siRNA or shRNA against a polynucleotide encoding galactosylceramide expression factor-1.
(11) The tumor growth inhibitor according to (10) above, wherein the polynucleotide encoding galactosylceramide expression factor-1 comprises a nucleotide sequence encoding a polypeptide shown in (a) or (b) below:
   (a) a polypeptide which consists of the amino acid sequence shown in SEQ ID NO: 2, 4 or 6; or
   (b) a polypeptide which consists of an amino acid sequence with deletion, substitution or addition of one or several amino acids in the amino acid sequence shown in SEQ ID NO: 2, 4 or 6 and which has a tumor growth effect.
(12) The tumor growth inhibitor according to (10) above, wherein the polynucleotide encoding galactosylceramide expression factor-1 comprises a polynucleotide shown in (a) or (b) below:
   (a) a polynucleotide which consists of the nucleotide sequence shown in SEQ ID NO: 1, 3 or 5; or
   (b) a polynucleotide which is hybridizable under stringent conditions with a polynucleotide consisting of a nucleotide sequence complementary to the nucleotide sequence shown in SEQ ID NO: 1, 3 or 5 and which encodes a polypeptide having a tumor growth effect.
(13) An angiogenesis inhibitor, which contains a polypeptide of galactosylceramide expression factor-1 excluding the Q region, wherein the polypeptide comprises at least the C region or a part thereof.
(14) An angiogenesis inhibitor, which comprises a polypeptide shown in (a) or (b) below:
   (a) a polypeptide which consists of the amino acid sequence shown in SEQ ID NO: 8, 10 or 12; or
   (b) a polypeptide which consists of an amino acid sequence with deletion, substitution or addition of one or several amino acids in the amino acid sequence shown in SEQ ID NO: 8, 10 or 12 and which has an anti-angiogenic effect.
(15) An angiogenesis inhibitor, which comprises a polypeptide shown in (a) or (b) below:
   (a) a polypeptide which comprises at least 10 consecutive amino acid residues in the amino acid sequence shown in SEQ ID NO: 8, 10 or 12; or
   (b) a polypeptide which consists of an amino acid sequence with deletion, substitution or addition of one or several amino acids in an amino acid sequence comprising at least 10 consecutive amino acid residues in the amino acid sequence shown in SEQ ID NO: 8, 10 or 12 and which has an anti-angiogenic effect.
(16) The angiogenesis inhibitor according to (15) above, wherein the polypeptide shown in (a) comprises an amino acid sequence shown in any of SEQ ID NOs: 44 to 65.
(17) An angiogenesis inhibitor, which contains a polynucleotide encoding a polypeptide of galactosylceramide expression factor-1 excluding the Q region, wherein the polypeptide comprises at least the C region or a part thereof.
(18) An angiogenesis inhibitor, which comprises a polynucleotide encoding a polypeptide shown in (a) or (b) below:
   (a) a polypeptide which consists of the amino acid sequence shown in SEQ ID NO: 8, 10 or 12; or
   (b) a polypeptide which consists of an amino acid sequence with deletion, substitution or addition of one or several amino acids in the amino acid sequence shown in SEQ ID NO: 8, 10 or 12 and which has an anti-angiogenic effect.
(19) An angiogenesis inhibitor, which comprises a polynucleotide encoding a polypeptide shown in (a) or (b) below:
   (a) a polypeptide which comprises at least 10 consecutive amino acid residues in the amino acid sequence shown in SEQ ID NO: 8, 10 or 12; or
   (b) a polypeptide which consists of an amino acid sequence with deletion, substitution or addition of one or several amino acids in an amino acid sequence comprising at least 10 consecutive amino acid residues in the amino acid sequence shown in SEQ ID NO: 8, 10 or 12 and which has an anti-angiogenic effect.
(20) An angiogenesis inhibitor, which comprises a polynucleotide shown in (a) or (b) below:
   (a) a polynucleotide which consists of the nucleotide sequence shown in SEQ ID NO: 7, 9 or 11; or
   (b) a polynucleotide which is hybridizable under stringent conditions with a polynucleotide consisting of a nucleotide sequence complementary to the nucleotide sequence shown in SEQ ID NO: 7, 9 or 11 and which encodes a polypeptide having an anti-angiogenic effect.
(21) An angiogenesis inhibitor, which contains an expression inhibitor of galactosylceramide expression factor-1.
(22) The angiogenesis inhibitor according to (21) above, wherein the expression inhibitor of galactosylceramide expression factor-1 is siRNA or shRNA against a polynucleotide encoding galactosylceramide expression factor-1.
(23) The angiogenesis inhibitor according to (22) above, wherein the polynucleotide encoding galactosylceramide expression factor-1 comprises a nucleotide sequence encoding a polypeptide shown in (a) or (b) below:
   (a) a polypeptide which consists of the amino acid sequence shown in SEQ ID NO: 2, 4 or 6; or
   (b) a polypeptide which consists of an amino acid sequence with deletion, substitution or addition of one or several amino acids in the amino acid sequence shown in SEQ ID NO: 2, 4 or 6 and which has an angiogenic effect.
(24) The angiogenesis inhibitor according to (22) above, wherein the polynucleotide encoding galactosylceramide expression factor-1 comprises a polynucleotide shown in (a) or (b) below:
   (a) a polynucleotide which consists of the nucleotide sequence shown in SEQ ID NO: 1, 3 or 5; or
   (b) a polynucleotide which is hybridizable under stringent conditions with a polynucleotide consisting of a nucleotide sequence complementary to the nucleotide sequence shown in SEQ ID NO: 1, 3 or 5 and which encodes a polypeptide having an angiogenic effect.
(25) An eliminator of cancer cell characteristics, which comprises a polypeptide shown in (a) or (b) below:
   (a) a polypeptide which consists of the amino acid sequence shown in SEQ ID NO: 8, 10 or 12; or
   (b) a polypeptide which consists of an amino acid sequence with deletion, substitution or addition of one or several amino acids in the amino acid sequence shown in SEQ ID NO: 8, 10 or 12 and which has an elimination effect on cancer cell characteristics.
(26) The eliminator of cancer cell characteristics according to (25) above, which allows cancer cells to acquire the ability of contact inhibition.
(27) A method for tumor growth inhibition, which comprises inhibiting the expression of galactosylceramide expression factor-1.
(28) A method for angiogenesis inhibition, which comprises inhibiting the expression of galactosylceramide expression factor-1.

The present invention enables the provision of an anti-tumor agent which contains a polypeptide of galactosylceramide expression factor-1 excluding the Q region, wherein the polypeptide comprises at least the C region or a part thereof. The present invention also enables the provision of an anti-tumor agent which contains an expression inhibitor of galactosylceramide expression factor-1. The present invention is very useful as an anti-tumor agent because of having not only an anti-metastatic effect on cancer, but also an anti-angiogenic effect and/or an anti-tumor growth effect.

### BRIEF DESCRIPTION OF THE DRAWINGS

Figure 1 is a schematic view showing the structure of GEF-1.
Figure 2A shows the colony morphology of B16 cells, B16/bsr cells, B16/GEF-1 cells, B16/C cells and B16/shRNA cells.
Figure 2B shows images of lung metastasis at 21 days after administration of B16 cells, B16/GEF-1 cells, B16/C cells and B16/shRNA cells. In the figure, "HE" denotes HE-stained images.
Figure 2C shows the longevity (survival days) of mice after administration of B 16 cells, B 1 6/bsr cells, B16/GEF-1 cells, B16/C cells and B16/shRNA cells.
Figure 3 shows the results of tube formation test. A) Results of tube formation obtained for KOP cells and KOP/C cells. B) Results of tube formation obtained for MDCK/GEF-1 cells, MDCK cells and MDCK/C cells.
Figure 4 shows the results of angiogenesis induction and inhibition under the dorsal skin of mice (*in vivo).* The upper panel shows the condition of angiogenesis, while the lower panel shows the results measured for vascular length and vascular area.
Figure 5 shows the results measured for *in vitro* growth ability of B16/C cells.
Figure 6 shows the results measured for cell density and cell cycle of B16/C cells.
Figure 7 is a schematic view showing the structure of vectors used for measurement of transcription factor activity and promoter activity.
Figure 8 shows the results measured for effects of HGF and TGF-β on SMAD transcriptional activity in MDCK/GEF-1 cells and MDCK/C cells.
Figure 9 shows the results measured for activity of various transcription factors in MDCK/GEF-1 cells and MDCK/C cells.
Figure 10 shows the results measured for activity of various transcription factors in B16/GEF-1 cells, B16/shRNA cells and B16/C cells.
Figure 11 shows the results measured for TGF-β-SMAD signal-induced PAI-1 promoter activity in B16/bsr cells, B16/GEF-1 cells and B16/C cells.
Figure 12 shows the results measured for changes in TGF-β levels contained in the medium.
Figure 13A shows the results measured for growth ability of B16/GEF-1 cells and B16/C cells in soft agar medium. The upper panel shows the condition of colony formation, while the lower panel shows the results measured for relative values of colony counts.
Figure 13B shows the results measured for growth ability of LLC/C cells in soft agar medium. The upper panel shows the condition of colony formation, while the lower panel shows the results measured for relative values of colony counts.
Figure 13C shows the growth morphology of MDCK cells and MDCK/GEF-1 cells in soft agar medium.
Figure 14A shows the results measured for tumorigenicity/tumor growth ability of B16/GEF-1 cells, B16/shRNA cells and B16/C cells in C57BL/6 mice.
Figure 14B shows the results measured for tumorigenicity/tumor growth ability of MDCK cells and MDCK/GEF-1 cells in nude mice.
Figure 15 shows the results tested for inhibitory effects of GEF-1 siRNAs on tube formation. The upper panel shows the condition of tube formation in siRNA-transfected cells, while the lower panel shows the results measured for relative values of % tube formation.
Figure 16 shows the results tested for *in vivo* anti-tumor growth effect of GEF-1 siRNA #3. The left panel shows the results measured for tumor volume and tumor weight, while the right panel shows the appearance of tumors formed in nude mice.
Figure 17 is a schematic view of GEF-1/C constituent oligopeptides.
Figure 18A shows the results measured for effects of GEF-1/C constituent oligopeptides on SMAD transcriptional activity in B16 cells.
Figure 18B shows the results measured for effects of GEF-1/C constituent oligopeptides on SMAD transcriptional activity in COLO205 cells.
Figure 19 shows the results measured for effects of GEF-1/C constituent oligopeptides on tube formation in KOP cells.
Figure 20A shows the results measured for effects of GEF-1/C constituent oligopeptides on B16 cell growth in soft agar medium.
Figure 20B shows the results measured for effects of GEF-1/C constituent oligopeptides on HeLa cell growth in soft agar medium.
Figure 20C shows the results measured for effects of GEF-1/C constituent oligopeptides on COLO205 cell growth in soft agar medium.
Figure 20D shows the results measured for effects of GEF-1/C constituent oligopeptides on KATOIII cell growth in soft agar medium.
Figure 21A shows the results measured for effects of GEF-1/C constituent oligopeptides on A549 cell growth in soft agar medium.
Figure 21B shows the results measured for effects of GEF-1/C constituent oligopeptides on PT45 cell growth in soft agar medium.
Figure 22 shows the results tested for *in vivo* anti-tumor growth effects of GEF-1/C constituent oligopeptides using B16 cells. The left panel shows the results measured for tumor volume and tumor weight, while the right panel shows the appearance of tumors formed in nude mice.
Figure 23 shows the results tested for *in vivo* anti-tumor growth effects of GEF-1/C constituent oligopeptides using COLO205 cells. The left panel shows the results measured for tumor volume and tumor weight, while the right panel shows the appearance of tumors formed in nude mice.
Figure 24 shows the results tested for *in* vivo anti-tumor growth effects of GEF-1/C constituent oligopeptides using A549 cells. The left panel shows the results measured for tumor volume and tumor weight, while the right panel shows the appearance of tumors formed in nude mice.
Figure 25 shows the results measured for *in vitro* growth ability of EL4/C cells.
Figure 26 shows the results measured for growth ability of PT45/C cells in soft agar medium. The upper panel shows the condition of colony formation, while the lower panel shows the results measured for relative values of colony counts.
Figure 27 shows the results measured for growth ability of COLO205/C cells in soft agar medium. The upper panel shows the condition of colony formation, while the lower panel shows the results measured for relative values of colony counts.
Figure 28 shows the results tested for *in vivo* anti-tumor growth effect of GEF-1/C constituent oligopeptide (r9-OP10-11) linked to a cell membrane permeable peptide using COLO205 cells. The left panel shows the results measured for changes in tumor volume and wet tumor weight, while the right panel shows the appearance of tumors formed in nude mice.

### BEST MODES FOR CARRYING OUT THE INVENTION

The present invention will be described in more detail below. The following embodiments are illustrated to describe the present invention, and it is not intended to limit the present invention only to these embodiments. The present invention can be implemented in various modes without departing from the spirit of the present invention. Moreover, this specification incorporates the contents disclosed in the specification and drawings of Japanese Patent Application No. 2010-144763 (filed on June 25, 2010), based on which the present application claims priority.

### 1. Summary

Galactosylceramide expression factor-1 (GalCer expression factor-1 (GEF-1)) is a factor capable of inducing galactosylceramide expression (Ogura, K., et al., J. Neurochem., 71, 1827-1836, 1998). The inventors of the present invention have already succeeded in inhibiting the metastatic ability of cancer cells by causing the cancer cells to express a polypeptide which comprises the C region of GEF-1 and is free from the Q region (JP 2005-247735 A). On the other hand, this polypeptide was found not to significantly inhibit tumor growth (ibid).

However, the inventors of the present invention have studied again the effect of GEF-1 on tumor growth, and hence have found that GEF-1 enhances tumor growth. The inventors of the present invention have further studied the effect of GEF-1 on angiogenesis, and hence have found that GEF-1 also enhances angiogenesis. Based on these findings, the C region of GEF-1 was then studied for its effects. As a result, it has been found that tumor growth and angiogenesis can be inhibited when the C region of GEF-1 or a constituent oligopeptide thereof is allowed to express itself. Moreover, it has been found that tumor growth and angiogenesis can also be inhibited when GEF-1 expression is inhibited by using a GEF-1 expression inhibitor. These results have indicated that a polypeptide covering the C region of GEF-1, a constituent oligopeptide thereof and a GEF-1 expression inhibitor have all of the three effects, i.e., cancer cell metastasis inhibition, angiogenesis inhibition and tumor growth inhibition, and are very useful as anti-tumor agents. Moreover, it has also been indicated that the anti-tumor agent of the present invention not only indirectly inhibits metastasis of cancer cells and tumor growth through inhibition of tumor-induced angiogenesis, but also directly inhibits cancer metastasis through inhibition of the migration capacity of cancer cells per se, and further directly inhibits tumor growth through inhibition of the expression of transcription factors involved in tumor growth of cancer cells. Thus, the anti-tumor agent of the present invention can exert the above three effects in a synergistic manner and hence is very useful for cancer or tumor treatment.

### 2. Galactosylceramide expression factor-1

### (1) Functions of galactosylceramide expression factor-1

GEF-1 has the function of inducing high level expression of GalCer and its sulfated derivative sulfatide in MDCK cells derived from epithelial cells. In addition, GEF-1 has the function of inducing epithelial-mesenchymal transition (EMT) in MDCK cells to convert the MDCK cells into a fibroblast-like morphology. Epithelial-mesenchymal transition (EMT) is an essential process in the early stage of embryonic morphogenesis induced by signals such as Wnt, and is also greatly involved in invasion and intravascular migration during cancer cell metastasis (Bean, A., et al., Nature, 385, 826-829, 1997). In fact, GEF-1 has been shown to promote metastasis of cancer cells when expressed in mouse melanoma B16 cells, which are cancer cells. Moreover, GEF-1 also has an angiogenic effect and a tumor growth effect.

### (2) GEF-1 polypeptide

The GEF-1 polypeptide to be used in the present invention is not limited in any way and may be of rat, human or mouse origin. Rat GEF-1 is a homolog of mouse or human HGF-regulated tyrosine kinase substrate (hereinafter referred to as "Hrs" or "Hgs"). Amino acid sequence homologies between Rat GEF-1 and mouse Hrs, between rat GEF-1 and human Hrs, and between mouse Hrs and human Hrs are as follows:
(a) rat GEF-1 and mouse Hrs have an amino acid sequence homology of 97%;
(b) rat GEF-1 and human Hrs have an amino acid sequence homology of 93%; and
(c) mouse Hrs and human Hrs have an amino acid sequence homology of 93%.

Thus, the GEF-1 polypeptide to be used in the present invention encompasses a GEF-1 polypeptide of rat origin, an Hrs polypeptide of human origin, and an Hrs polypeptide of mouse origin.

The amino acid sequences of GEF-1 (Hrs) polypeptides of rat, human and mouse origin are shown in SEQ ID NOs: 2, 4 and 6, respectively. Likewise, the nucleotide sequences of polynucleotides encoding GEF-1 of rat, human and mouse origin are shown in SEQ ID NOs: 1, 3 and 5, respectively. The above nucleotide sequences and amino acid sequences are registered in GenBank. GenBank accession numbers of the respective GEF-1 (Hrs) nucleotide sequences and amino acid sequences are shown below.
Rat GEF-1 (SEQ ID NOs: 1 and 2): AB002811
Human Hrs (SEQ ID NOs: 3 and 4): U43895
Mouse Hrs (SEQ ID NOs: 5 and 6): D50050

The GEF-1 polypeptide to be used in the present invention encompasses not only the above polypeptide which consists of the amino acid sequence shown in SEQ ID NO: 2, 4 or 6, but also a polypeptide which consists of an amino acid sequence mutated by deletion, substitution or addition (or any combination thereof) of one or several amino acids in the amino acid sequence shown in SEQ ID NO: 2, 4 or 6 and which has an angiogenic effect or a tumor growth effect.

Examples of the above amino acid sequence mutated by deletion, substitution or addition (or any combination thereof) of one or several amino acids in the amino acid sequence shown in SEQ ID NO: 2, 4 or 6 include:
(i) an amino acid sequence with deletion of 1 to 10 (e.g., 1 to 5, preferably 1 to 3, more preferably 1 to 2, even more preferably 1) amino acids from the amino acid sequence shown in SEQ ID NO: 2, 4 or 6;
(ii) an amino acid sequence with substitution of other amino acids for 1 to 10 (e.g., 1 to 5, preferably 1 to 3, more preferably 1 to 2, even more preferably 1) amino acids in the amino acid sequence shown in SEQ ID NO: 2, 4 or 6;
(iii) an amino acid sequence with addition of 1 to 10 (e.g., 1 to 5, preferably 1 to 3, more preferably 1 to 2, even more preferably 1) amino acids to the amino acid sequence shown in SEQ ID NO: 2, 4 or 6; and
(iv) an amino acid sequence mutated by any combination of (i) to (iii) above.

In the context of the present invention, the term "angiogenic effect" is intended to mean the effect of inducing *in vitro* tube formation or the effect of inducing *in vivo* angiogenesis. Likewise, the term "tumor growth effect" is intended to mean the effect of promoting the growth of cancer cells.

Moreover, "having an angiogenic effect or a tumor growth effect" is intended to mean having 10% or more, 20% or more, 30% or more, 40% or more, 50% or more, 60% or more, 70% or more, 80% or more, preferably 90% or more activity, when compared to the angiogenic effect or tumor growth effect of a polypeptide having the amino acid sequence shown in SEQ ID NO: 2, 4 or 6, which is set to 100.

The angiogenic effect may be confirmed in a known manner. For example, MDCK cells are caused to express a mutated polypeptide and tested to examine whether their tube formation ability is enhanced or not, in comparison with control MDCK cells. Alternatively, the angiogenic effect may be measured by determining the vascular length or vascular area in an angiogenesis induction test conducted under the dorsal skin of mice (DAS assay). On the other hand, the tumor growth effect may be measured by causing cancer cells of any type to express a mutated polypeptide and measuring the amount of tumor growth using known procedures.

To prepare the above mutation-carrying polypeptides, mutations may be introduced into polynucleotides by using a kit for mutation introduction based on site-directed mutagenesis (e.g., Kunkel method or Gapped duplex method), as exemplified by a QuikChange™ Site-Directed Mutagenesis Kit (Stratagene), GeneTailor™ Site-Directed Mutagenesis Systems (Invitrogen), and TaKaRa Site-Directed Mutagenesis Systems (e.g., Mutan-K, Mutan-Super Express Km; Takara Bio Inc., Japan). Alternatively, it is also possible to use techniques for site-directed mutagenesis as described in "Molecular Cloning, A Laboratory Manual 2nd ed." (Cold Spring Harbor Press (1989)), "Current Protocols in Molecular Biology" (John Wiley & Sons (1987-1997)), Kunkel (1985) Proc. Natl. Acad. Sci. USA 82: 488-92, Kramer and Fritz (1987) Method. Enzymol. 154: 350-67, Kunkel (1988) Method. Enzymol. 85: 2763-6, etc.

Furthermore, the GEF-1 polypeptide to be used in the present invention encompasses not only those having the amino acid sequence shown in SEQ ID NO: 2, 4 or 6, but also those having amino acid sequences sharing a homology of about 85% or more, preferably about 90% or more, more preferably about 93% or more with the amino acid sequence shown in SEQ ID NO: 2, 4 or 6 and having an angiogenic effect or a tumor growth effect (i.e., amino acid sequences substantially equivalent to the amino acid sequence shown in SEQ ID NO: 2, 4 or 6). For homology determination, it is possible to use homology search tools such as FASTA, BLAST, PSI-BLAST and so on in a homology search site on the Internet (e.g., DNA Data Bank of Japan (DDBJ)). Alternatively, it is possible to conduct a BLAST search in the National Center for Biotechnology Information (NCBI).

### (3) Polynucleotide encoding GEF-1

As described above, rat GEF-1 is a homolog of mouse or human Hrs. Thus, the polynucleotide encoding GEF-1 of the present invention encompasses polynucleotides encoding Hrs, e.g., those encoding human and mouse Hrs (SEQ ID NOs: 3 and 5), as well as polynucleotides encoding human and mouse Hrs polypeptides consisting of the amino acid sequences shown in SEQ ID NOs: 4 and 6, respectively. Moreover, a polynucleotide encoding rat Hrs is also included.

In the present invention, the polynucleotide encoding GEF-1 is not limited in any way, as long as it is a polynucleotide comprising the nucleotide sequence shown in SEQ ID NO: 1, 3 or 5 or a nucleotide sequence encoding any of the above GEF-1 polypeptides. For example, in the present invention, it is possible to use not only a polynucleotide encoding a polypeptide which consists of the amino acid sequence shown in SEQ ID NO: 2, 4 or 6, but also a polynucleotide encoding a mutated polypeptide which consists of an amino acid sequence with deletion, insertion, substitution or addition of one or several amino acids in the amino acid sequence shown in SEQ ID NO: 2, 4 or 6 and which has an angiogenic effect or a tumor growth effect. As used herein, the term "polynucleotide" refers to a polymer composed of a plurality of bases or base pairs such as deoxyribonucleic acids (DNA) or ribonucleic acids (RNA), and encompasses DNA, cDNA, genomic DNA, chemically synthesized DNA and RNA. In addition, polynucleotides optionally containing unnatural artificial bases are also included.

The polynucleotide encoding GEF-1 of the present invention encompasses a polynucleotide which consists of the nucleotide sequence shown in SEQ ID NO: 1, 3 or 5, or a polynucleotide which is hybridizable under stringent conditions with a polynucleotide consisting of a sequence complementary to the nucleotide sequence and which encodes a polypeptide having an angiogenic effect or a tumor growth effect. Such a polynucleotide may be obtained from human cDNA and genomic libraries by known hybridization techniques (e.g., colony hybridization, plaque hybridization, Southern blotting) using the polynucleotide which consists of the nucleotide sequence shown in SEQ ID NO: 1, 3 or 5 or a fragment thereof as a probe. For preparation of cDNA libraries, reference may be made to "Molecular Cloning, A Laboratory Manual 2nd ed." (Cold Spring Harbor Press (1989)). Alternatively, commercially available cDNA and genomic libraries may also be used for this purpose.

Hybridization conditions in the present invention include, for example, "2 × SSC, 0.1% SDS, 25°C," "1 × SSC, 0.1% SDS, 25°C" or "0.2 × SSC, 0.1% SDS, 42°C" for stringent conditions, and "0.1 × SSC, 0.1% SDS, 68°C" for more stringent conditions. Those skilled in the art would be able to determine the conditions required for obtaining the polynucleotide encoding the GEF-1 gene of the present invention in consideration of not only these conditions including buffer salt concentration and temperature, but also other various conditions including probe concentration, probe length, reaction time and so on.

As to detailed procedures for hybridization, reference may be made to "Molecular Cloning, A Laboratory Manual 2nd ed." (Cold Spring Harbor Press (1989); particularly Section 9.47-9.58), "Current Protocols in Molecular Biology" (John Wiley & Sons (1987-1997); particularly Section 6.3-6.4), etc.

The nucleotide sequence of the polynucleotide of the present invention can be confirmed by conventional sequencing techniques. For example, dideoxynucleotide chain termination sequencing (Sanger et al. (1977) Proc. Natl. Acad. Sci. USA 74: 5463) may be used for this purpose. Alternatively, an appropriate DNA sequencer (e.g., ABI PRISM (Applied Biosystems)) may be used for sequence analysis.

### 3. C region and Q region of GEF-1

### (1) Functions of the C region and Q region of GEF-1

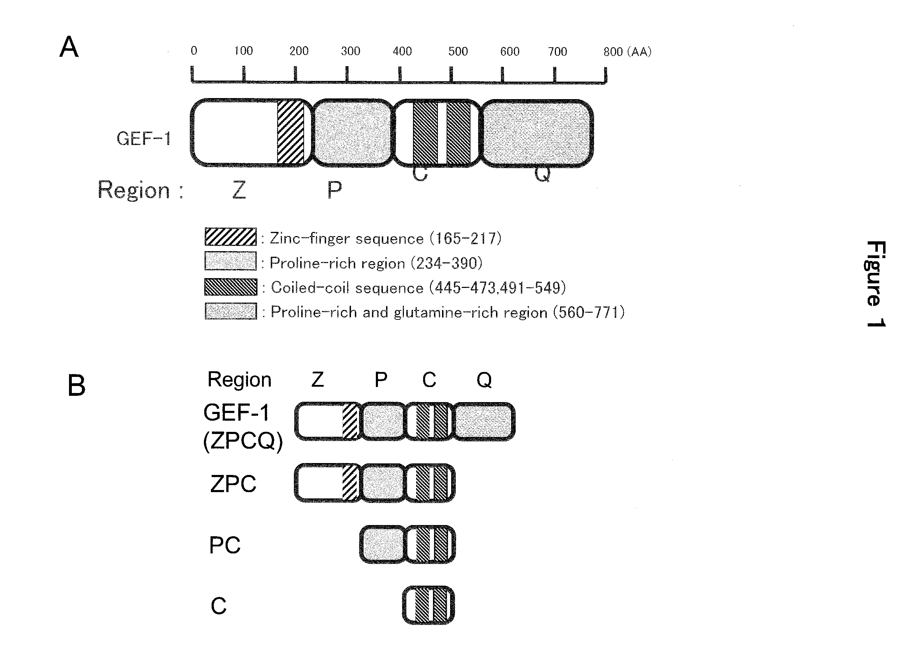

As shown in Figure 1, the GEF-1 protein is composed of 4 regions (domains), i.e., a zinc-finger sequence (FYVE)-containing region (Z), a proline-rich region (P), a coiled-coil sequence region (C) and a proline rich-2/glutamine-rich region (Q) in this order from the amino-terminal end.

Polypeptides comprising at least the C-Q region of GEF-1 have the ability to induce EMT, while polypeptides free from the Q region and comprising the C region (e.g., ZPC, PC, C) have inhibitory activity against EMT induction. Moreover, in an *in vitro* cell migration experiment using a transwell chamber, cells expressing the full-length GEF-1 (ZPCQ) enhance their migration capacity, whereas GEF-1 mutants lacking the Q region and comprising the C region (ZPC, PC, C) cause a significant decrease in their migration capacity, and in particular, cells expressing the C region show a remarkably significant decrease in their migration capacity (JP 2005-247735 A).

Furthermore, as described later in the Example section, polypeptides covering the C region of GEF-1 inhibit metastasis of cancer cells.

Thus, polypeptides of GEF-1 excluding the Q region, which comprise at least the C region, i.e., polypeptides covering the ZPC region of GEF-1, preferably the PC region, more preferably the C region have an anti-metastatic effect on cancer cells. Moreover, polypeptides covering the C region of GEF-1 have an anti-angiogenic effect and an anti-tumor growth effect.

### (2) Polypeptides of GEF-1 excluding the Q region, which comprise at least the C region

In the context of the present invention, the "polypeptide of galactosylceramide expression factor-1 excluding the Q region, which comprises at least the C region" is intended to mean a polypeptide which is free from the Q region of GEF-1 and comprises the C region of GEF-1. Examples of such a polypeptide include a polypeptide covering the C region of GEF-1 (GEF-1/C), as well as a polypeptide comprising the full-length P region or a part thereof at the amino-terminal side of the C region (GEF-1/PC) and a polypeptide comprising the full-length Z region or a part thereof at the amino-terminal side of the PC region (GEF-1/ZPC), with the polypeptide covering the C region (GEF-1/C) being preferred.

The polypeptides GEF-1/ZPC, GEF-1/PC and GEF-1/C and mutants thereof can be readily prepared by those skilled in the art on the basis of Table 1 shown below and the mutant preparation techniques mentioned above. The positions of the Z, P, C and Q regions in the amino acid sequences shown in SEQ ID NO: 2, SEQ ID NO: 4 and SEQ ID NO: 6 are shown in Table 1 below.

**Table 1**

| Positions of Z, P, C and Q regions in the full-length amino acid sequence of GEF-1/Hrs | | | | |
|---|---|---|---|---|
| | Z | P | C | Q |
| Rat (SEQ ID NO: 2) | 1 to 233 | 234 to 390 | 391 to 562 | 563 to 771 |
| Human (SEQ ID NO: 4) | 1 to 233 | 234 to 390 | 391 to 563 | 564 to 777 |
| Mouse (SEQ ID NO: 6) | 1 to 233 | 234 to 390 | 391 to 561 | 562 to 775 |

### (3) Polynucleotides encoding polypeptides of GEF-1 excluding the Q region, which comprise at least the C region

Examples of polynucleotides encoding the polypeptides described in (2) above include polynucleotides encoding GEF-1/C, GEF-1/PC and GEF-1/ZPC shown above. These polynucleotides and mutants thereof can be readily prepared by those skilled in the art on the basis of Table 2 shown below and the hybridization techniques mentioned above. The positions of the Z, P, C and Q regions in the nucleotide sequences of SEQ ID NO: 1 (rat GEF-1), SEQ ID NO: 3 (human Hrs) and SEQ ID NO: 5 (mouse Hrs) are shown in Table 2 below.

**Table 2**

| Positions of polynucleotides encoding Z, P, C and Q regions | | | | |
|---|---|---|---|---|
| | Z | P | C | Q |
| Rat (SEQ ID NO: 1) | 21 to 719 | 720 to 1190 | 1191 to 1706 | 1707 to 2333 |
| Human (SEQ ID NO: 3) | 76 to 774 | 775 to 1245 | 1246 to 1764 | 1765 to 2406 |
| Mouse (SEQ ID NO: 5) | 37 to 735 | 736 to 1206 | 1207 to 1719 | 1720 to 2361 |

### 4. Polypeptide covering the C region of GEF-1

### (1) Polypeptide covering the C region of GEF-1 (GEF-1/C)

In the context of the present invention, the polypeptide of GEF-1 excluding the Q region, which comprises at least the C region is intended to include a polypeptide covering the C region of GEF-1.

The polypeptide covering the C region of GEF-1 has an anti-metastatic effect on cancer cells, an anti-angiogenic effect and an anti-tumor growth effect.

The polypeptide covering the C region of GEF-1 includes a polypeptide which consists of the amino acid sequence shown in SEQ ID NO: 8 (rat GEF-1/C), SEQ ID NO: 10 (human Hrs/C) or SEQ ID NO: 12 (mouse Hrs/C).

In addition to the above polypeptide which consists of the amino acid sequence shown in SEQ ID NO: 8, 10 or 12, other members of the polypeptide covering the C region of GEF-1 include a polypeptide which consists of an amino acid sequence mutated by deletion, substitution or addition (or any combination thereof) of one or several amino acids in the amino acid sequence shown in SEQ ID NO: 8, 10 or 12 and which has an angiogenic effect or a tumor growth effect.

Examples of the above amino acid sequence mutated by deletion, substitution or addition (or any combination thereof) of one or several amino acids in the amino acid sequence shown in SEQ ID NO: 8, 10 or 12 include:
(i) an amino acid sequence with deletion of 1 to 10 (e.g., 1 to 5, preferably 1 to 3, more preferably 1 to 2, even more preferably 1) amino acids from the amino acid sequence shown in SEQ ID NO: 8, 10 or 12;
(ii) an amino acid sequence with substitution of other amino acids for 1 to 10 (e.g., 1 to 5, preferably 1 to 3, more preferably 1 to 2, even more preferably 1) amino acids in the amino acid sequence shown in SEQ ID NO: 8, 10 or 12;
(iii) an amino acid sequence with addition of 1 to 10 (e.g., 1 to 5, preferably 1 to 3, more preferably 1 to 2, even more preferably 1) amino acids to the amino acid sequence shown in SEQ ID NO: 8, 10 or 12; and
(iv) an amino acid sequence mutated by any combination of (i) to (iii) above.

The angiogenic effect and tumor growth effect in the context of the present invention have the same definitions and may be measured in the same manner as described above. Likewise, to prepare the above mutation-carrying polypeptides, mutations may be introduced into polynucleotides in the same manner as described above.

The polypeptide covering the C region of GEF-1 encompasses not only those having the amino acid sequence shown in SEQ ID NO: 8, 10 or 12, but also those having amino acid sequences sharing a homology of about 85% or more, preferably about 90% or more, more preferably about 95% or more with the amino acid sequence shown in SEQ ID NO: 8, 10 or 12 and having an anti-angiogenic effect or an anti-tumor growth effect (i.e., amino acid sequences substantially equivalent to the amino acid sequence shown in SEQ ID NO: 8, 10 or 12). Homology search may be accomplished in the same manner as described above.

### (2) Polynucleotide encoding the polypeptide covering the C region of GEF-1 (GEF-1/C)

In the present invention, the polynucleotide encoding GEF-1/C is not limited in any way, as long as it is a polynucleotide comprising the nucleotide sequence shown in SEQ ID NO: 7, 9 or 11 or a nucleotide sequence encoding the above polypeptide covering the C region of GEF-1. For example, in the present invention, it is possible to use not only a polynucleotide encoding a polypeptide which consists of the amino acid sequence shown in SEQ ID NO: 8, 10 or 12, but also a polynucleotide encoding a mutated polypeptide which consists of an amino acid sequence with deletion, insertion, substitution or addition of one or several amino acids in the amino acid sequence shown in SEQ ID NO: 8, 10 or 12 and which has an angiogenic effect or a tumor growth effect.

The polynucleotide encoding GEF-1/C of the present invention encompasses a polynucleotide which consists of the nucleotide sequence shown in SEQ ID NO: 7, 9 or 11, or a polynucleotide which is hybridizable under stringent conditions with a polynucleotide consisting of a sequence complementary to the nucleotide sequence and which encodes a polypeptide having an anti-angiogenic effect or an anti-tumor growth effect. Such a polynucleotide may be obtained in a known manner on the basis of the polynucleotide which consists of the nucleotide sequence shown in SEQ ID NO: 7, 9 or 11. Moreover, conditions required for the above hybridization and techniques used for nucleotide sequencing are the same as described above.

### 5. Constituent oligopeptides derived from the C region of GEF-1 (GEF-1/C constituent oligopeptides)

In view of the fact that the polypeptide covering the C region of GEF-1 has an anti-metastatic effect on cancer cells, an anti-angiogenic effect and an anti-tumor growth effect, partial polypeptides thereof may also have the same effects. As described later in the Example section, constituent oligopeptides derived from the C region of GEF-1 actually have an anti-metastatic effect on cancer cells, an anti-angiogenic effect and an anti-tumor growth effect.

Thus, the polypeptide to be used in the present invention encompasses polypeptides of GEF-1 excluding the Q region, which comprise at least the C region or a part thereof. As used herein, the phrase "a part thereof" is intended to mean a part of the C region, and its amino acid sequence may have any length. Moreover, oligopeptides composed of 2 to 20 amino acid residues also fall within the "polypeptide" in the context of the present invention.

Examples of polypeptides comprising a part of the C region include polypeptides comprising at least 6 or more consecutive amino acid residues, preferably 8 or more consecutive amino acid residues, more preferably 10 or more consecutive amino acid residues in the amino acid sequence shown in SEQ ID NO: 8, 10 or 12. More specific examples include polypeptides comprising 6 to 140 consecutive amino acid residues, preferably 8 to 30 consecutive amino acid residues, more preferably 8 to 20 consecutive amino acid residues, even more preferably 10 consecutive amino acid residues in the amino acid sequence shown in SEQ ID NO: 8, 10 or 12.

The range of amino acid sequences selected as "a part of the C region" is not limited in any way, as long as the selected amino acid sequences are within the amino acid sequence shown in SEQ ID NO: 8, 10 or 12. For example, in the case of the amino acid sequence shown in SEQ ID NO: 10, preferred is an amino acid sequence covering residues 33 (methionine) to 172 (alanine), and more preferred is an amino acid sequence covering residues 83 (lysine) to 152 (glutamic acid). Likewise, in the case of the amino acid sequence shown in SEQ ID NO: 8 or 12, an amino acid sequence corresponding to these residues can also be selected as a part of the C region.

Further, amino acid sequences covering a part of the C region include, but are not limited to, those comprising any of the following amino acid sequences, by way of example:
OP20-1: MKSNHMRGRSITNDSAVLSL (SEQ ID NO: 44)
OP20-2: ITNDSAVLSLFQSINTMHPQ (SEQ ID NO: 45)
OP20-3: FQSINTMHPQLLELLNQLDE (SEQ ID NO: 46)
OP20-4: LLELLNQLDERRLYYEGLQD (SEQ ID NO: 47)
OP20-5: RRLYYEGLQDKLAQIRDARG (SEQ ID NO: 48)
OP20-6: KLAQIRDARGALSALREEHR (SEQ ID NO: 49)
OP20-7: ALSALREEHREKLRRAAEEA (SEQ ID NO: 50)
OP20-8: EKLRRAAEEAERQRQIQLAQ (SEQ ID NO: 51)
OP20-9: ERQRQIQLAQKLEIMRQKKQ (SEQ ID NO: 52)
OP20-10: KLEIMRQKKQEYLEVQRQLA (SEQ ID NO: 53)
OP20-11: EYLEVQRQLAIQRLQEQEKE (SEQ ID NO: 54)
OP20-12: IQRLQEQEKERQMRLEQQKQ (SEQ ID NO: 55)
OP20-13: RQMRLEQQKQTVQMRAQMPA (SEQ ID NO: 56)
OP10-1: MGRGSGTFER (SEQ ID NO: 57)
OP10-3: FQSINTMHPQ (SEQ ID NO: 58)
OP10-6: KLAQIRDARG (SEQ ID NO: 59)
OP10-7: ALSALREEHR (SEQ ID NO: 60)
OP10-8: EKLRRAAEEA (SEQ ID NO: 61)
OP10-9: ERQRQIQLAQ (SEQ ID NO: 62)
OP10-10: KLEIMRQKKQ (SEQ ID NO: 63)
OP10-11: EYLEVQRQLA (SEQ ID NO: 64)
OP10-12: IQRLQEQEKE (SEQ ID NO: 65)

The above polypeptides comprising a part of the C region also encompass a polypeptide which consists of an amino acid sequence mutated by deletion, substitution or addition (or any combination thereof) of one or several amino acids in an amino acid sequence comprising at least 6 to 10 consecutive amino acid residues in the amino acid sequence shown in SEQ ID NO: 8, 10 or 12 and which has an anti-angiogenic effect or an anti-tumor growth effect. As used herein, the term "several amino acids" is intended to mean 1 to 10 (e.g., 1 to 5, preferably 1 to 3, more preferably 1 to 2, even more preferably 1) amino acids. When the total number of amino acid residues contained in the above polypeptide is around 20, the term is intended to mean 1 to 5 amino acids. When the total number of amino acid residues contained in the above polypeptide is around 10, the term is intended to mean 1 to 3 amino acids.

The above polypeptides comprising a part of the C region further encompass peptides consisting of an amino acid sequence covering a part of the C region, or mutants thereof, each having a cell membrane permeable peptide added (linked) thereto.

A cell membrane permeable peptide is a basic peptide rich in lysine residues and arginine residues, and known examples include HIV-1 virus-derived TAT protein (trans-activator of transcription protein) and so on. The percentage of basic amino acid residues contained in a cell membrane permeable peptide is, for example, 30% to 100%, preferably 100%. As an artificial synthetic cell membrane permeable peptide, a cell membrane permeable peptide composed of 7 to 11 arginine residues linked to each other is known. Moreover, arginine residues contained in such a cell membrane permeable peptide are preferably in D-form.

A cell membrane permeable peptide may be linked to any substance, and the substance carrying this peptide will be able to permeate the cell membrane and migrate into cells.

In the present invention, for example, a cell membrane permeable peptide may be linked to the above peptide consisting of an amino acid sequence covering a part of the C region, whereby this peptide consisting of an amino acid sequence covering a part of the C region can be transferred into target cells.

For example, a cell membrane permeable peptide comprising 9 residues of D-arginine (rrrrrrrrrGPG (SEQ ID NO: 66)) is linked to the above peptide consisting of an amino acid sequence covering a part of the C region, OP10-11 (EYLEVQRQLA), to prepare a polypeptide (rrrrrrrrrGPGEYLEVQRQLA (SEQ ID NO: 67)), and this polypeptide is brought into contact with target cells, whereby OP10-11 can be introduced into the target cells.

The angiogenic effect and tumor growth effect of the polypeptides comprising a part of the C region have the same definitions and may be measured in the same manner as described above. Likewise, to prepare the above mutation-carrying polypeptides, mutations may be introduced into polynucleotides in the same manner as described above.

Moreover, polynucleotides encoding the above polypeptides comprising a part of the C region can be readily prepared by those skilled in the art in accordance with known procedures on the basis of the amino acid sequence information of the above polypeptides comprising a part of the C region and/or the nucleotide sequence information of polynucleotides encoding the above C region polypeptides, etc.

### 6. Preparation of polypeptide

The polypeptide of GEF-1 excluding the Q region, which comprises at least the C region or a part thereof (hereinafter referred to as "the polypeptide of the present invention") may be prepared by using known procedures. Detailed procedures are as follows. (1) Preparation of expression vector

Any vector may be used to express the polypeptide of the present invention, as long as it can be held in a host cell where expression is to occur, and examples include plasmid DNAs, bacteriophages, etc.

Examples of plasmid DNAs include pME18S, pcDNA3, pBR322, pUC18, pUC19, pUC118, pUC119, pBluescript and so on, although other plasmids are also possible, such as those derived from *E*. *coli, Bacillus subtilis,* yeast, etc. Examples of phage DNAs include λ phages (Charon4A, Charon21A, EMBL3, EMBL4, λgt10, λgt11) and so on.

For integration of a polynucleotide encoding the polypeptide of the present invention into a vector, procedures involving cleavage with an appropriate restriction enzyme and the subsequent ligation by treatment with ligase may be used for this purpose (see, e.g., Molecular Cloning, CSHL Press cited above).

For example, a polynucleotide encoding the polypeptide of the present invention may be integrated as follows: primers are designed for each of the Z, P and C regions to amplify their respective polynucleotides, these regions are each amplified by PCR or other techniques, and only a desired segment of each region is then ligated to a vector using a restriction enzyme or ligase. Alternatively, primers may be designed to amplify a target segment, and the target segment may be amplified by PCR, and ligated to a vector.

### (2) Transformation

Any host may be used in the present invention, as long as it will express a polypeptide comprising the C region of GEF-1 and so on upon introduction of a gene encoding the polypeptide comprising the C region of GEF-1 and so on. Examples include, but are not limited to, mammalian cells, bacteria (e.g., bifidobacteria, lactic acid bacteria, *E. coli),* insect cells, yeast, fungi, etc.

Introduction of recombinant DNA into a host may be accomplished in a known manner. Techniques used to introduce the above vector into a host include, for example, calcium phosphate transfection, DEAE-dextran transfection, electroporation, cationic lipid transfection, etc.

Moreover, confirmation as to whether DNA has been introduced may be accomplished by using a selection marker gene (e.g., ampicillin resistance gene, neomycin resistance gene, hygromycin resistance gene, tetracycline resistance gene, chloramphenicol resistance gene, kanamycin resistance gene, zeocin resistance gene, blasticidin resistance gene).

### (3) Production of polypeptide

The polypeptide of the present invention can be obtained by culturing the above transformant carrying a polynucleotide encoding this polypeptide or a mutant thereof, and collecting the polypeptide from the cultured product.

The term "cultured product" is intended to mean a culture supernatant, cultured cells, cultured microorganisms, or a homogenate of cells or microorganisms. The transformant of the present invention may be cultured in accordance with standard procedures commonly used for host culture.

In the case of culturing a recombinant carrying an expression vector in which an inducible transcription promoter is used as a promoter, an inducer may optionally be added to the medium. When IPTG is used as an inducer, IPTG is added in an amount of 0.1 to 1.0 mM at 2 to 12 hours after the initiation of culture, and after IPTG addition, culture is further continued for 1 to 12 hours.

If the polypeptide of the present invention is accumulated within the cultured microorganisms or cells, a homogenizer or the like may be used to homogenize the microorganisms or cells, thereby collecting the desired polypeptide. If the polypeptide of the present invention is produced outside microorganisms or cells, the cultured solution is used directly or further treated by centrifugation or the like to remove the microorganisms or cells. Then, the above cultured solution is subjected to ammonium sulfate precipitation or other operations to collect the polypeptide, which is optionally further isolated and purified by various chromatographic techniques, etc.

Alternatively, in the present invention, a cell-free protein synthesis system may be used to produce the polypeptide of the present invention, without using any living cells.

A cell-free protein synthesis system is a system for protein synthesis from a cell extract in an artificial container (e.g., a test tube). For example, such a system is configured to synthesize a protein on a ribosome by reading the information of mRNA. It should be noted that the cell-free protein synthesis system to be used in the present invention encompasses a cell-free transcription system in which RNA is synthesized using DNA as a template.

The above cell extract may be an extract derived from eukaryotic or prokaryotic cells, as exemplified by extracts of wheat germ, rabbit reticulocytes, mouse L-cells, HeLa cells, CHO cells, budding yeast, *E. coli* cells and so on. It should be noted that these cell extracts may or may not be concentrated.

In the present invention, cell-free protein synthesis may be accomplished by using a commercially available kit. Examples of such a kit include reagent kits PROTEIOS™ (Toyobo Co., Ltd., Japan) and TNT™ System (Promega), as well as synthesizers PG-Mate™ (Toyobo Co., Ltd., Japan) and RTS (Roche Diagnostics), etc.

The polypeptide of the present invention obtained by cell-free protein synthesis can be purified by selecting an appropriate chromatographic technique, as described above. Moreover, confirmation as to whether the polypeptide of the present invention has been isolated and purified may be accomplished by SDS-PAGE or other techniques.

Alternatively, the polypeptide of the present invention may also be obtained from the full-length GEF-1 or the like by cleavage with cyanogen bromide or a peptidase, etc. Any peptidase may be used for this purpose as long as it is capable of cleaving the intended polypeptide, and examples include trypsin, chymotrypsin, lysyl endopeptidase and so on.

### (4) Peptide synthesis

The polypeptide of the present invention can be obtained by chemical synthesis. Peptide synthesis may be accomplished in a known manner using a synthesizer such as a Model 433A peptide synthesizer (Applied Biosystems) or PSSM-8 (Shimadzu Corporation, Japan). Alternatively, the polypeptide of the present invention may also be obtained by being purchased from a company (e.g., Hayashi-Kasei Co., Ltd., Japan) entrusted with peptide synthesis. A polypeptide having a cell membrane permeable peptide linked thereto may also be obtained in the same manner.

### 7. GEF-1 expression inhibitor

### (1) GEF-1 expression inhibitor

GEF-1 promotes cancer metastasis, induces angiogenesis and promotes tumor growth. Thus, these effects can be inhibited when inhibiting the expression of this GEF-1.

Examples of GEF-1 expression inhibitors include nucleic acids used for RNA interference (microRNA, shRNA, siRNA), antisense nucleic acids, decoy nucleic acids, or aptamers. These expression inhibitors are capable of inhibiting GEF-1 expression. The nucleotide sequence of the GEF-1 gene to be inhibited is already known, as described above, and sequence information can be obtained for each case. In the present invention, the nucleotide sequence of the GEF-1 gene is as shown in SEQ ID NO: 1, 3 or 5, although not only a coding region of GEF-1, but also a non-coding region may be used as a target.

### (2) RNA interference

In the present invention, RNA interference (RNAi) against the GEF-1 gene may be used to inhibit the gene from being expressed. Examples of nucleic acids used for such RNAi include siRNA, microRNA (miRNA) and shRNA.

siRNA (small interfering RNA) molecules are various RNAs corresponding to the target GEF-1 gene. Such RNAs include mRNA, post-transcriptionally modified RNA of the GEF-1 gene, etc.

In general, a target sequence on mRNA may be selected from the cDNA sequence corresponding to the mRNA. However, the target sequence is not limited to this region in the present invention.

siRNA molecules may be designed on the basis of the criteria well known in the art. For example, as a target segment in the target mRNA, it is possible to select a segment covering 15 to 30 consecutive bases, preferably 19 to 25 consecutive bases, preferably starting with AA, TA, GA or CA. siRNA molecules have a GC ratio of 30% to 70%, preferably 35% to 55%.

Because of forming double-stranded portions, siRNA can be produced as a single-stranded hairpin RNA molecule folding on its own nucleic acid. Although siRNA molecules may be obtained by standard chemical synthesis, they can also be biologically produced using an expression vector.

For introduction of siRNA into cells, it is possible to use, e.g., procedures in which synthesized siRNA is ligated to plasmid DNA and then introduced into cells, or procedures in which double-stranded RNA is annealed.

In the present invention, it is also possible to use commercially available siRNAs such as Stealth™ RNAi, Stealth™ Select RNAi (Invitrogen), etc.

In the present invention, shRNA may also be used for providing RNAi effect. shRNA is an RNA molecule called short hairpin RNA, which has a stem loop structure because some single-stranded regions form complementary strands with other regions.

shRNA may be designed to form a stem loop structure as a part thereof. For example, assuming that a sequence covering a certain region is designated as sequence A, and a strand complementary to the sequence A is designated as sequence B, shRNA is design to comprise the sequence A, a spacer and the sequence B linked in this order on a single RNA strand and to have an overall length of 45 to 60 bases. The spacer may also have any length. Although the sequence A is a sequence covering a partial region of the target GEF-1 gene, there is no particular limitation on the target region and any region may be selected as a candidate for the target region. In addition, the sequence A has a length of 19 to 25 bases, preferably 19 to 21 bases.

The thus designed shRNA may be produced, for example, as follows: a polynucleotide serving as a template of shRNA is amplified by PCR and then introduced into an appropriate vector for shRNA expression such as pSuperior.neo.gfp vector (Oligoengine), followed by gene transfer to introduce the resulting shRNA expression vector into target cells.

Further, in the present invention, microRNA may be used to inhibit GEF-1 expression. microRNA (miRNA) is an intracellular single-stranded RNA molecule having a length of about 20 to 25 bases and is a kind of ncRNA (non-coding RNA) which is considered to have the function of regulating the expression of other genes. miRNA is generated through processing upon transcription into RNA and is present as a nucleic acid capable of forming a hairpin structure which inhibits the expression of a target sequence.

Since miRNA is also a nucleic acid based on RNAi, miRNA may also be designed and synthesized in the same manner as in the case of shRNA or siRNA.

### (3) Antisense nucleic acid

In another embodiment of the present invention, an antisense nucleic acid may be used to inhibit GEF-1 expression. An antisense nucleic acid is a single-stranded nucleic acid sequence (either RNA or DNA) capable of binding to mRNA or DNA sequence of the GEF-1 gene. Such an antisense nucleic acid sequence has a length of at least 14 nucleotides, preferably 14 to 100 nucleotides. The antisense nucleic acid binds to the above gene sequence to form a duplex and thereby inhibit the transcription or translation of the GEF-1 gene.

The antisense nucleic acid may be prepared by chemical or biochemical synthesis procedures known in the art. For example, it is possible to use nucleic acid synthesis procedures using a commonly used DNA synthesizer. The antisense nucleic acid is introduced into cancer cells expressing GEF-1, for example, by various gene transfer techniques including DNA transfection or electroporation.

### (4) Decoy nucleic acid

In yet another embodiment of the present invention, a nucleic acid serving as a decoy, which is called a decoy nucleic acid, may be used to inhibit GEF-1 expression.

A decoy nucleic acid is a nucleic acid that inhibits GEF-1 gene expression through binding to the GEF-1 gene to inhibit promoter activity, and is intended to mean a short nucleic acid serving as a decoy that comprises a binding site for a transcription factor. When this nucleic acid is introduced into cells, a transcription factor will bind to this nucleic acid. As a result, the transcription factor will be competitively inhibited from binding to its genomic binding site and thus inhibited from being expressed.

Examples of a decoy nucleic acid preferred in the present invention include a nucleic acid binding to a promoter for the GEF-1 gene, or a nucleic acid binding to a promoter for mRNA of GEF-1 or for a factor located upstream of GEF-1. Such a decoy nucleic acid may be designed as a single or double strand on the basis of the promoter sequence for the GEF-1 gene. Although the decoy nucleic acid may have any length, its length is 15 to 60 bases, preferably 20 to 30 bases.

The decoy nucleic acid may be either DNA or RNA, and may also include modified nucleic acids.

The decoy nucleic acid to be used in the present invention may be prepared by chemical or biochemical synthesis procedures known in the art. For example, it is possible to use nucleic acid synthesis procedures using a DNA synthesizer commonly used in gene recombination technology. Alternatively, a nucleotide sequence serving as a template may be isolated or synthesized, followed by PCR or gene amplification using a cloning vector. Further, to obtain a decoy nucleic acid which is more stable within cells, bases and other constituents may be modified by chemical modifications such as alkylation, acylation, etc.

It should be noted that in the case of using a decoy nucleic acid, analysis of the promoter's transcriptional activity may be accomplished by using standard assays such as luciferase assay, gel shift assay, RT-PCR, etc. Kits for performing these assays are also commercially available (e.g., a promega dual luciferase assay kit).

### (5) Aptamer

Furthermore, in the present invention, an aptamer may be used to inhibit GEF-1 expression.

An aptamer is a synthetic DNA or RNA molecule or a peptidic molecule, which has the ability to bind specifically to a target substance, and can be synthesized chemically in a test tube within a short time. Thus, an aptamer may be obtained on the basis of the nucleotide sequence shown in SEQ ID NO: 1, 3 or 5, or alternatively, may be obtained by evolutionary engineering procedures known as *in vitro* selection or SELEX techniques.

A nucleic acid aptamer is rapidly degraded and removed in blood flow by the action of nucleases, and hence it is preferred that the aptamer has optionally been subjected to molecular modification, e.g., with a polyethylene glycol (PEG) chain to thereby extend its half-life.

### 8. Anti-tumor agent

The polypeptide of the present invention, the vector expressing this polypeptide, as well as shRNA and siRNA prepared in the present invention have an anti-metastatic effect on cancer, an anti-angiogenic effect and/or an anti-tumor growth effect. Thus, compositions comprising them can be used as anti-tumor agents.

The anti-tumor agent of the present invention is capable of not only indirectly inhibiting cancer metastasis and tumor growth through inhibition of tumor-induced angiogenesis, but also inhibiting cancer metastasis through inhibition of the migration capacity of cancer cells, and further inhibiting tumor growth through inhibition of the expression of transcription factors involved in tumor growth. Thus, the anti-tumor agent of the present invention is very useful for cancer treatment when compared to known anti-tumor agents, because it achieves direct inhibition of three events, i.e., cancer metastasis, angiogenesis and tumor growth, and further allows tumor treatment as a result of a synergistic effect produced thereby.

In the context of the present invention, the term "cancer" or "tumor" is intended to include, but is not limited to, solid cancers such as brain tumor, esophageal cancer, tongue cancer, lung cancer, breast cancer, pancreatic cancer, gastric cancer, small intestinal or duodenal cancer, colorectal cancer (colon cancer, rectal cancer), bladder cancer, kidney cancer, liver cancer, prostate cancer, uterine cancer, uterine cervical cancer, ovarian cancer, thyroid cancer, gallbladder cancer, pharyngeal cancer, sarcoma (e.g., osteosarcoma, chondrosarcoma, Kaposi's sarcoma, myosarcoma, angiosarcoma, fibrosarcoma), melanoma and so on, as well as blood tumors such as leukemia (e.g., chronic myelogenous leukemia (CML), acute myelogenous leukemia (AML), chronic lymphocytic leukemia (CLL) and acute lymphocytic leukemia (ALL)), lymphoma, multiple myeloma (MM) and so on.

In the context of the present invention, the term "angiogenesis" is intended to include both physiological angiogenesis and pathological angiogenesis, preferably refers to pathological angiogenesis. Physiological angiogenesis is observed at a limited site and during a limited period, and is inhibited under normal conditions. In contrast, pathological angiogenesis is not under the control of *in vivo* regulatory mechanisms and hence will occur disorderly, and is observed in cancer or tumor, diabetic retinopathy, rheumatoid arthritis, psoriasis vulgaris, atherosclerosis, etc.

In the context of the present invention, pathological angiogenesis is preferably tumor-induced angiogenesis.

The term "metastasis" is intended to mean that cancer cells are transported via the blood or lymphatic system or during surgical operation from their primary focus to another distant site where the cancer cells will form a new growth focus. Alternatively, the term "metastasis" refers to "the ability of cancer cells to propagate themselves and form a new growth focus at a discrete site (i.e., the ability to form metastasis)" (Hill, R. P, "Metastasis", The Basic Science of Oncology, edited by Tannock, et. al., 178-195 (McGraw-Hill, New York, 1992)).

The anti-tumor agent of the present invention may comprise a pharmaceutically acceptable carrier, in addition to the polypeptide of the present invention, the vector expressing this polypeptide, or shRNA or siRNA prepared in the present invention. The term "pharmaceutically acceptable carrier" refers to any type of carrier (e.g., liposomes, lipid vesicles, micelles), diluent, excipient, wetting agent, buffering agent, suspending agent, lubricant, adjuvant, emulsifier, disintegrant, absorbent, preservative, surfactant, coloring agent, flavoring agent or sweetener, which is suitable for use as an anti-tumor agent.

The anti-tumor agent of the present invention may be formulated into any dosage form, such as injections, lyophilized preparations, tablets, hard capsules, soft capsules, granules, powders, pills, syrups, suppositories, poultices, ointments, creams, eye drops, etc.

The anti-tumor agent of the present invention is topically or systemically administered by any means known to those skilled in the art. The dose will vary depending on factors such as the age, body weight, health condition, sex, symptoms of a patient, the route of administration, the frequency of administration, the dosage form of the agent, etc. Detailed procedures for administration may be determined by those skilled in the art. For example, for adults, the anti-tumor agent of the present invention may be administered in tablet form at a dose of 0.1 µg to 10 g, preferably 1 µg to 1 g, more preferably 10 µg to 100 mg, once to five times a day.

Further, if the anti-tumor agent of the present invention is used as an agent for gene therapy, the anti-tumor agent of the present invention may be administered directly by injection, or alternatively, a vector carrying the nucleic acid may be administered. Examples of such a vector include adenovirus vector, adeno-associated virus vector, herpes virus vector, vaccinia virus vector, retrovirus vector, lentivirus vector and so on. The use of these virus vectors facilitates efficient administration.

Moreover, the anti-tumor agent of the present invention may be encapsulated within phospholipid vesicles (e.g., liposomes), and these vesicles may be used for administration. Vesicles holding siRNA or shRNA are introduced into predetermined cells by lipofection. Then, the resulting cells are systemically administered, for example, by the intravenous or intraarterial route. They may also be topically administered to the brain, etc. For example, for adults, such an anti-tumor agent may be administered at a dose of 0.1 µg/kg to 1000 mg/kg per day, preferably 1 µg/kg to 100 mg/kg per day. To introduce siRNA or shRNA into a target tissue or organ, a commercially available gene transfer kit (e.g., AdenoExpress: Clontech) may also be used.

Further, the vector expressing the polypeptide of the present invention may be transformed into a host (e.g., bifidobacteria, lactic acid bacteria, yeast, filamentous fungi) and the resulting transformant may be used as an anti-tumor agent. Examples of bifidobacteria include *Bifidobacterium longum, Bifidobacterium bifidum, Bifidobacterium breve,* etc. Examples of lactic acid bacteria include bacteria of the genera *Lactobacillus, Streptoccoccus, Leuconostoc, Pediococcus,* etc. Such a transformant may be used directly or may be formulated into any of the above dosage forms as appropriate before use as the anti-tumor agent of the present invention.

### 9. Angiogenesis inhibitor and tumor growth inhibitor

The polypeptide of the present invention, the vector expressing this polypeptide, as well as shRNA and siRNA prepared in the present invention have an anti-angiogenic effect and an anti-tumor growth effect. Thus, compositions comprising them can be used as angiogenesis inhibitors and as tumor growth inhibitors. Angiogenesis in the context of the present invention is as defined above in "8. Anti-tumor agent."

The angiogenesis inhibitor and tumor growth inhibitor of the present invention can be used as reagents or used for treatment of mammals, and various conditions including the mode of administration, the type of additive, the route of administration, the target to be administered, and the dose to be administered may be selected as appropriate as described above in "8. Anti-tumor agent."

### 10. Eliminator of cancer cell characteristics

The polypeptide of the present invention and the vector expressing this polypeptide have an elimination effect on cancer cell characteristics. Thus, compositions comprising them can be used as eliminators of cancer cell characteristics. In the context of the present invention, the term "cancer cell characteristics" is intended to include the migration capacity, the metastatic ability, the angiogenic capacity, the property of growing without causing contact inhibition, the growth ability in soft agar medium (i.e., the property of growing in an anchorage-independent manner) and so on.

The eliminator of cancer cell characteristics of the present invention may be administered to cancer cells to thereby allow the cancer cells to acquire the ability of contact inhibition. As used herein, the term "contact inhibition" is intended to mean that normal cells stop their growth at a certain level due to contact with a physical obstacle and/or cell-cell contact during their growth process. For example, when animal cells are cultured in a culture dish, contact with a physical obstacle and/or cell-cell contact will cause a change in the cytoskeleton of the animal cells through the cell membrane to thereby change the direction of cell movement or reduce the amount of cell movement. Moreover, normal fibroblasts and normal epithelial cells will stop their growth once they have spread over the surface of a culture dish, unlike cancer cells. Namely, normal cells are inhibited from growing in a cell density-dependent manner and stop their growth upon reaching a confluent state. When they are seeded again at a low cell density, they can grow again. In contrast, cancer cells continue to grow even after the cell density has reached a confluent state, and further continue to grow until oxygen or nutrients are depleted. Namely, contact inhibition is an event that is observed in normal cells, but not in cancer cells. Thus, contact inhibition is indicative of elimination of cancer cell characteristics. Contact inhibition also includes contact growth inhibition and contact blocking.

The elimination effect on cancer cell characteristics in the polypeptide of the present invention (e.g., a polypeptide which consists of the amino acid sequence shown in SEQ ID NO: 8, 10 or 12) and a mutant thereof may be evaluated in a known manner, for example, by introducing the polypeptide or expression vector of the present invention into cancer cells, and determining whether the transfected cancer cells are inhibited from growing in a culture dish in a cell density-dependent manner.

The eliminator of cancer cell characteristics of the present invention can be used as a reagent or used for treatment of mammals, and various conditions including the mode of administration, the type of additive, the route of administration, the target to be administered, and the dose to be administered may be selected as appropriate as described above in "8. Anti-tumor agent."

### 11. Method for angiogenesis inhibition and method for tumor growth inhibition

The polypeptide of the present invention, the vector expressing this polypeptide, as well as shRNA and siRNA prepared in the present invention can be administered to mammals or mammalian cells to inhibit GEF-1 expression, thereby inhibiting angiogenesis and tumor growth. Thus, the present invention provides a method for angiogenesis inhibition and a method for tumor growth inhibition.

Various conditions required for the polypeptide of the present invention and so on used in the methods of the present invention, including the mode of administration, the type of additive, the route of administration, the target to be administered, and the dose to be administered may be selected as appropriate as described above in "8. Anti-tumor agent."

### EXAMPLES

The present invention will be further described in more detail by way of the following illustrative examples, which are not intended to limit the scope of the invention.

### Example 1

### Preparation of GEF-1 mutant cells and study of their metastatic ability

### 1. Cell lines

Mouse melanoma cell line B16 and Lewis lung carcinoma (LLC) cells used in this example were obtained from the Cell Resource Center for Biomedical Research, the Institute of Development, Aging and Cancer, Tohoku University, Japan, while human colorectal cancer-derived cell line COLO205, human lung cancer cell line A549, human gastric cancer-derived cell line KATOIII and human uterine cervical cancer cell line HeLa cells were obtained from the BioResource Center, RIKEN, Japan (RIKEN BRC).

The human pancreatic cancer-derived cells used were PT45 cells (Egawa N. et al., Virchows Arch. 1996 429:59-68), while the mouse vascular endothelial cells used were KOP2.16 cells (Shimamura M. et al., Int J Cancer. 2004 111:111-6).

Canine renal epithelium-derived cell line MDCK cells and mouse lymphoma-derived EL4 cells were obtained from the National Institute of Biomedical Innovation, Japan.

The respective cells were cultured in RPMI medium (Invitrogen) containing penicillin, streptomycin and 10% FBS (HyClone) under conditions of 37°C and 5% CO₂.

### 2. Preparation of GEF-1 mutant cells

### (1) Preparation of expression vectors for GEF-1 and deficient mutants thereof

Using primers designed for GEF-1 or each deficient mutant, i.e., a sense strand primer containing a restriction enzyme EcoRI site and the FLAG epitope sequence as well as an antisense strand primer containing a restriction enzyme NotI site, GEF-1 was used as a template in PCR to prepare each mutant cDNA. GEF-1 of rat origin was used for this purpose.

PCR was performed for 20 cycles under conditions of denaturation at 94°C for 30 seconds, annealing at 60°C for 30 seconds, and elongation at 72°C for 90 seconds to 300 seconds. As primers, the following sense primer and antisense primer were used. Z sense primer: 
5'-TTGAATTCATGGACTACAAGGACGACGATGACAAGATGGGGCGAGGCAGC
GGCACC-3' (SEQ ID NO: 13)
 P sense primer: 
5'-TTGAATTCATGGACTACAAGGACGACGATGACAAGCCCCCAGAGTACCTG
ACCAGC-3' (SEQ ID NO: 14)
 C sense primer: 
5'-TTGAATTCATGGACTACAAGGACGACGATGACAAGTTTAGTGAGCAGTAC
CAGAAC-3' (SEQ ID NO: 15)
 Q sense primer: 
5'-TTGAATTCATGGACTACAAGGACGACGATGACAAGCCCTTGCCTTATGCCC
AGCTC-3' (SEQ ID NO: 16)
 Z-antisense primer:
5'-ATAGTTTATGCGGCCGCTAAGTGGTAGAGGCAGCTTT-3' (SEQ ID NO: 17)
P-antisense primer:
5'-ATAGTTTATGCGGCCGCTCAGGAAGTTATGGGCTGAGA-3' (SEQ ID NO: 18)
C-antisense primer:
5'-ATAGTTTATGCGGCCGCTAGGCACGCATCTGGACAGTCT-3' (SEQ ID NO: 19)
Q-antisense primer:
5'-ATAGTTTATGCGGCCGCTCAGTCGAAGGAGATGAGCTGGGT-3' (SEQ ID NO: 20)

After the sequence of each amplification product was confirmed, each fragment cleaved with EcoRI and NotI was introduced into the EcoRI-NotI site of pcDNA3 animal cell expression vector (Invitrogen) to prepare an expression vector for each of GEF-1 and Q region-deficient mutants.

### (2) Preparation of GEF-1-expressing cells and Q region-deficient mutant-expressing cells

The following cells were prepared in a known manner (Patki, V, et al., Nature, 394, 433-434, 1998). More specifically, the expression vectors prepared above for GEF-1 (ZPCQ) and Q region-deficient mutants (ZPC, PC and C regions) were each introduced together with an expression vector for the blasticidin selection gene (pSV2bsr vector) (Funakoshi Co., Ltd., Japan) into various cells, followed by selection with blasticidin (Funakoshi Co., Ltd., Japan) (2 mg/L) to obtain stably expressing cells. The expressing cells were each cloned by limiting dilution techniques.
a) GEF-1 (ZPCQ region)-expressing MDCK cells (MDCK/GEF-1 cells)
b) GEF-1 (ZPCQ region)-expressing B16 cells (B16/GEF-1 cells)
c) Q region-deficient mutant (ZPC, PC or C region)-expressing MDCK cells (MDCK/ZPC cells, MDCK/PC cells, or MDCK/C cells)
d) Q region-deficient mutant (ZPC, PC or C region)-expressing B16 cells (B16/ZPC cells, B 16/PC cells, or B16/C cells)
e) C region-expressing LLC cells (LLC/C cells)
f) C region-expressing KOP2.16 cells (KOP/C cells)
g) C region-expressing PT45 cells (PT45/C cells)
h) C region-expressing COLO205 cells (COLO205/C cells)
i) C region-expressing EL4 cells (EL4/C cells)

For use as controls, an empty vector and pSV2bsr vector were introduced together to prepare control MDCK cells (MDCK/bsr cells) and control B16 cells (B 16/bsr cells).

### (3) Preparation of B16 cells expressing shRNA against the GEF-1 gene

### i) Preparation of shRNA expression vector

Expression vectors for three shRNAs (shRNA-74, shRNA-157, shRNA-207) were prepared in the following manner. It should be noted that "74", "157" and "207" represent the positions of the 5'-terminal bases of the respective shRNAs in the open reading frame (ORF). For example, in the case of shRNA-74, the third guanine (G) from the 5'-terminal end of the Hgs74 template corresponds to the 74th base in the ORF of the GEF-1 gene. The GEF-1 (Hrs/Hgs) gene used in the preparation of shRNA expression vectors was of mouse origin.

pSuperior.neo.gfp vector (Oligoengine) was cleaved with restriction enzymes BglII and XhoI, followed by BAP treatment.

On the other hand, three fragments to be introduced into pSuperior.neo.gfp vector were obtained by PCR. PCR was performed in a reaction solution containing a Hgs template (200 pmol), a Hgs sense primer (400 pmol), a Hgs antisense primer (400 pmol), a specifically prepared 1× buffer, KOD DNA polymerase (2.5 U, TOYOBO), 0.2 mM dNTP and 1 mM MgCl₂ under the following temperature conditions: [95°C for 30 seconds, 55°C for 30 seconds and 68°C for 30 seconds ] × 24 cycles + [95°C for 30 seconds, 55°C for 30 seconds and 68°C for 7 minutes] × 1 cycle. The respective Hgs templates, Hgs sense primers and Hgs antisense primers used in PCR are shown below.
Hgs74 sense primer:
ATATAGATCTCCGGGAGTCCATTCTACAGAT (SEQ ID NO: 21)
Hgs74 antisense primer:
GGTACCTCGAGTAAAAAGGGAGTCCA (SEQ ID NO: 22)
Hgs74 template: 
CCGGGAGTCCATTCTACAGATTTCAAGAGAATCTGTAGAATGGACTCCCTTTT
TA (SEQ ID NO: 23)
 Hgs157 sense primer:
ATATAGATCTCCGTTAATGATAAGAACC (SEQ ID NO: 24)
HgS157 antisense primer:
GGTACCTCGAGCTTAAAAAGTTAATGAT (SEQ ID NO: 25)
Hgs157 template: 
CCGTTAATGATAAGAACCCACTTCAAGAGAGTGGGTTCTTATCATTAACTTTT
TA (SEQ ID NO: 26)
 Hgs207 sense primer:
ATATAGATCTCCGTCTGTGGTAAAGAACT (SEQ ID NO: 27)
Hgs207 antisense primer:
GGTACCTCGAGCTTAAAAAGTCTGTGGT (SEQ ID NO: 28)
Hgs207 template:

CCGTCTGTGGTAAAGAACTGTTTCAAGAGAACAGTTCTTTACCACAGACTTT
TTA (SEQ ID NO: 29)

The above three fragments obtained by PCR were purified and then cleaved with restriction enzymes BglII and XhoI, and each introduced into the BAP-treated pSuperior.neo.gfp vector.

### ii) Preparation of shRNA-expressing B 16 cells

The respective shRNA expression vectors prepared in i) above were each introduced into B16 cells by gene transfer and subjected to drug selection using G418 drug (1 mg/ml). B16 cells expressing the respective shRNAs (B16/shRNA-74 cells, B16/shRNA-157 cells and B16/shRNA-207 cells) were finally obtained by cloning. These cells were confirmed for their GEF-1 expression levels by Western blotting and also examined for their ability of cell migration using a Boyden chamber.

As a result, the three types of cells all showed 80% or more inhibition of both Hgs expression and cell migration ability. The B16/shRNA-157 cells showed 95% inhibition of both Hgs expression and cell migration ability, and hence showed the highest inhibition among the three types of cells. Then, B16/shRNA-157 cells were used as B16/shRNA cells in the subsequent experiments.

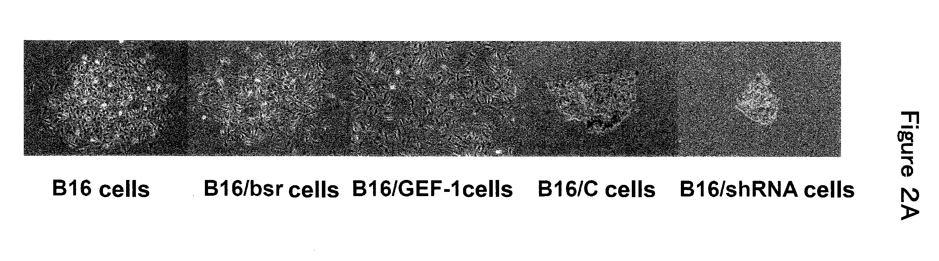

Among the cells prepared in (2) and (3) above, B 16/bsr cells, B16/GEF-1 cells, B16/C cells, B 1 6/shRNA cells and their parent B16 cells are morphologically shown in Figure 2A.

As shown in Figure 2A, the B16/GEF-1 cells showed a more fibroblast-like morphology with less cell-cell contact than their parent B16 cells or the B16/bsr cells. In contrast, the B16/C cells and the B16/shRNA cells showed a morphology with more cell-cell contact.

This result indicated that expression of GEF-1 enhanced the migration capacity of B16 cells, whereas expression of the C region and shRNA of GEF-1 inhibited the migration capacity of B16 cells.

Further, the B16/GEF-1 cells, the B16/C cells, the B16/shRNA cells and the B16 cells were examined for their metastatic ability. More specifically, the B16/GEF-1 cells, the B16/C cells, the B16/shRNA cells and the B16 cells (1 × 10⁶/0.2 mL PBS) were each administered to C57BL6 mice via the tail vein, and metastasis in their lungs were observed at 21 days after administration. In addition, HE staining was also conducted. Further, the number of days from cell administration until the death of each animal was counted to determine the number of days to reach 50% mortality

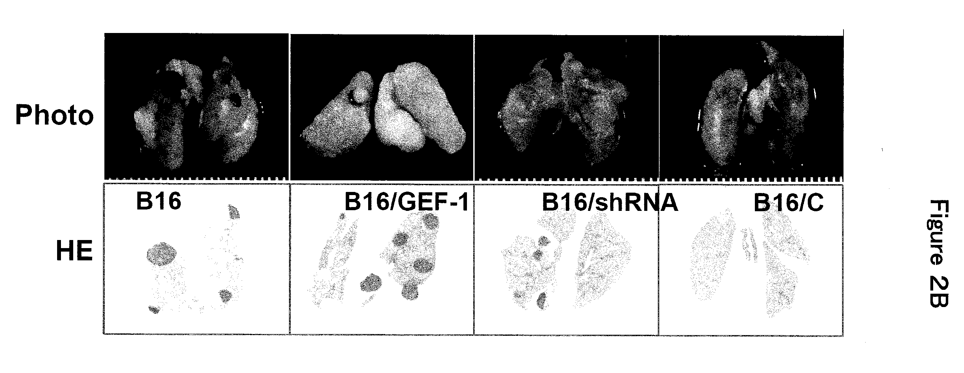

As a result, in the mice at 21 days after administration, the B16/GEF-1 cells enhanced their metastasis to the lung, whereas the B16/shRNA and B16/C cells were inhibited from metastasizing to the lung. In particular, the B16/C cells were significantly inhibited from metastasizing to the lung (Figure 2B).

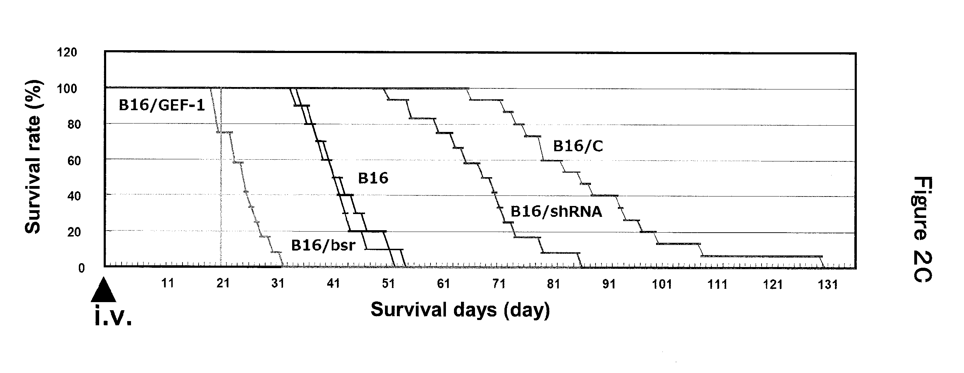

Moreover, the 50% longevity (survival days) was 42 days in the B16 cells and the B16/bsr cells, whereas the 50% longevity was greatly reduced to 25 days in the B16/GEF-1 cells. In contrast, the 50% longevity was 87 days in the B16/C cells, which was increased twice or more than in the B16 cells and the B16/bsr cells. Likewise, the B16/shRNA cells also showed a great increase in 50% longevity as compared to the B16 cells and the B16/bsr cells (Figure 2C).

These results indicated that expression of GEF-1 enhanced metastasis of cancer cells, whereas expression of the C region and shRNA of GEF-1 significantly inhibited metastasis of cancer cells.

### Example 2

### Study of polypeptides covering the C region of GEF-1 for their effect on angiogenesis

### 1. In vitro tube formation test

KOP2.16 cells were used as vascular endothelial cells. KOP2.16 cells were modified to stably express the C region of GEF-1 (KOP/C cells) and measured for their tube formation ability.

More specifically, the above KOP2.16 cells and KOP/C cells were each adjusted to fixed cell counts and seeded on Matrigel in a microplate. After culture for 18 to 48 hours, the cells were photographed under an inverted microscope. Based on the resulting images, the state of tube formation was scored using measurement software to determine the tube formation ability.

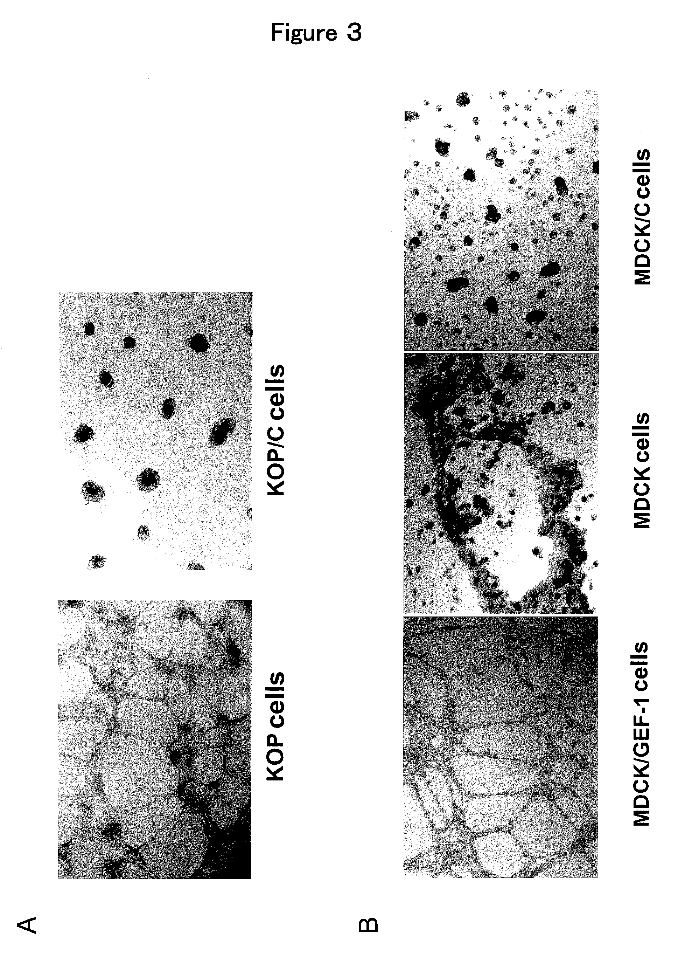

As a result, the mouse vascular endothelial cells (KOP2.16) showed a tube morphology where the cells were assembled, whereas the tube formation ability disappeared in the KOP/C cells (Figure 3A).

Moreover, MDCK cells, which are renal epithelium-derived cells, but not vascular endothelium-derived cells, were also subjected to the same tube formation test as in the case of the KOP cells, because MDCK cells slightly have the tube formation ability

As a result, MDCK/GEF-1 cells significantly enhanced their tube formation ability. In contrast, the tube formation ability completely disappeared in MDCK/C cells (Figure 3B).

These results indicated that polypeptides comprising the C region of GEF-1 inhibited tube formation during angiogenesis.

### 2. In vivo angiogenesis induction test

Using an *in vivo* dorsal air sac (DAS) assay, B16 cells were examined for their ability to induce angiogenesis. The DAS assay is an assay used for study of pathological angiogenesis including tumor-induced angiogenesis.

Chambers holding the respective cells (B16 cells, B16/GEF-1 cells, B16/C cells, B16/shRNA cells) were transplanted under the dorsal skin of mice. After 7 days, each chamber was removed and the dorsal skin of each mouse was then photographed under a stereoscopic microscope. Based on the resulting images, the number and area of neovascular vessels induced by the tumor cells were scored using measurement software to determine the tube formation ability.

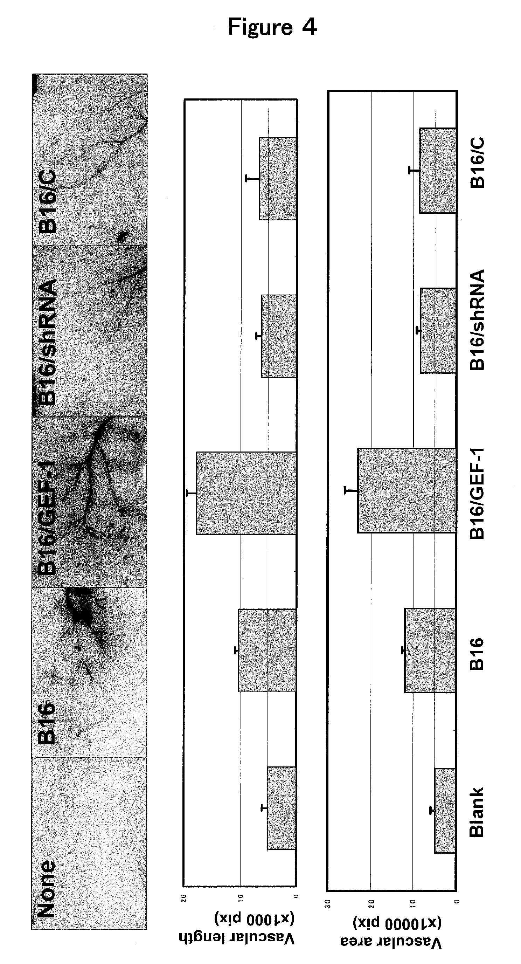

As a result, the B16/GEF-1 cells enhanced their ability to induce angiogenesis as compared to the B16 cells. In contrast, the B16/C cells and the B16/shRNA cells showed a reduction in their ability to induce angiogenesis (Figure 4).

These results indicated that expression of GEF-1 induced, both *in vitro* and *in vivo,* cancer cell-induced angiogenesis (pathological angiogenesis) and vascular endothelial cell angiogenesis.

Moreover, the polypeptides (polypeptides comprising the C region of GEF-1) and inhibitory substances (shRNAs against a polynucleotide encoding GEF-1) in the present invention were found to inhibit cancer cell-induced angiogenesis and vascular endothelial cell angiogenesis.

### Example 3

### 1. Study of polypeptides covering the C region of GEF-1 for their effect on tumor growth

### (1) In vitro growth of B16/C cells and EL4/C cells

The relationship between GEF-1 expression and tumor growth was studied using GEF-1 mutant cell lines of B16 cells and EL4 cells.

The respective cells (B16 cells, B16/GEF-1 cells, B16/C cells, B16/shRNA cells, EL4 cells, EL4/C cells) were washed with PBS and then treated with TrypLE Express^{®} (Invitrogen) to separate the cells, which were then suspended again in Opti-MEM I^{®} Reduced-Serum Medium (Opti-MEM I medium, Invitrogen). Cell counts in these suspensions were measured using a Countess^{®}.

As a result, the B16/GEF-1 cells and the B16/shRNA cells showed the same growth pattern as that of their parent B16 cells. These cells all died when the cell density reached about 3 × 10⁶/cm².

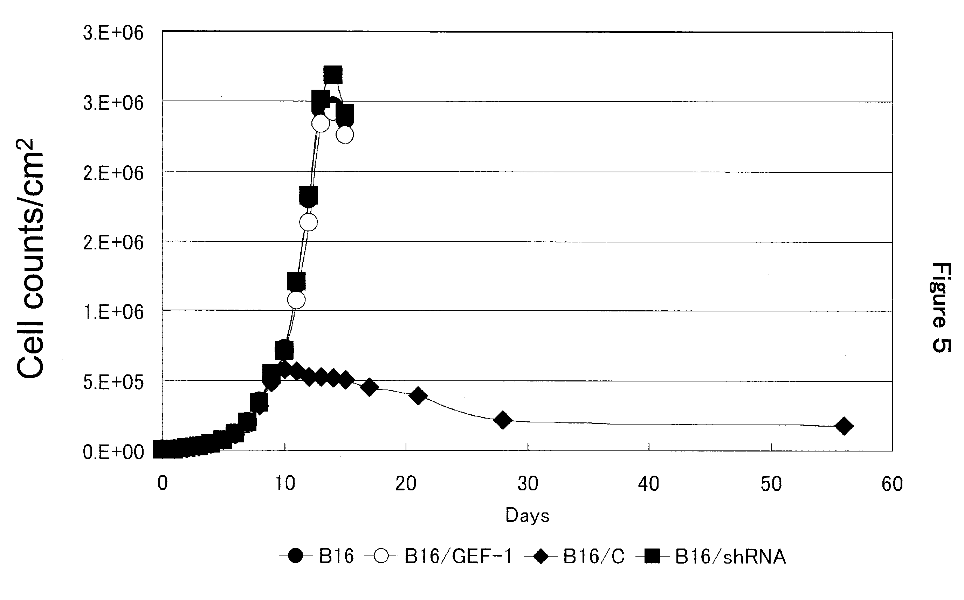

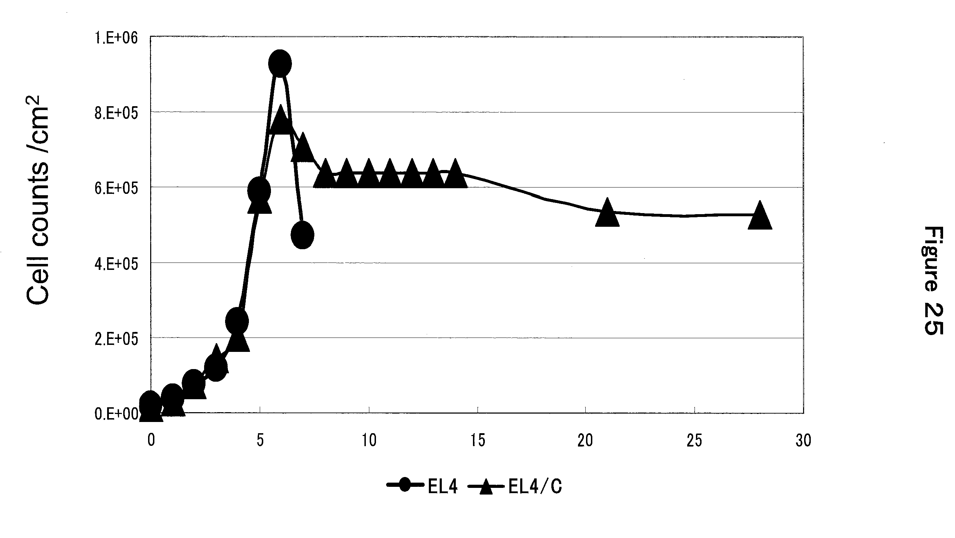

In contrast, the B16/C cells reached a confluent state and stopped cell growth when the cell density reached 5 × 10⁵/cm² (Figure 5). When seeded again at a low cell density, these B16/C cells were found to show the same growth ability as before. Likewise, the EL4 cells all died when the cell density reached about 9 × 10⁵/cm². In contrast, the EL4/C cells reached a confluent state and stopped cell growth when the cell density reached 6 × 10⁵/cm². The EL4/C cells were also found to stop cell growth in a cell density-dependent manner (Figure 25).

These results indicated that the B16/C and EL4/C cells each expressing a polypeptide covering the C region of GEF-1 had the ability to inhibit contact growth in a cell density-dependent manner, as observed in normal cells, and their cancer cell characteristics disappeared.

### (2) Cell growth and cell cycle of B16/C cells

To study polypeptides covering the C region of GEF-1 for their effect on cell cycle, the B16/C cells were analyzed for their cell cycle by FACS assay. More specifically, the cells prepared with a CycleTest^{®} Plus kit (Becton Dickinson (hereinafter referred to as BD)) were analyzed with a FACS calibur (BD).

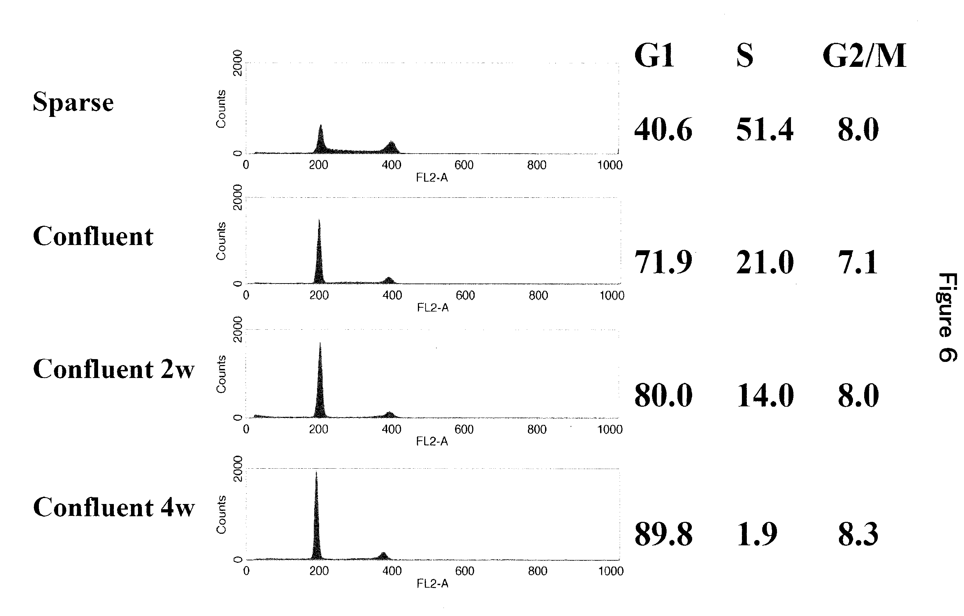

As a result, the B16/C cells were found to show G1 phase:S phase:G2/M phase =41:51:8 during growth phase at a low cell density, 72:21:7 upon reaching a confluent state, and 90:2:8 at 4 weeks after reaching a confluent state (Figure 6).

This result indicated that the B16/C cells were arrested in the G1 phase in a cell density-dependent manner and hence entered G1 arrest, so that their growth ability was significantly reduced and their cancer cell characteristics disappeared.

### 2. DNAmicroarray analysis and profiling analysis

To clarify the cell characteristics of the B16/GEF-1 cells, the B16/shRNA cells, the B16/C cells and the B16 cells, mRNAs were extracted from the respective cells and subjected to DNA microarray (Agilent Technologies) analysis. Further, analysis software KeyMolnet (IMMD Inc., Japan) was used to profile the resulting data.

The results obtained are shown in the table below.

| Cell | Rank | Pathway based on Molecule | Score |
|---|---|---|---|
| B16/GEF-1 | 1 | SMAD-mediated expression regulation | 78.3 |
| | 2 | RB/E2F-mediated expression regulation | 64.7 |
| B16/shRNA | 1 | RB/E2F-mediated expression regulation | 235.4 |
| | 2 | SMAD-mediated expression regulation | 84.8 |
| B16/C | 1 | RB/E2F-mediated expression regulation | 197.1 |
| | 2 | SMAD-mediated expression regulation | 97.2 |

This result indicated that the B16/GEF-1 cells, the B16/shRNA cells and the B16/C cells showed great changes in protein expression downstream of the SMAD pathway and the Rb/E2F pathway, as compared to their parent B 16 cells.

This result also indicated that changes in intracellular GEF-1 expression levels affected protein expression downstream of the TGF-β-SMAD signaling pathway and the Rb/E2F signaling pathway through these pathways. The TGF-β-SMAD signaling pathway is greatly involved in cell growth, differentiation and apoptosis. Rb is among the most major tumor suppressor gene products, as in the case of p53. Rb also has the function of controlling the cell cycle through binding to E2F.

Thus, the above results suggested that regulation of GEF-1 expression levels would allow regulation of the TGF-β-SMAD signaling pathway and the Rb/E2F signaling pathway, and further would allow control or inhibition of tumor cell growth.

Further, in experiments using MDCK cells, MDCK/GEF-1 cells showed reduced levels of SMAD7 and Rb expression, whereas MDCK/C cells showed increased levels of Rb expression and reduced levels of Cyclin A, D, E, p53, c-Ski, CTGF (connective tissue growth factor), HDAC4 and ATM expression. Likewise, MDCK/shRNA cells showed increased levels of c-Myc and c-Fos expression and reduced levels of Cyclin A, B, D, E and Aurora B expression.

### 3. Activity analysis of transcriptional regulatory factors

The results of the above DNA array analysis suggested that changes in GEF-1 expression levels would affect the activity of SMAD and Rb transcription factors. Then, various transcription factors were examined for their activity and other properties in the MDCK/GEF-1 cells and the MDCK/C cells.

### (1) Effects of HGF and TGF-β on SMAD transcriptional activity in MDCK/GEF-1 cells and MDCK/C cells

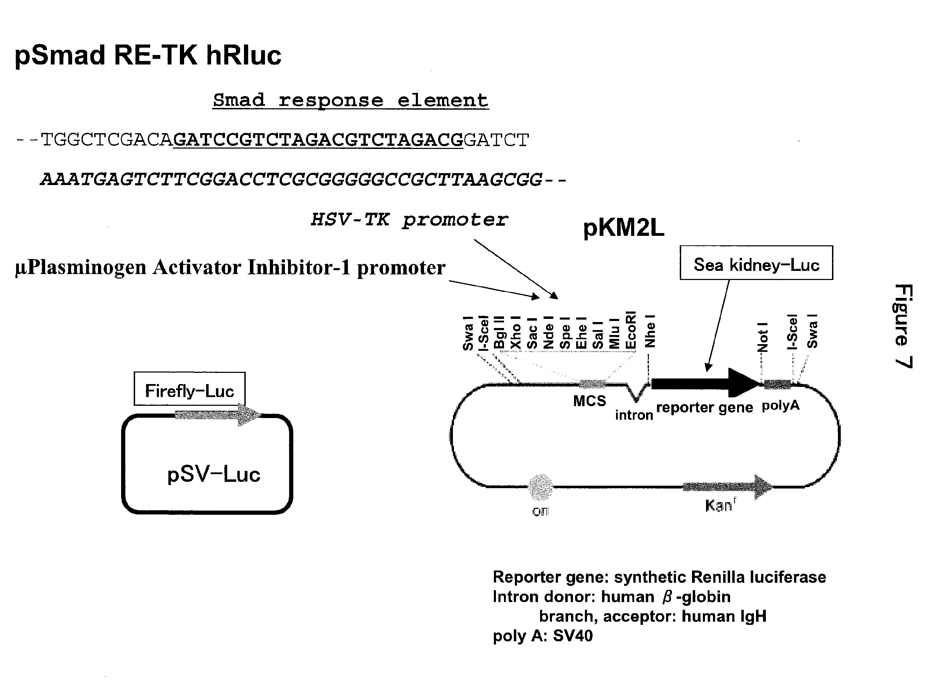

For measurement of SMAD transcriptional activity, pSmad RE-TK hRluc(F) and phRL-TK (both available from RIKEN BRC)) were used. pSmad RE-TK hRluc(F) is a vector having each response element (cis-acting DNA sequence) upstream of a TATA-like promoter and the luciferase gene as a reporter gene downstream of the TATA-like promoter. A schematic view of this vector is shown in Figure 7.

The MDCK/GEF-1 cells and the MDCK/C cells were each seeded in 48-well plates at 5.0 × 10⁴ cells per well. After 24 hours, the cells were washed with Opti-MEM medium, and an Opti-MEM medium mixture (100 µl) containing 0.5 µg of pSmad-RE-TK-hRluc(F), 0.05 µg of pGL4.13 (Promega) and 0.5 µl of Lipofectamin 2000 (Invitrogen) was added to the cells to effect gene transfer. After culture in a CO₂ incubator for 4 hours, 0.4 ml of Opti-MEM medium was added. At 24 hours after gene transfer, the medium was replaced with Opti-MEM medium containing HGF (40 ng/ml, PeproTech EC) or TGF-β (5 ng/ml, PeproTech EC). At 48 hours after gene transfer, the cells were washed with PBS and measured for luciferase activity with a system for dual-luciferase quantification.

As a result, the SMAD transcriptional activity in the MDCK/GEF-1 cells was enhanced about 570 times, as compared to MDCK cells.

The SMAD transcriptional activity in the MDCK cells was enhanced about twice upon HGF stimulation, whereas no further enhancement was observed in the MDCK/GEF-1 cells. Moreover, upon TGF-β stimulation, the SMAD transcriptional activity was enhanced about 11 times in the MDCK cells and about 6.4 times in the MDCK/GEF-1 cells.

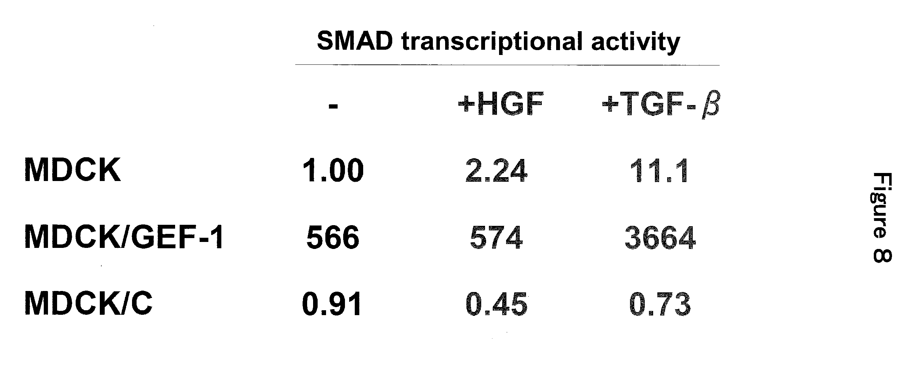

In contrast, the SMAD transcriptional activity in the MDCK/C cells was inhibited as compared to the SMAD transcriptional activity in the MDCK cells. Further, even upon HGF stimulation or TGF-β stimulation, the MDCK/C cells showed no enhancement of the SMAD transcriptional activity (Figure 8).

This result indicated that upon expression of polypeptides covering the C region of GEF-1, the SMAD transcriptional activity in MDCK cells was inhibited and was also no longer affected by HGF stimulation or TGF-β stimulation.

### (2) Transcription factor activity in MDCK mutant cells

In the above study, the SMAD transcriptional activity was enhanced in the MDCK/GEF-1 cells and inhibited in the MDCK/C cells, as compared to the MDCK cells. Then, further study was conducted to measure the activity of transcription factors related to various signaling pathways in the MDCK/GEF-1 cells and the MDCK/C cells.

For measurement of the transcriptional activity of various transcription factors including Rb and p53, a BD Pathway Profiling Luciferase System (BD) and a BD Pathway Profiling Luciferase System 4 (BD) were used.

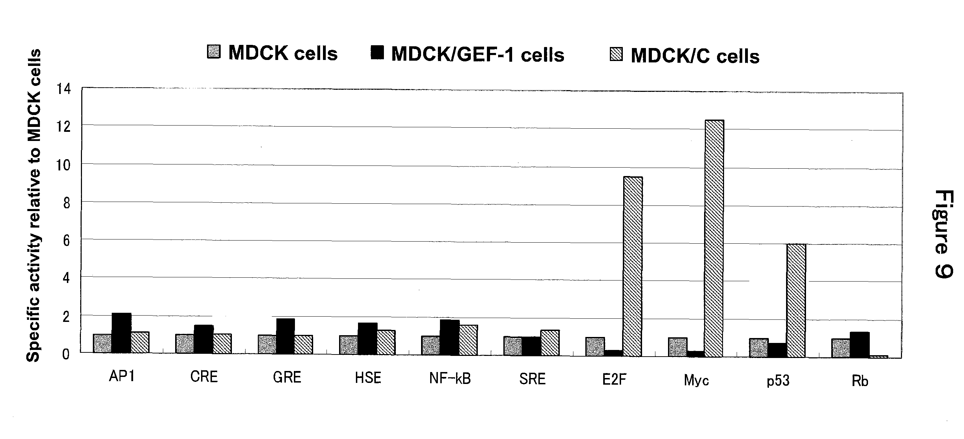

As a result, the respective cells were controlled to enhance or inhibit the expression of proteins downstream of SMAD. Particularly in the MDCK/C cells, the p53 transcriptional activity was enhanced while the Rb transcriptional activity was inhibited (Figure 9). A reduction in the Rb transcriptional activity is indicative of an increase in E2F/Rb complex levels.

### (3) Transcription factor activity in B16 mutant cells

The same procedure as used above for MDCK cells was repeated to measure the activity of transcription factors related to various signaling pathways in the B16/GEF-1 cells and the B16/C cells.

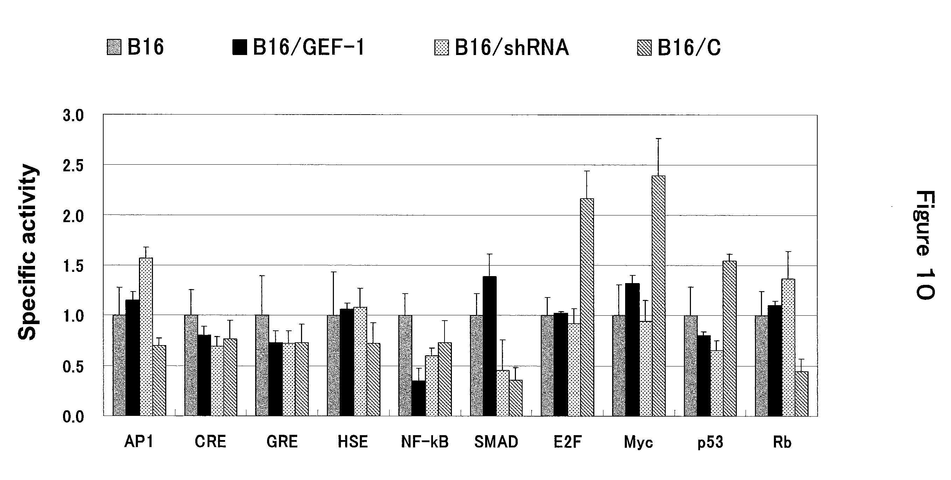

As a result, the SMAD transcriptional activity was enhanced in the B16/GEF-1 cells, whereas the SMAD transcriptional activity and the Rb transcriptional activity were inhibited and the p53 transcriptional activity was enhanced in the B16/C cells (Figure 10). A reduction in the Rb transcriptional activity is indicative of an increase in E2F/Rb complex levels.

As shown above, the MDCK/C cells and the B16/C cells both showed reduced Rb transcriptional activity and enhanced p53 transcriptional activity. A reduction in the Rb transcriptional activity is indicative of an increase in Rb/E2F complex formation, which promotes cell cycle arrest in the G1 phase. In addition, enhanced p53 transcriptional activity induces p21 expression and, in turn, p21 inhibits Rb phosphorylation, whereby the cell cycle is arrested in the G1 phase.

Thus, the above results indicated that upon expression of polypeptides covering the C region of GEF-1, E2F/Rb complex formation and p53 expression were enhanced, which in turn would arrest the cell cycle in the G1 phase and hence inhibit cell growth.

### (4) PAI-1 promoter activity in B16 mutant cells

µPlasminogen activator inhibitor-1 (PAI-1) is known to be induced to express itself by the SMAD transcription factor. Based on this knowledge, TGF-β-SMAD signaling levels in the MDCK/GEF-1 cells and the MDCK/C cells were determined by measuring the promoter activity of PAI-1. The promoter activity of PAI-1 was measured in the same manner as shown in (1) above by measuring luciferase activity with a system for dual-luciferase quantification. A schematic view of a PAI-1 promoter-carrying vector is shown in Figure 7.

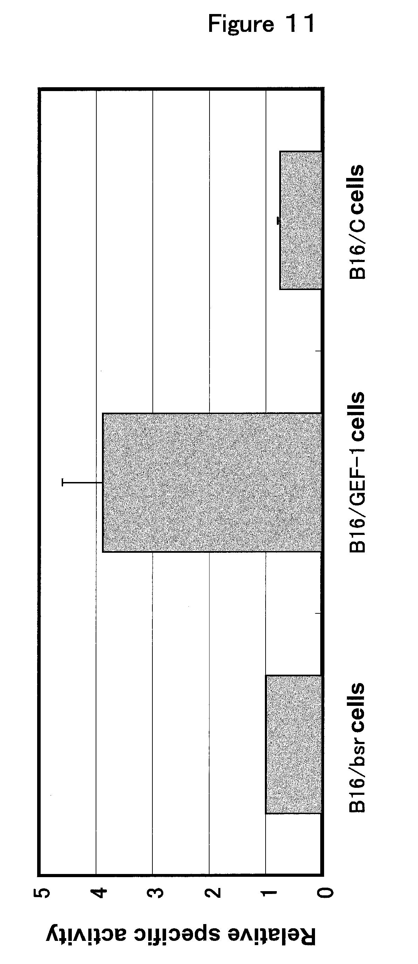

As a result, the PAI-1 promoter activity was increased about four times in the B16/GEF-1 cells and reduced to 3/4 in the B16/C cells, as compared to the B16 cells (Figure 11).

This result indicated that TGF-β-SMAD signaling was enhanced about four times in the B16/GEF-1 cells and inhibited to 3/4 in the B16/C cells, as compared to the B16 cells.

### 4. Measurement of TGF-β expression levels

TGF-β-SMAD signals are transmitted to intracellular SMAD molecules through binding of extracellular TGF-β to its receptors on the cell surface. Then, the SMAD molecules migrate into the nuclei and bind to DNA, thereby enhancing the transcriptional activity of proteins corresponding to TGF-β-SMAD signaling. Moreover, TGF-β molecules per se are also protein molecules whose expression is induced by TGF-β-SMAD signaling, so that enhanced TGF-β-SMAD signaling also enhances TGF-β protein synthesis and extracellular release. Thus, TGF-β levels in a cell culture are proportional to TGF-β-SMAD signaling levels in the cells.

Then, TGF-β expression levels in a cell culture were measured and analyzed with a Quantikine^{®} TGF-β ELISA assay kit (R&D Systems, Inc.).

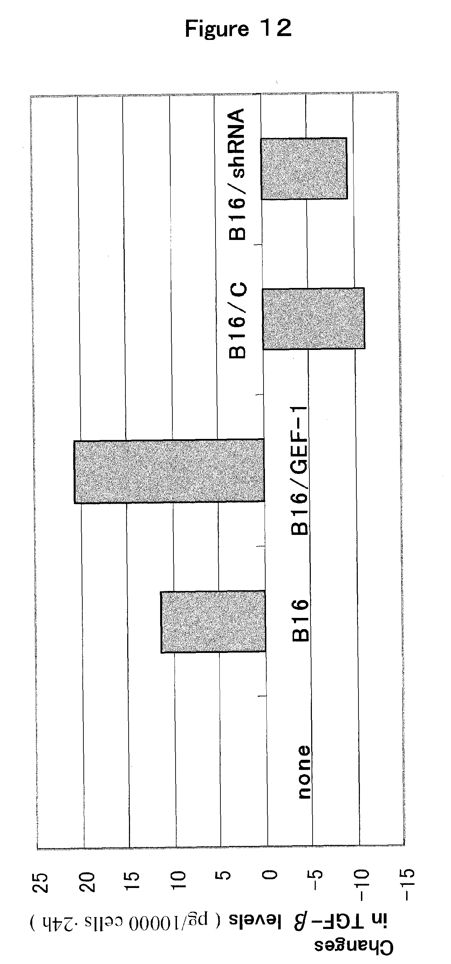

As a result, protein levels of TGF-β were increased in cultures of the B16 cells and the B16/GEF-1 cells, whereas they were reduced in cultures of the B16/C cells and the B16/shRNA cells (Figure 12).

Thus, TGF-β-SMAD signaling would have been enhanced in the B16 cells and the B16/GEF-1 cells and would have been inhibited in the B16/C cells. TGF-β levels in the B16/GEF-1 cell culture were about twice higher than those of the B16 cell culture, thus suggesting that TGF-β-SMAD signaling levels in the B16/GEF-1 cells would be enhanced about twice, as compared to the B16 cells.

### 5. Measurement of in vitro cell growth ability in soft agar

Anchorage-independent growth ability is one of the cancer cell characteristics. Normal (non-cancer) cells can grow only in an anchorage-dependent manner and hence cannot grow in soft agar medium. In contrast, cancer cells can grow in soft agar medium to form colonies. Then, the respective cells were measured for their growth ability in soft agar medium.

A cell culture solution (1.5 ml), in which agarose (low gelling temperature agarose) was dissolved at a concentration of 0.5% (w/v), was added to a 35 mm dish, cooled and gelled. Onto the resulting gel, a 0.33% agarose/cell culture medium containing 5.0 × 10³ cells was added in a volume of 1.5 ml. After gelling, the cells were cultured in a CO₂ incubator for 2 weeks. A 0.005% (w/v) aqueous solution of crystal violet (0.5 ml) was added onto the gel and incubated overnight to stain the cells. The 35 mm dish was scanned with a scanner, and colony counts were measured from the resulting image using image analysis software.

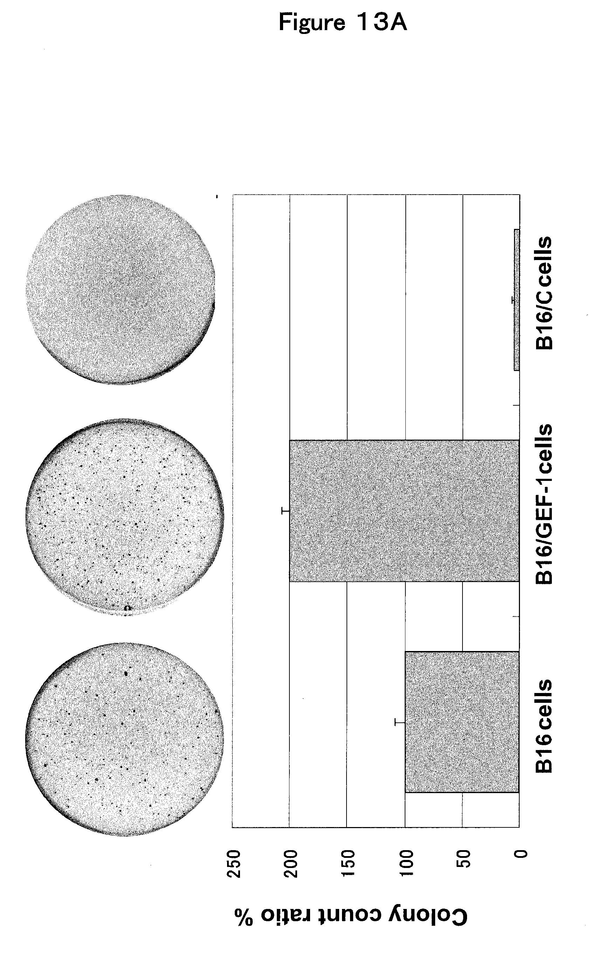

As a result, the growth ability of the B16/GEF-1 cells in soft agar medium was enhanced about twice, as compared to the B16 cells, whereas the growth ability of the B16/C cells in soft agar medium was reduced to less than 5% of that of the B16 cells (Figure 13A).

In the same manner, a stably GEF-1/C protein-expressing cell line of mouse lung cancer-derived LLC cells (LLC/C cells), a stably GEF-1/C protein-expressing cell line of human pancreatic cancer-derived cell line PT45 cells (PT45/C cells), and a stably GEF-1/C protein-expressing cell line of human colorectal cancer-derived cell line COLO205 cells (COLO205/C cells) were measured for their growth ability in soft agar medium.

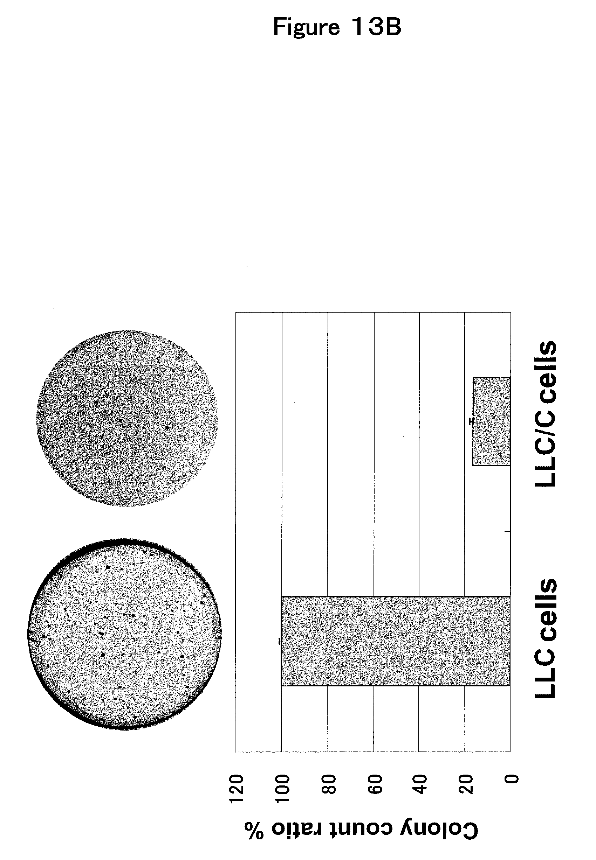

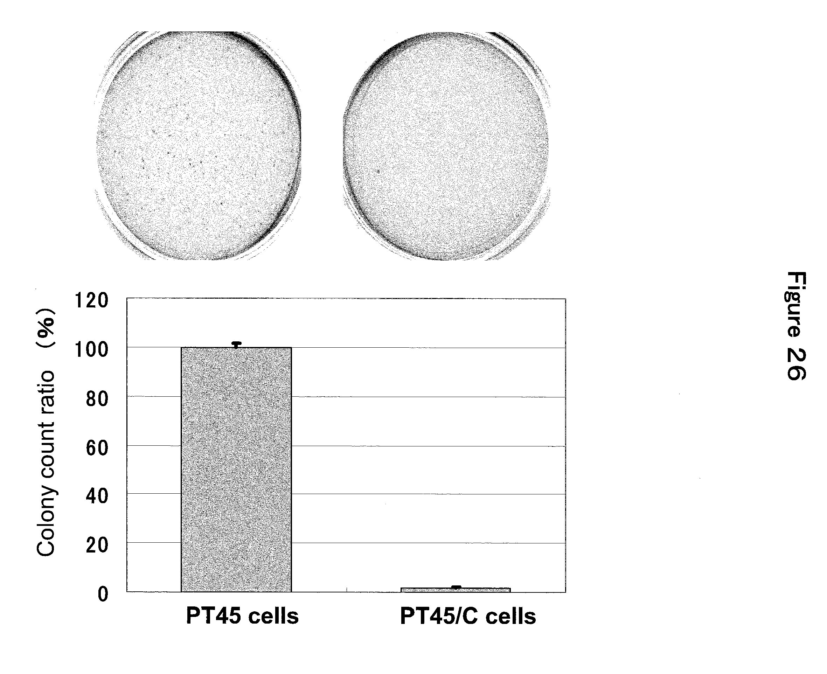

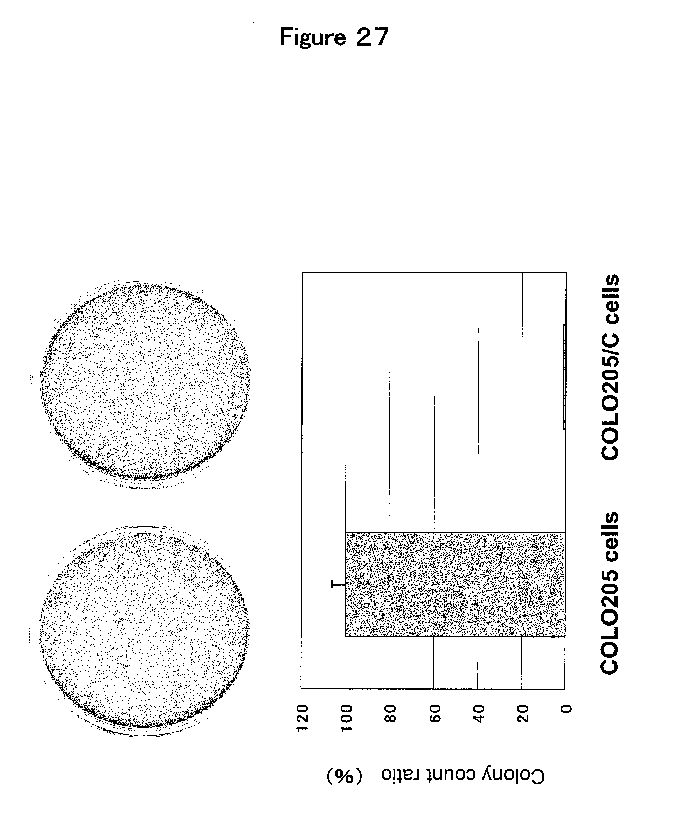

As a result, the growth ability in soft agar medium was reduced to less than 20% in the LLC/C cells, as compared to their parent LLC cells (Figure 13B). Likewise, the growth ability in soft agar medium was reduced to less than 5% in the PT45/C cells, as compared to their parent PT45 cells (Figure 26), while the growth ability in soft agar medium was reduced to less than 1% in the COLO205/C cells, as compared to their parent COLO205 cells (Figure 27).

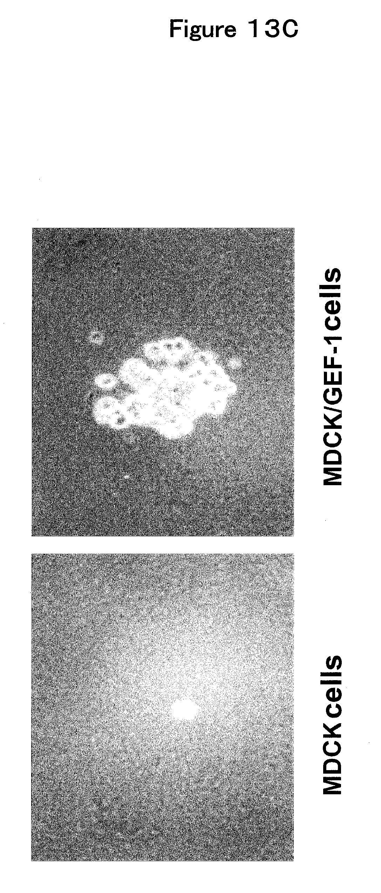

Next, MDCK cells were measured for their growth ability in soft agar medium. MDCK cells, which are derived from canine renal epithelial cells, are not cancerous and hence retain a normal cell-like morphology. Thus, MDCK cells cannot grow in soft agar medium. However, a stably GEF-1 protein-expressing cell line of MDCK cells (MDCK/GEF-1 cells) acquired the growth ability in soft agar medium (Figure 13C).

These results indicated that when GEF-1 was expressed in B16 cells and LLC cells, both of which are cancer cells, their growth ability in soft agar medium was enhanced, whereas GEF-1 expression in MDCK cells, which are not cancer cells, allowed the MDCK cells to acquire the growth ability in soft agar medium, which is not inherent to the MDCK cells. On the other hand, when polypeptides covering the C region of GEF-1 were expressed in B16 cells, LLC cells, PT45/C cells and COLO205/C cells, there was a significant reduction in their tumor growth ability in soft agar medium.

Namely, it was indicated that when polypeptides covering the C region of GEF-1 were expressed in B16 cells, LLC cells, PT45/C cells and COLO205/C cells, these cells lost their anchorage-independent growth ability, which is among the cancer cell characteristics.

### 6. In vivo tumorigenicity test

The respective cells (B16 cells, B16/GEF-1 cells, B16/C cells, B16/shRNA cells) were each transplanted subcutaneously into C57BL/6 mice, and the tumor volume was measured at intervals of a week.

C57BL/6 mice were shaved and inoculated subcutaneously with the respective cells (2.0 × 10⁶ cells/0.2 ml PBS). The long and short axes of tumor were measured at intervals of a week to calculate the tumor volume (= long axis × (short axis)² × π/6).

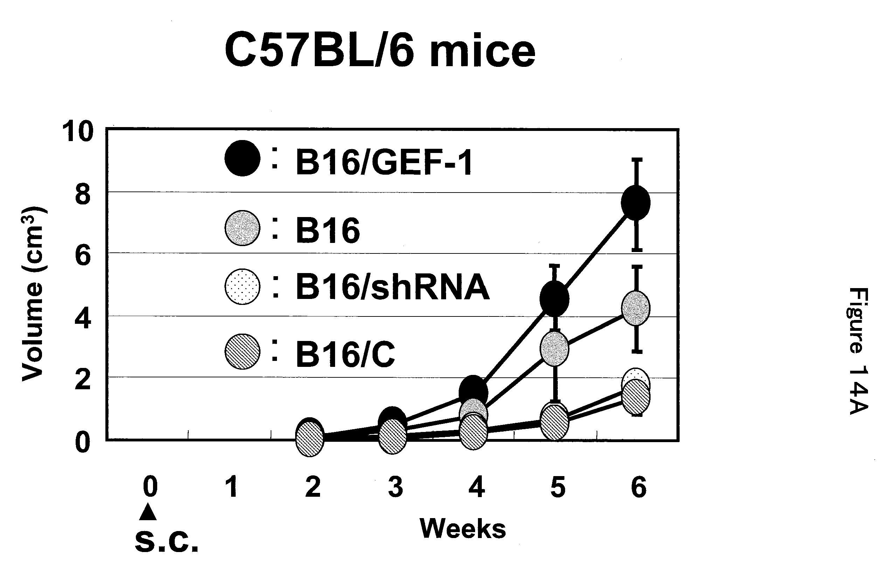

As a result, the B16/GEF-1 cells showed about 2-fold enhancement of their subcutaneous tumorigenicity in C57BL/6 mice, as compared to the B16 cells. In contrast, the B16/C cells and the B16/shRNA cells both showed a reduction in their tumorigenicity (Figure 14A).

This result indicated that the B16/GEF-1 cells had higher *in vivo* tumorigenicity than the B16 cells. In contrast, the B16/C cells and the B16/shRNA cells were both found to have suppressed tumorigenicity.

Further, to measure MDCK cells and MDCK/GEF-1 cells for their *in vivo* tumorigenicity, these cells (1.0 × 10⁶ cells/0.2 ml PBS) were transplanted subcutaneously into nude mice. At 4 weeks after transplantation, the mice were euthanized with carbon dioxide gas, and tumor was then excised from each mouse and measured for its wet weight.

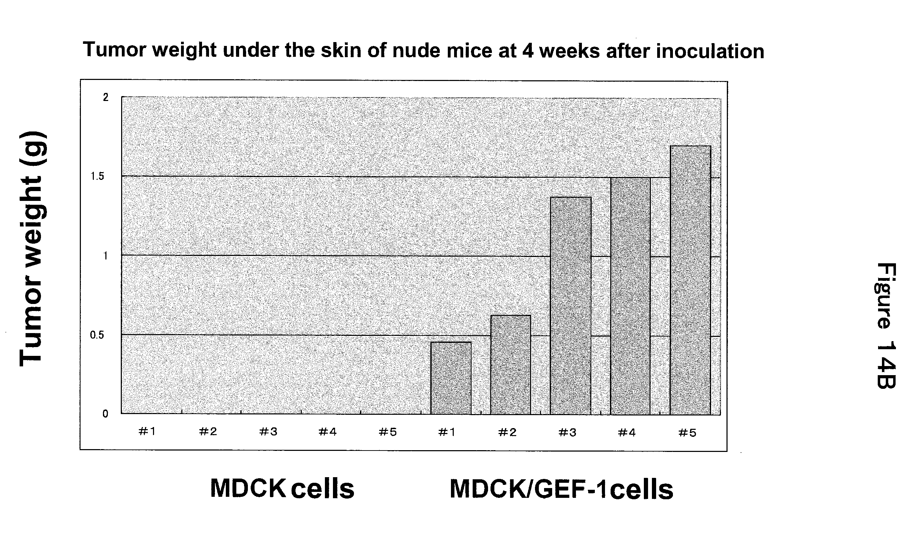

As a result, the MDCK cells formed no tumor under the skin of nude mice because they were not cancerous and retained a normal cell-like morphology. In contrast, the MDCK/GEF-1 cells formed tumor under the skin of nude mice (Figure 14B).

In the above *in vivo* tumorigenicity test, the B16/GEF-1 cells and the MDCK/GEF-1 cells showed enhancement of their tumor growth ability. On the other hand, the B16/C cells and the B16/shRNA cells showed a reduction in their tumor growth ability. These results indicated that increased intracellular levels of GEF-1 protein enhanced tumor growth ability, whereas suppressed levels of GEF-1 protein inhibited tumor growth ability. Thus, it was indicated that when controlling the expression and functions of GEF-1, the tumor growth ability of cancer cells can be controlled.

Moreover, upon expression of polypeptides covering the C region of GEF-1, B16 cells and LLC cells showed a reduction in their tumor growth ability, indicating that the polypeptides of the present invention inhibited tumor growth.

### Example 4

### Preparation of GEF-1-related small molecules

The results of Example 3 described above indicated that tumor growth can be inhibited when the expression levels of GEF-1 are suppressed or when the GEF-1/C protein is expressed to inhibit GEF-1 functions.

The above B16/shRNA cells are derived from B16 cells by introducing an interference-inducing vector to suppress the expression levels of GEF-1 by RNA interference effects. Likewise, the B16/C cells are derived from B16 cells by introducing a GEF-1/C protein expression vector to induce the expression of GEF-1/C protein. The RNA interference-inducing vector and the GEF-1/C protein expression vector are both macromolecules and hence are difficult to introduce into cancer cells in the human body and are not easy to use in clinical practice.

However, small molecule siRNAs capable of inducing RNA interference and GEF-1/C region constituent oligopeptides are easier to introduce or deliver (DDS) into cancer cells in the human body and can also be expected to be used in clinical practice.

Then, further study was conducted to investigate a method for tumor growth inhibition by GEF-1-related small molecules targeting GEF-1, i.e., siRNAs and GEF-1/C region constituent oligopeptides.

### 1. siRNAs

For use as siRNAs targeting GEF-1 (GEF-1-siRNAs), siRNAs (stealth RNAi) against mouse Hgs (NM_008244) were synthesized by entrusting their synthesis to Invitrogen. The nucleotide sequences of six siRNAs (siRNA#1 to #6) and siRNA#3-scramble are shown below. It should be noted that siRNA#3-scramble is intended to mean scramble siRNA of siRNA#3, i.e., a duplex RNA whose nucleotide sequence is different from that of siRNA#3, but whose ratio of constituent nucleotides is equal to that of siRNA#3, and whose homologous sequence is not found in cells or animals to be used. siRNA#3-scramble was used as a control for siRNA#3.
siRNA #1
NM_008244_stealth_1064
Sense strand: 5'-ACCUCCACGUCUCUGUCGAUCGAUA (SEQ ID NO: 30)
Antisense strand: 5'- UAUCGAUCGACAGAGACGUGGAGGU (SEQ ID NO: 31)
siRNA #2
NM_008244_stealth_677
Sense strand: 5'-GGACGAUACUCGUCGACUUGUUCUU (SEQ ID NO: 32)
Antisense strand: 5'-AAGAACAAGUCGACGAGUAUCGUCC (SEQ ID NO: 33)
siRNA #3
NM_008244_stealth_99
Sense strand: 5'-UAGAAUGGACUCCCAGUCUGACUCC (SEQ ID NO: 34)
Antisense strand: 5'- GGAGUCAGACUGGGAGUCCAUUCUA (SEQ ID NO: 35)
siRNA #3 scramble
NM_008244_stealth_scramble_99
Sense strand: 5'-UAGCAAUAGGCACUCCGACUGUUCC (SEQ ID NO: 36)
Antisense strand: 5'- GGAACAGUCGGAGUGCCUAUUGCUA (SEQ ID NO: 37)
siRNA #4
NM_008244_stealth_74
Sense strand: 5'-AACAGAAGCUGGCUGGUGGCUUUGU (SEQ ID NO: 38)
Antisense strand: 5'-ACAAAGCCACCAGCCAGCUUCUGUU (SEQ ID NO: 39)
siRNA #5
NM_008244_stealth_255
Sense strand: S'-AUCAUGGACUGUCUGGCCACAGUUC (SEQ ID NO: 40)
Antisense strand: 5'- GAACUGUGGCCAGACAGUCCAUGAU (SEQ ID NO: 41)
siRNA #6
NM_008244_stealth_1026
Sense strand: 5'-UUUCUUCUCCCAGUAGUUCCGGUUG (SEQ ID NO: 42)
Antisense strand: 5'- CAACCGGAACUACUGGGAGAAGAAA (SEQ ID NO: 43)

### 2. GEF-1/C constituent oligopeptides

The GEF-1/C constituent oligopeptides used in this example were synthesized by entrusting their synthesis to Hayashi-Kasei Co., Ltd., Japan.

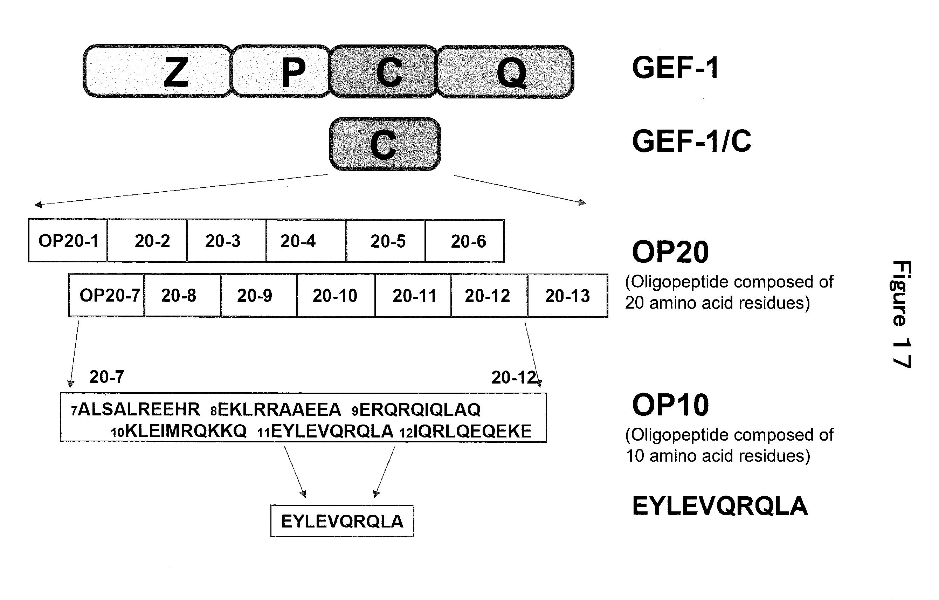

A schematic view of the GEF-1/C constituent oligopeptides is shown in Figure 17. The amino acid sequences of the synthesized oligopeptides are also shown below. Among the synthesized oligopeptides shown below, r9-OP10-11 is a peptide composed of OP10-11 linked to a cell membrane permeable peptide (rrrrrrrrrGPG (where r represents D-arginine) (SEQ ID NO: 66)). Although the GEF-1/C constituent oligopeptides shown below are of mouse origin, they differ from oligopeptides of human origin only in T (G in human) at position 16 from the N-terminal end of OP20-2 (at position 6 from the N-terminal end of OP-20-3 or OP 10-3), and the other residues are common to the mouse and human oligopeptides.
OP20-1: MKSNHMRGRSITNDSAVLSL (SEQ ID NO: 44)
OP20-2: ITNDSAVLSLFQSINTMHPQ (SEQ ID NO: 45)
OP20-3: FQSINTMHPQLLELLNQLDE (SEQ ID NO: 46)
OP20-4: LLELLNQLDERRLYYEGLQD (SEQ ID NO: 47)
OP20-5: RRLYYEGLQDKLAQIRDARG (SEQ ID NO: 48)
OP20-6: KLAQIRDARGALSALREEHR (SEQ ID NO: 49)
OP20-7: ALSALREEHREKLRRAAEEA (SEQ ID NO: 50)
OP20-8: EKLRRAAEEAERQRQIQLAQ (SEQ ID NO: 51)
OP20-9: ERQRQIQLAQKLEIMRQKKQ (SEQ ID NO: 52)
OP20-10: KLEIMRQKKQEYLEVQRQLA (SEQ ID NO: 53)
OP20-11: EYLEVQRQLAIQRLQEQEKE (SEQ ID NO: 54)
OP20-12: IQRLQEQEKERQMRLEQQKQ (SEQ ID NO: 55)
OP20-13: RQMRLEQQKQTVQMRAQMPA (SEQ ID NO: 56)
OP10-1: MGRGSGTFER (SEQ ID NO: 57)
OP10-3: FQSINTMHPQ (SEQ ID NO: 58)
OP10-6: KLAQIRDARG (SEQ ID NO: 59)
OP10-7: ALSALREEHR (SEQ ID NO: 60)
OP10-8: EKLRRAAEEA (SEQ ID NO: 61)
OP10-9: ERQRQIQLAQ (SEQ ID NO: 62)
OP10-10: KLEIMRQKKQ (SEQ ID NO: 63)
OP10-11: EYLEVQRQLA(SEQ ID NO: 64)
OP10-12: IQRLQEQEKE (SEQ ID NO: 65)
r9-OP10-11: rrrrrrrrrGPGEYLEVQRQLA (SEQ ID NO: 67)

### Example 5

### Inhibition of angio genesis and tumor growth by GEF-1-related small molecules

### 1. siRNA-induced inhibition of angiogenesis and tumor growth

### (1) Study of siRNA-induced angiogenesis inhibition

To confirm the *in vitro* anti-angiogenic effect of the GEF-1-siRNAs, a tube formation inhibition test was conducted using vascular endothelial KOP2.16 cells.

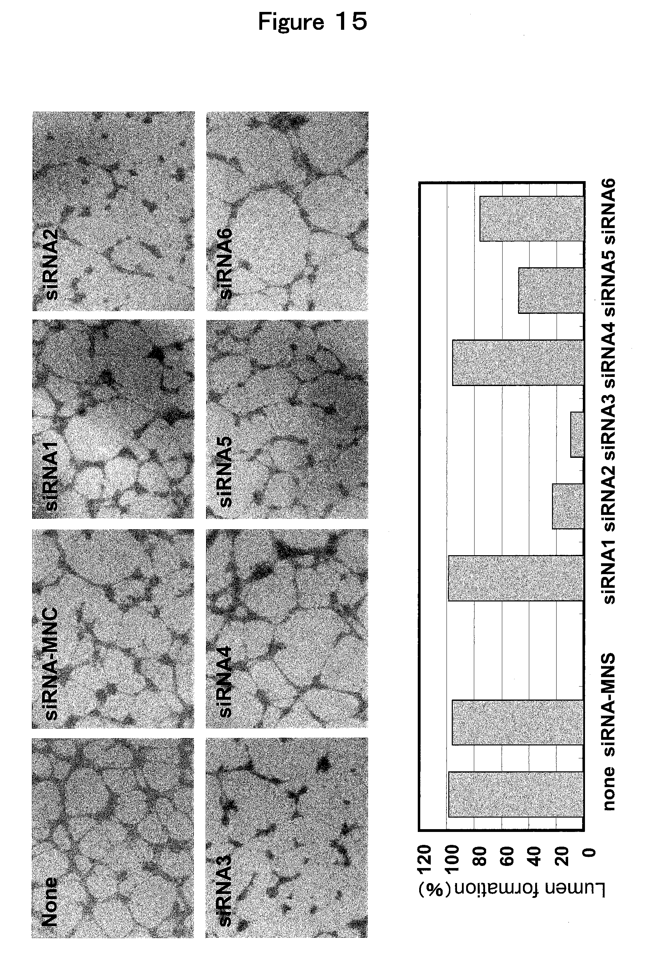

Each siRNA (40 nmol) and lipofectamine 2000 (10 µl) were mixed and then diluted to 1.0 ml with Opti-MEM I medium, and added to KOP2.16 cells in a 35 mm dish, which had been washed with Opti-MEM I medium. After 4 hours, 20% FBS-containing Opti-MEM I medium (1.0 ml) was added to the dish. After 48 hours, siRNA-transfected cells were collected and counted, and then suspended in Opti-MEM I medium and seeded again on Matrigel. After 18 to 48 hours, tube formation in the cells was observed. As negative controls, PBS ("None" in Figure 15) and Stealth RNA Negative Control Medium GC Duplex (Invitrogen) ("siRNA-MNC" in Figure 15) were used.

As a result, among the six siRNAs, siRNA#2 and siRNA#3 showed a significant inhibitory effect on tube formation (Figure 15).

This result indicated that the above siRNAs showed an anti-angiogenic effect through inhibition of GEF-1 expression.

### (2) Study of siRNA-induced tumor growth inhibition

Further, siRNA#3 showing the highest inhibitory effect on tube formation in (1) above was examined for its anti-tumor growth effect.

B16 cells were inoculated subcutaneously into nude mice. After about a week when tumor reached about 5 mm diameter (about 100 m³), an siRNA#3/atelocollagen mixture (0.2 ml) was injected near the tumor. Subsequently, the same siRNA#3/atelocollagen mixture was further injected twice near the tumor at intervals of a week.

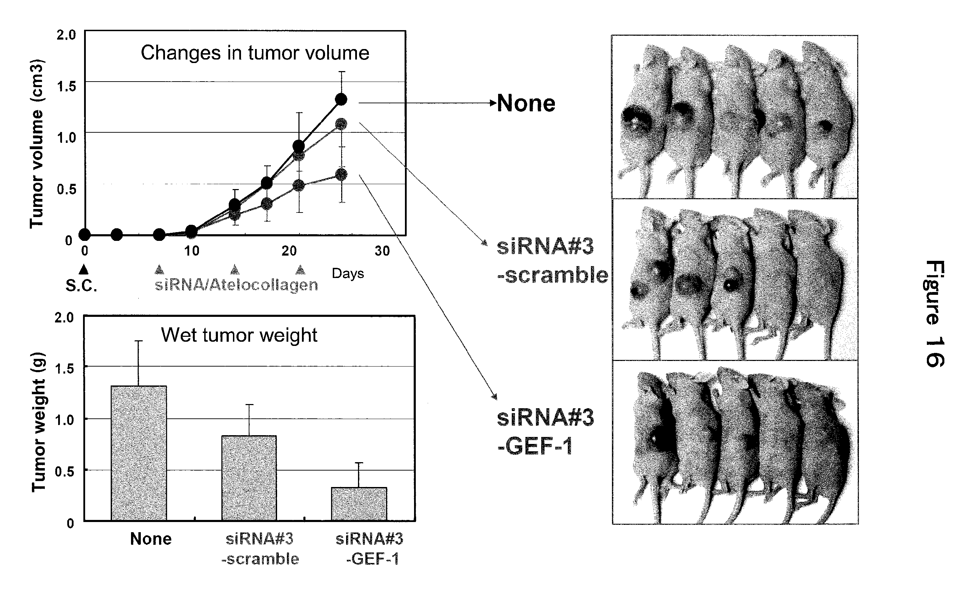

As a result, siRNA#3 was able to suppress the tumor volume and the tumor weight to 50% or less, as compared to the case where PBS or scramble siRNA of siRNA#3 (siRNA#3-scramble) was administered (Figure 16).

This result indicated that the above siRNA showed an *in vivo* anti-tumor growth effect through inhibition of GEF-1 expression.

### 2. Inhibition of angiogenesis and tumor growth by GEF-1/C constituent oligopeptides

### (1) Effect of GEF-1/C constituent oligopeptides on SMAD transcriptional activity

First, the GEF-1/C constituent oligopeptides were studied for their effect on the SMAD transcriptional activity in TGF-β-SMAD signaling. The TGF-β-SMAD signaling pathway is greatly involved in angiogenesis and cancer cell metastasis.

Each GEF-1/C constituent oligopeptide (2.5 nmol) and a peptide transporter Wr-T (0.2 nmol) were mixed and then diluted to 0.1 ml with PBS, and mixed with a DNA solution for SMAD transcriptional activity testing (0.1 ml). To the respective cells (B16 cells and COLO205 cells) in a 48-well plate, which had been washed with Opti-MEM I medium, the mixture thus prepared was added in a volume of 0.2 ml per well. After 4 hours, 20% FBS/Opti-MEM I medium (0.2 ml) was added to each well. After 48 hours, the respective cells were lysed and the luciferase activity in each lysate was measured.

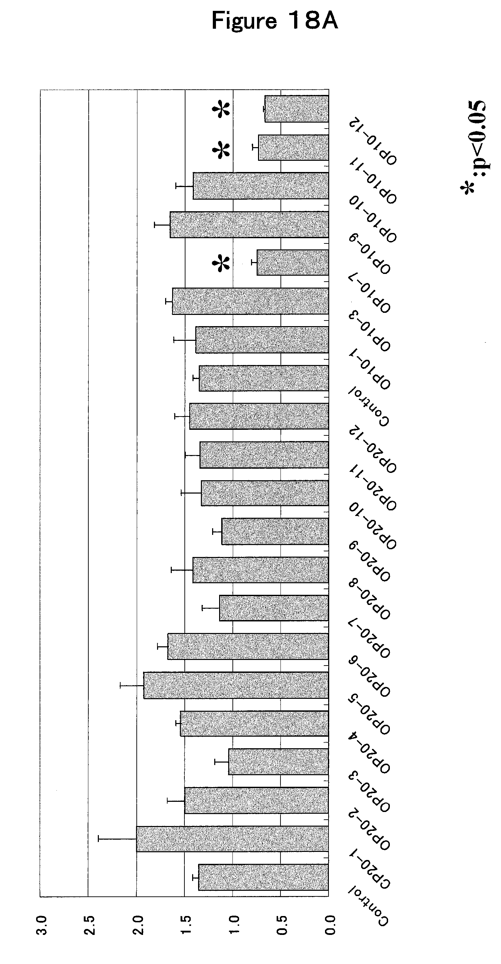

As a result, in the B16 cells, the GEF-1/C constituent oligopeptides OP10-7, OP10-11 and OP 10-12 caused about 50% inhibition of the SMAD transcriptional activity (Figure 18 A).

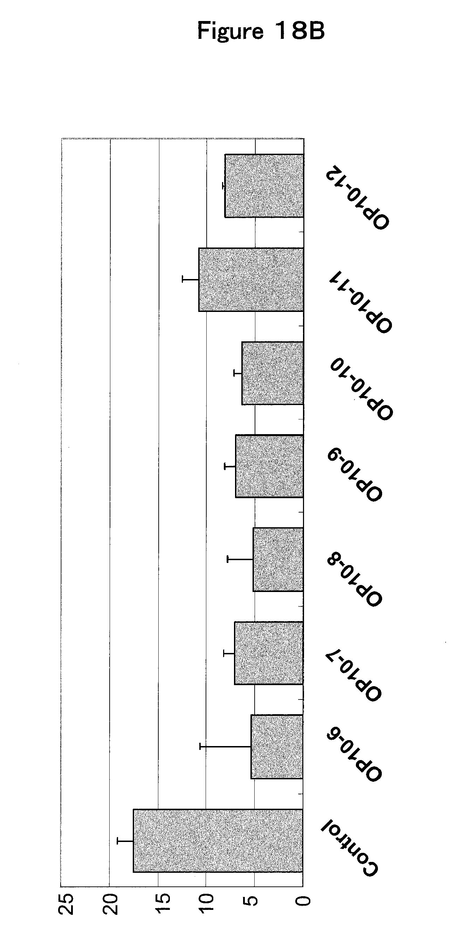

Likewise, in the human colorectal cancer COLO205 cells, OP10-6 to OP10-12 caused about 35% to 70% inhibition of the SMAD transcriptional activity (Figure 18B).

### (2) Study of GEF-1/C constituent oligopeptide-induced angiogenesis inhibition

To confirm the inhibitory effect of the GEF-1/C constituent oligopeptides on *in vitro* angiogenesis, a tube formation inhibition test was conducted using vascular endothelial KOP2.16 cells.

Each GEF-1/C constituent oligopeptide (2.5 nmol) and a peptide transporter Wr-T (0.2 nmol) were mixed and then diluted to 0.1 ml with Opti-MEM I medium, and added to KOP2.16 cells in a 48-well plate, which had been washed with Opti-MEM I medium. After 2 hours, 10% FBS-containing Opti-MEM I medium (0.4 ml) was added to each well. After 48 hours, GEF-1/C constituent oligopeptide-transfected cells were collected and counted, and then suspended in Opti-MEM I medium. These cells were seeded on Matrigel.

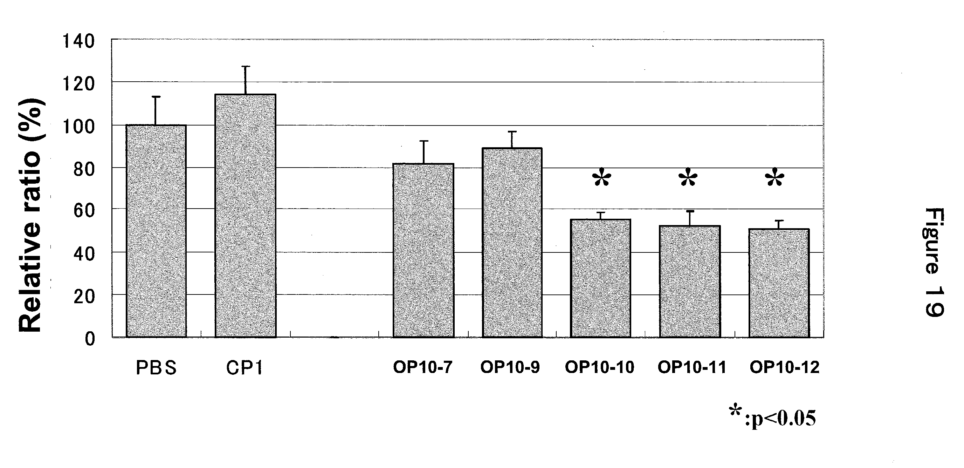

As a result, OP10-10 to OP10-12 caused about 50% inhibition of tube formation (Figure 19).

This result indicated that the above GEF-1/C constituent oligopeptides showed an anti-angiogenic effect.

### (3) Study of GEF-1/C constituent oligopeptides for their in vitro anti-tumor growth effect

The GEF-1/C constituent oligopeptides were studied for their inhibitory effect on the growth of various tumor cells (B16 cells, HeLa cells, COLO205 cells, KATO III cells, A549 cells and PT45 cells) in soft agar medium.

In the same manner as shown in (2) above, the GEF-1/C constituent oligopeptides were introduced into various tumor cells and these cells were seeded together with a 0.33% soft agar medium.

The results obtained are shown below for the respective cells.

### a) Mouse melanoma B16 cells

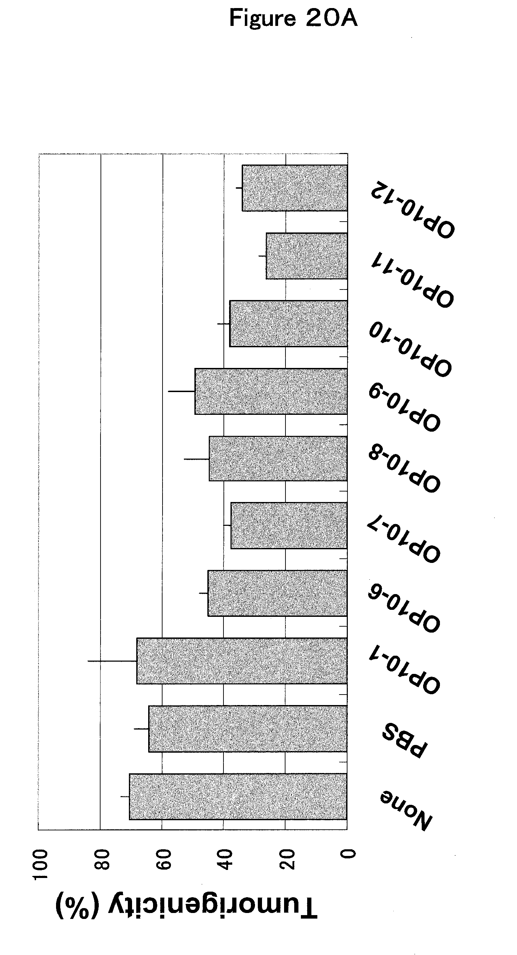

OP10-6 to OP10-12 caused about 30% or more inhibition of the B16 cells' growth ability in soft agar medium. In particular, OP10-11 caused about 60% inhibition (Figure 20A).

### b) Human uterine cervical cancer-derived HeLa cells

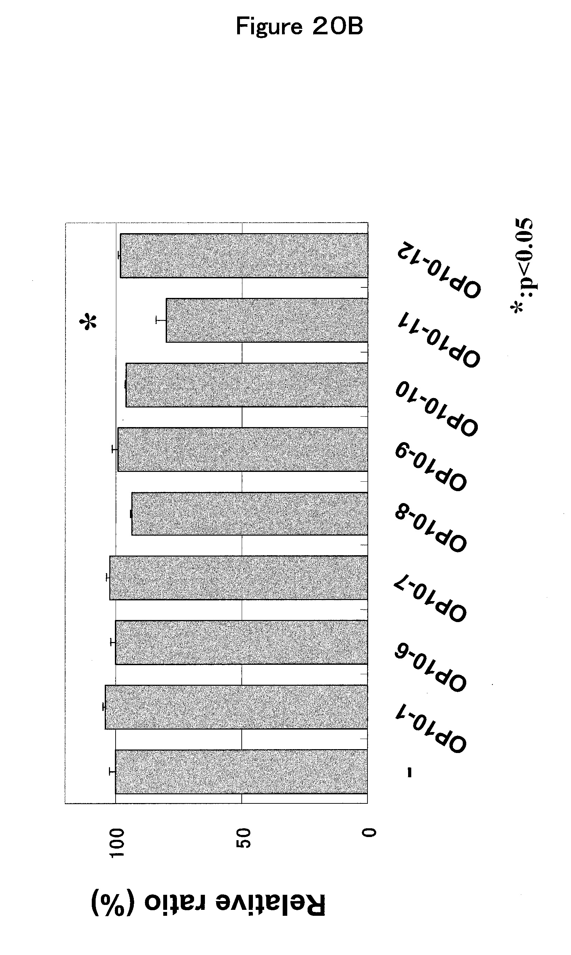

OP10-11 caused about 20% inhibition of the HeLa cells' growth ability in soft agar medium (Figure 20B).

### c) Human colorectal cancer-derived COLO205 cells

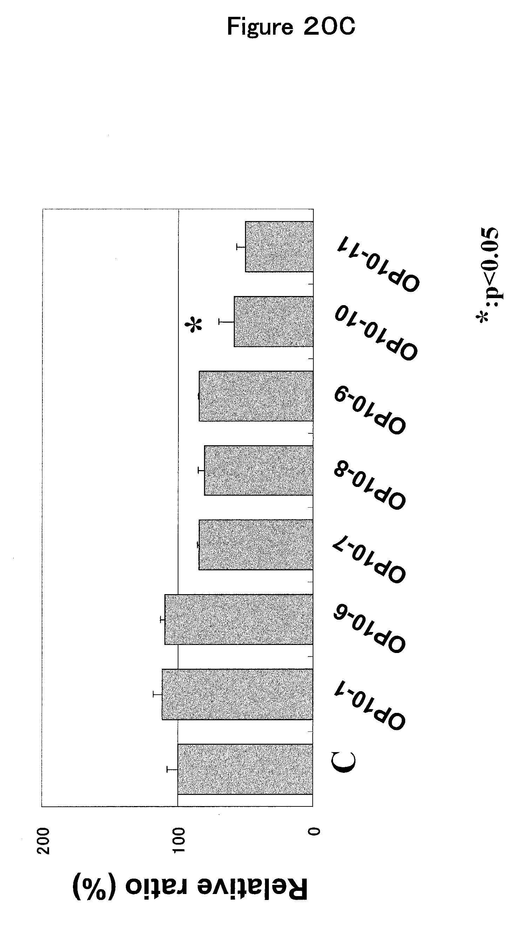

OP10-10 and OP10-11 caused about 50% inhibition of the COLO205 cells' growth ability in soft agar medium (Figure 20C).

### d) Human gastric cancer-derived KATO III cells

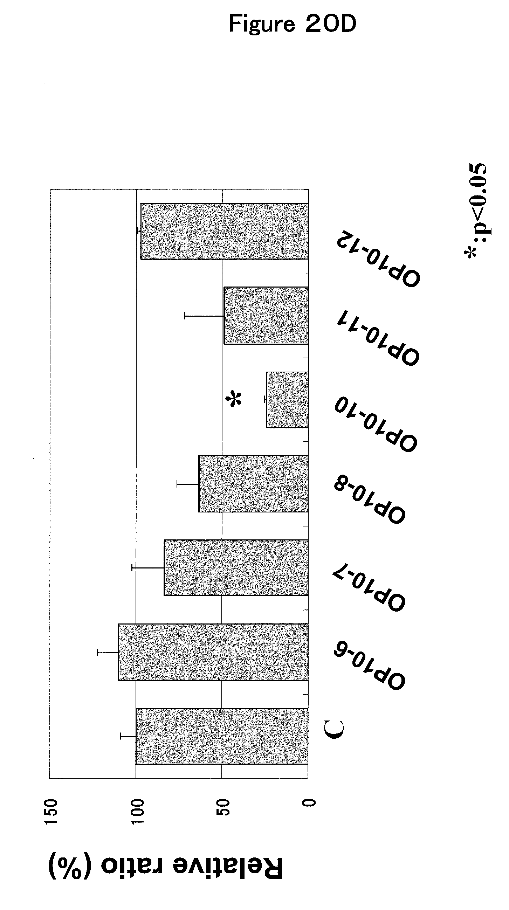

OP10-10 and OP10-11 caused about 50% to 70% inhibition of the KATO III cells' growth ability in soft agar medium (Figure 20D).

### e) Human lung cancer-derived A549 cells

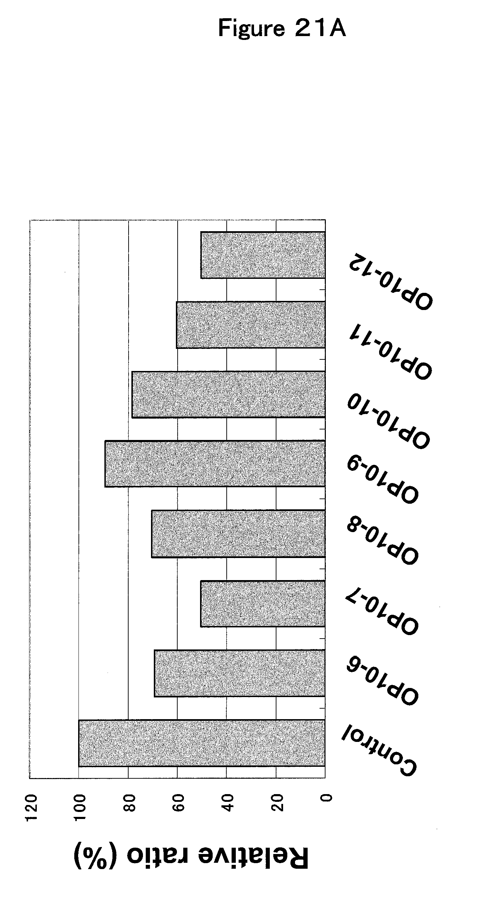

OP10-7, OP10-11 and OP10-12 caused about 40% to 50% inhibition of the A549 cells' growth ability in soft agar medium (Figure 21A).

### f) Human pancreatic cancer-derived PT45 cells

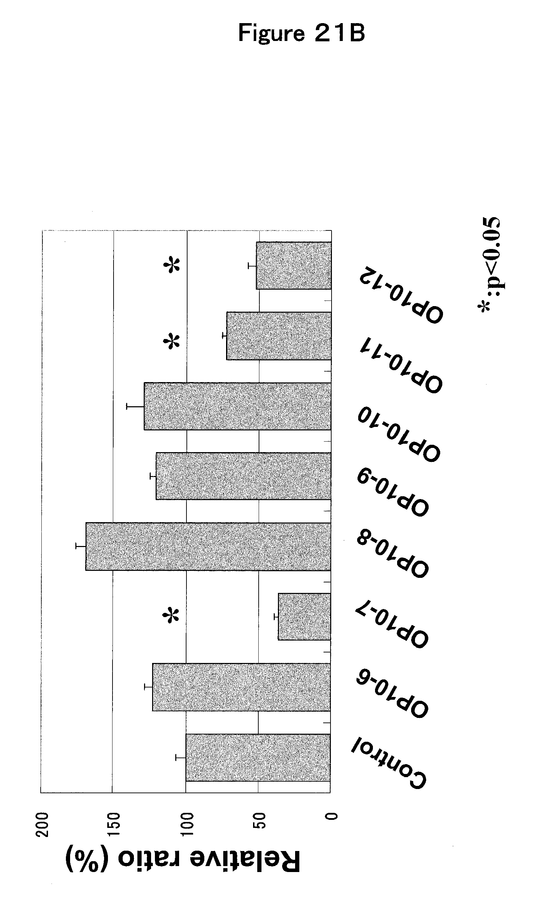

OP10-7, OP10-11 and OP10-12 caused about 30% to 60% inhibition of the PT45 cells' growth ability in soft agar medium (Figure 21B).

### (4) Study of GEF-1/C constituent oligopeptides for their in vivo anti-tumor growth effect

Next, the GEF-1/C constituent oligopeptides were studied for their *in vivo* anti-tumor growth effect.

Each GEF-1/C constituent oligopeptide (25 nmol) and a peptide transporter Wr-T (2 nmol) were mixed and then diluted to 0.1 ml with PBS, to which each cell suspension prepared at 5.0 × 10⁶ cells/ml Opti-MEM I medium was then added in a volume of 0.1 ml and mixed. This cell mixture was incubated in a CO₂ incubator at 37°C for 2 hours and then inoculated subcutaneously into nude mice. The long and short axes of tumor were measured at intervals of a week to calculate the tumor volume (= long axis × (short axis)² × π/6). After the mice were finally euthanized with carbon dioxide gas, tumor in each mouse was photographed. Subsequently, the tumor was excised and measured for its wet weight.

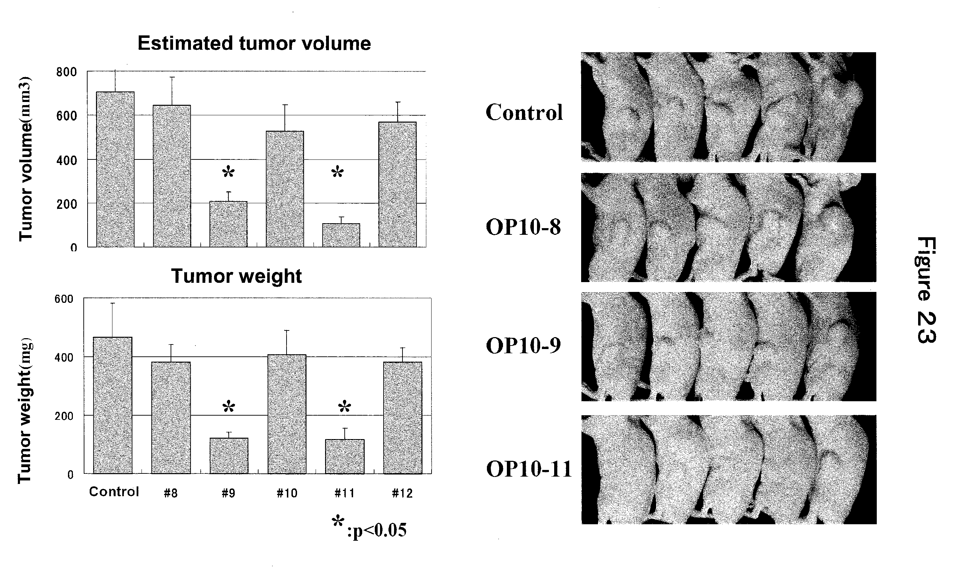

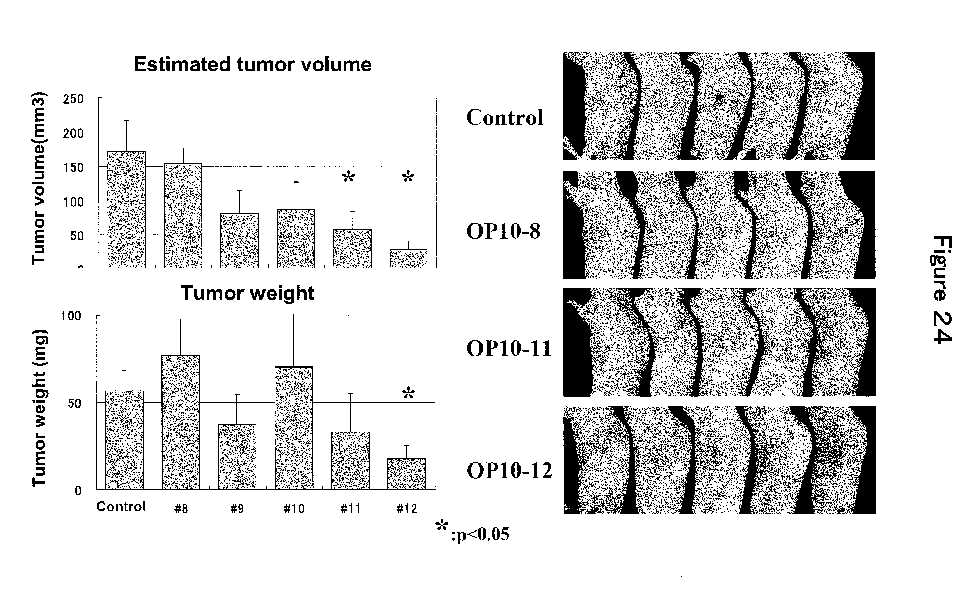

The results obtained are shown below for the respective tumor cells. Since OP10-11 showed the highest inhibitory effects on tube formation and growth ability in soft agar medium in the above *in vitro* tests, OP10-11 was also used in the *in vivo* test. It should be noted that #8 to #12 in Figures 23 and 24 represent OP10-8 to OP10-12, respectively.

### a) Mouse melanoma B16 cells

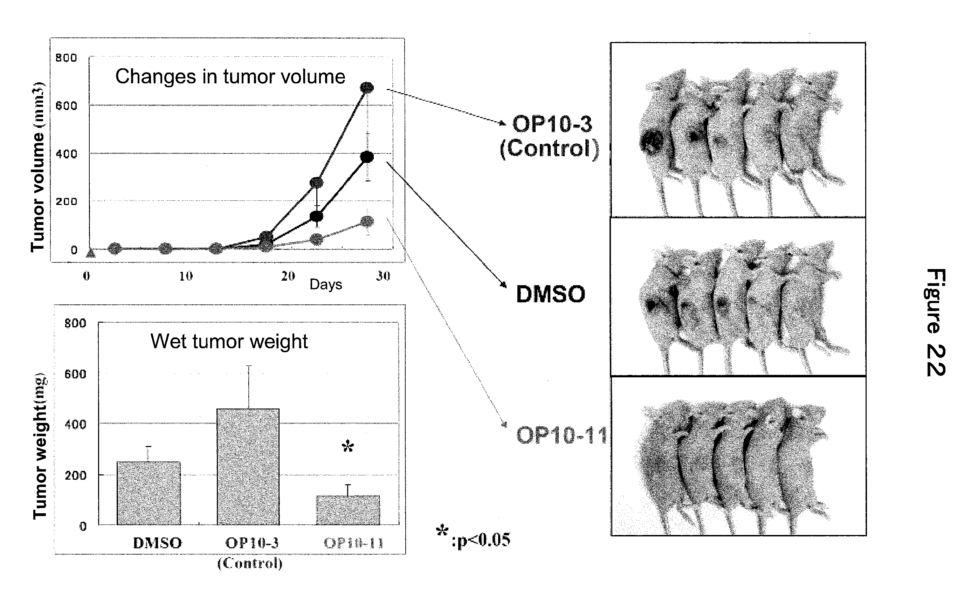

OP10-11 caused 50% or more inhibition of B16 cell tumor growth (Figure 22).

### b) Human colorectal cancer-derived COLO205 cells

OP10-9 and OP10-11 caused about 70% or more inhibition of COLO205 cell tumor growth (Figure 23).

### c) Human lung cancer-derived A549 cells

OP10-9, Op10-11 and OP10-12 inhibited A549 cell tumor growth by about 50% to 80% in tumor volume and about 35% to 70% in tumor weight (Figure 24).

Moreover, the r9-OP10-11 oligopeptide was also studied for its *in vivo* anti-tumor growth effect.

1.0 × 10⁶ COLO205 cells were inoculated subcutaneously into nude mice to prepare tumor of COLO205 cells. At 10 days after inoculation, a solution of r9-OP10-11 (35 nmol)/PBS was administered in a volume of 0.1 ml around the tumor whose diameter reached 5 to 6 mm (about 100 mm³). At 7, 14 and 21 days after this administration, the same administration was repeated. The long and short axes of tumor were measured to calculate the tumor volume (= long axis × (short axis)² × π/6). After the mice were finally euthanized with carbon dioxide gas, tumor in each mouse was photographed. Subsequently, the tumor was excised and measured for its wet weight.

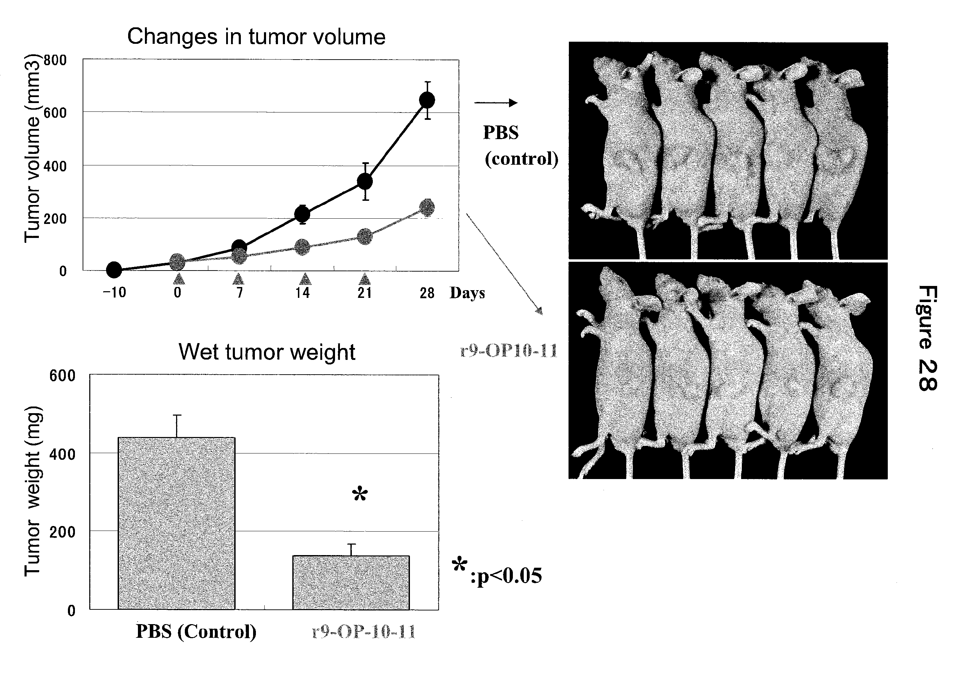

As a result, r9-OP10-11 inhibited the growth of human colorectal cancer-derived COLO205 cell tumor already formed under the skin of nude mice by about 65% in tumor volume and about 60% in tumor weight (Figure 28).

This result indicated that the r9-OP10-11 oligopeptide showed an *in vivo* anti-tumor growth effect.

In view of the foregoing, the siRNAs and GEF-1/C constituent oligopeptides of the present invention were found to have the ability to inhibit both *in vitro* and *in vivo* tumor growth through inhibition of GEF-1 expression by the siRNAs and through inhibition of GEF-1 functions by the GEF-1/C constituent oligopeptides.

### INDUSTRIAL APPLICABILITY

The present invention is used as an anti-tumor agent having an anti-angiogenic effect and/or an anti-tumor growth effect.

### Sequence Listing Free Text

SEQ ID NOs: 13 to 43: synthetic nucleotides
SEQ ID NOs: 44 to 67: synthetic peptides

## Claims

1. A tumor growth inhibitor, which contains a polypeptide of galactosylceramide expression factor-1 excluding the Q region, wherein the polypeptide comprises at least the C region or a part thereof.

2. A tumor growth inhibitor, which comprises a polypeptide shown in (a) or (b) below:
(a) a polypeptide which consists of the amino acid sequence shown in SEQ ID NO: 8,10 or 12; or
(b) a polypeptide which consists of an amino acid sequence with deletion, substitution or addition of one or several amino acids in the amino acid sequence shown in SEQ ID NO: 8, 10 or 12 and which has an anti-tumor growth effect.

3. A tumor growth inhibitor, which comprises a polypeptide shown in (a) or (b) below:
(a) a polypeptide which comprises at least 10 consecutive amino acid residues in the amino acid sequence shown in SEQ ID NO: 8, 10 or 12; or
(b) a polypeptide which consists of an amino acid sequence with deletion, substitution or addition of one or several amino acids in an amino acid sequence comprising at least 10 consecutive amino acid residues in the amino acid sequence shown in SEQ ID NO: 8, 10 or 12 and which has an anti-tumor growth effect.

4. The tumor growth inhibitor according to claim 3, wherein the polypeptide shown in (a) comprises an amino acid sequence shown in any of SEQ ID NOs: 44 to 65.

5. A tumor growth inhibitor, which contains a polynucleotide encoding a polypeptide of galactosylceramide expression factor-1 excluding the Q region, wherein the polypeptide comprises at least the C region or a part thereof.

6. A tumor growth inhibitor, which comprises a polynucleotide encoding a polypeptide shown in (a) or (b) below:
(a) a polypeptide which consists of the amino acid sequence shown in SEQ ID NO: 8,10 or 12; or
(b) a polypeptide which consists of an amino acid sequence with deletion, substitution or addition of one or several amino acids in the amino acid sequence shown in SEQ ID NO: 8, 10 or 12 and which has an anti-tumor growth effect.

7. A tumor growth inhibitor, which comprises a polynucleotide encoding a polypeptide shown in (a) or (b) below:
(a) a polypeptide which comprises at least 10 consecutive amino acid residues in the amino acid sequence shown in SEQ ID NO: 8, 10 or 12; or
(b) a polypeptide which consists of an amino acid sequence with deletion, substitution or addition of one or several amino acids in an amino acid sequence comprising at least 10 consecutive amino acid residues in the amino acid sequence shown in SEQ ID NO: 8, 10 or 12 and which has an anti-tumor growth effect.

8. A tumor growth inhibitor, which comprises a polynucleotide shown in (a) or (b) below:
(a) a polynucleotide which consists of the nucleotide sequence shown in SEQ ID NO: 7, 9 or 11; or
(b) a polynucleotide which is hybridizable under stringent conditions with a polynucleotide consisting of a nucleotide sequence complementary to the nucleotide sequence shown in SEQ ID NO: 7, 9 or 11 and which encodes a polypeptide having an anti-tumor growth effect.

9. A tumor growth inhibitor, which contains an expression inhibitor of galactosylceramide expression factor-1.

10. The tumor growth inhibitor according to claim 9, wherein the expression inhibitor of galactosylceramide expression factor-1 is siRNA or shRNA against a polynucleotide encoding galactosylceramide expression factor-1.

11. The tumor growth inhibitor according to claim 10, wherein the polynucleotide encoding galactosylceramide expression factor-1 comprises a nucleotide sequence encoding a polypeptide shown in (a) or (b) below:
(a) a polypeptide which consists of the amino acid sequence shown in SEQ ID NO: 2, 4 or 6; or
(b) a polypeptide which consists of an amino acid sequence with deletion, substitution or addition of one or several amino acids in the amino acid sequence shown in SEQ ID NO: 2, 4 or 6 and which has a tumor growth effect.

12. The tumor growth inhibitor according to claim 10, wherein the polynucleotide encoding galactosylceramide expression factor-1 comprises a polynucleotide shown in (a) or (b) below:
(a) a polynucleotide which consists of the nucleotide sequence shown in SEQ ID NO: 1,3 or 5; or
(b) a polynucleotide which is hybridizable under stringent conditions with a polynucleotide consisting of a nucleotide sequence complementary to the nucleotide sequence shown in SEQ ID NO: 1, 3 or 5 and which encodes a polypeptide having a tumor growth effect.

13. An angiogenesis inhibitor, which contains a polypeptide of galactosylceramide expression factor-1 excluding the Q region, wherein the polypeptide comprises at least the C region or a part thereof.

14. An angiogenesis inhibitor, which comprises a polypeptide shown in (a) or (b) below:
(a) a polypeptide which consists of the amino acid sequence shown in SEQ ID NO: 8,10 or 12; or
(b) a polypeptide which consists of an amino acid sequence with deletion, substitution or addition of one or several amino acids in the amino acid sequence shown in SEQ ID NO: 8, 10 or 12 and which has an anti-angiogenic effect.

15. An angiogenesis inhibitor, which comprises a polypeptide shown in (a) or (b) below:
(a) a polypeptide which comprises at least 10 consecutive amino acid residues in the amino acid sequence shown in SEQ ID NO: 8, 10 or 12; or
(b) a polypeptide which consists of an amino acid sequence with deletion, substitution or addition of one or several amino acids in an amino acid sequence comprising at least 10 consecutive amino acid residues in the amino acid sequence shown in SEQ ID NO: 8, 10 or 12 and which has an anti-angiogenic effect.

16. The angiogenesis inhibitor according to claim 15, wherein the polypeptide shown in (a) comprises an amino acid sequence shown in any of SEQ ID NOs: 44 to 65.

17. An angiogenesis inhibitor, which contains a polynucleotide encoding a polypeptide of galactosylceramide expression factor-1 excluding the Q region, wherein the polypeptide comprises at least the C region or a part thereof.

18. An angiogenesis inhibitor, which comprises a polynucleotide encoding a polypeptide shown in (a) or (b) below:
(a) a polypeptide which consists of the amino acid sequence shown in SEQ ID NO: 8, 10 or 12; or
(b) a polypeptide which consists of an amino acid sequence with deletion, substitution or addition of one or several amino acids in the amino acid sequence shown in SEQ ID NO: 8, 10 or 12 and which has an anti-angiogenic effect.

19. An angiogenesis inhibitor, which comprises a polynucleotide encoding a polypeptide shown in (a) or (b) below:
(a) a polypeptide which comprises at least 10 consecutive amino acid residues in the amino acid sequence shown in SEQ ID NO: 8, 10 or 12; or
(b) a polypeptide which consists of an amino acid sequence with deletion, substitution or addition of one or several amino acids in an amino acid sequence comprising at least 10 consecutive amino acid residues in the amino acid sequence shown in SEQ ID NO: 8, 10 or 12 and which has an anti-angiogenic effect.

20. An angiogenesis inhibitor, which comprises a polynucleotide shown in (a) or (b) below:
(a) a polynucleotide which consists of the nucleotide sequence shown in SEQ ID NO: 7, 9 or 11; or
(b) a polynucleotide which is hybridizable under stringent conditions with a polynucleotide consisting of a nucleotide sequence complementary to the nucleotide sequence shown in SEQ ID NO: 7, 9 or 11 and which encodes a polypeptide having an anti-angiogenic effect.

21. An angiogenesis inhibitor, which contains an expression inhibitor of galactosylceramide expression factor-1.

22. The angiogenesis inhibitor according to claim 21, wherein the expression inhibitor of galactosylceramide expression factor-1 is siRNA or shRNA against a polynucleotide encoding galactosylceramide expression factor-1.

23. The angiogenesis inhibitor according to claim 22, wherein the polynucleotide encoding galactosylceramide expression factor-1 comprises a nucleotide sequence encoding a polypeptide shown in (a) or (b) below:
(a) a polypeptide which consists of the amino acid sequence shown in SEQ ID NO: 2, 4 or 6; or
(b) a polypeptide which consists of an amino acid sequence with deletion, substitution or addition of one or several amino acids in the amino acid sequence shown in SEQ ID NO: 2, 4 or 6 and which has an angiogenic effect.

24. The angiogenesis inhibitor according to claim 22, wherein the polynucleotide encoding galactosylceramide expression factor-1 comprises a polynucleotide shown in (a) or (b) below:
(a) a polynucleotide which consists of the nucleotide sequence shown in SEQ ID NO: 1, 3 or 5; or
(b) a polynucleotide which is hybridizable under stringent conditions with a polynucleotide consisting of a nucleotide sequence complementary to the nucleotide sequence shown in SEQ ID NO: 1, 3 or 5 and which encodes a polypeptide having an angiogenic effect.

25. An eliminator of cancer cell characteristics, which comprises a polypeptide shown in (a) or (b) below:
(a) a polypeptide which consists of the amino acid sequence shown in SEQ ID NO: 8, 10 or 12; or
(b) a polypeptide which consists of an amino acid sequence with deletion, substitution or addition of one or several amino acids in the amino acid sequence shown in SEQ ID NO: 8, 10 or 12 and which has an elimination effect on cancer cell characteristics.

26. The eliminator of cancer cell characteristics according to claim 25, which allows cancer cells to acquire the ability of contact inhibition.

27. A method for tumor growth inhibition, which comprises inhibiting the expression of galactosylceramide expression factor-1.

28. A method for angiogenesis inhibition, which comprises inhibiting the expression ofgalactosylceramide expression factor-1.


SEQUENCE LISTING

<110>  Tokyo Metropolitan Institute of Medical Science

<120>  A method for regulating tumor growth by targeting
       galactosylceramide expression factor-1

<130>  PCT11-0037

<150>  JP2010-144763
<151>  2010-06-25

<160>  67

<170>  PatentIn version 3.5

<210>  1
<211>  2827
<212>  DNA
<213>  Rattus norvegicus

<220>
<221>  CDS
<222>  (21)..(2336)

<400>  1
cggtccggag tggggtcgcc atg ggg cga ggc agc ggc acc ttc gag cgt ctc      53
                      Met Gly Arg Gly Ser Gly Thr Phe Glu Arg Leu
                      1               5                   10

cta gac aaa gcc acc agc cag ctt cta ttg gag aca gac tgg gag tcc      101
Leu Asp Lys Ala Thr Ser Gln Leu Leu Leu Glu Thr Asp Trp Glu Ser
            15                  20                  25

att cta cag atc tgc gac ctg atc cgt cag ggg gac aca caa gca aaa      149
Ile Leu Gln Ile Cys Asp Leu Ile Arg Gln Gly Asp Thr Gln Ala Lys
        30                  35                  40

tat gct gta aac tcc atc aag aag aag gtc aat gat aag aac cca cat      197
Tyr Ala Val Asn Ser Ile Lys Lys Lys Val Asn Asp Lys Asn Pro His
    45                  50                  55

gtg gct ttg tat gct ctg gag gtg atg gag tct gtg gta aag aac tgt      245
Val Ala Leu Tyr Ala Leu Glu Val Met Glu Ser Val Val Lys Asn Cys
60                  65                  70                  75

ggc cag aca gtc cat gat gaa gtg gcc aac aaa cag acc atg gaa gaa      293
Gly Gln Thr Val His Asp Glu Val Ala Asn Lys Gln Thr Met Glu Glu

                80                  85                  90

ctg aag gag ctg ctg aag agg caa gtg gaa gtt aat gtt cgg aac aag          341
Leu Lys Glu Leu Leu Lys Arg Gln Val Glu Val Asn Val Arg Asn Lys
            95                  100                 105

atc ttg tac ctg atc cag gcc tgg gca cat gcg ttc cgg aat gaa ccc          389
Ile Leu Tyr Leu Ile Gln Ala Trp Ala His Ala Phe Arg Asn Glu Pro
        110                 115                 120

aag tac aag gtg gtc cag gac aca tac cag atc atg aag gta gaa gga          437
Lys Tyr Lys Val Val Gln Asp Thr Tyr Gln Ile Met Lys Val Glu Gly
    125                 130                 135

cat gtc ttc cct gag ttt aag gag agt gac gcc atg ttt gct gct gaa          485
His Val Phe Pro Glu Phe Lys Glu Ser Asp Ala Met Phe Ala Ala Glu
140                 145                 150                 155

aga gcc cct gac tgg gtg gat gct gag gag tgc cat cgg tgc aga gta          533
Arg Ala Pro Asp Trp Val Asp Ala Glu Glu Cys His Arg Cys Arg Val
                160                 165                 170

cag ttt gga gtg gtg acc cgc aag cat cac tgc cga gcg tgt ggg cag          581
Gln Phe Gly Val Val Thr Arg Lys His His Cys Arg Ala Cys Gly Gln
            175                 180                 185

atc ttt tgt ggc aag tgt tcc tcc aag tac tcc acc atc ccc aag ttc          629
Ile Phe Cys Gly Lys Cys Ser Ser Lys Tyr Ser Thr Ile Pro Lys Phe
        190                 195                 200

ggc att gag aag gaa gtg cgc gtg tgt gag ccc tgc tat gag cag ctg          677
Gly Ile Glu Lys Glu Val Arg Val Cys Glu Pro Cys Tyr Glu Gln Leu
    205                 210                 215

aac aag aag gca gaa ggg aaa gct gcc tct acc act gag ctg ccc cca          725
Asn Lys Lys Ala Glu Gly Lys Ala Ala Ser Thr Thr Glu Leu Pro Pro
220                 225                 230                 235

gag tac ctg acc agc ccc ctg tca cag cag tct cag ctg ccc cca aag          773
Glu Tyr Leu Thr Ser Pro Leu Ser Gln Gln Ser Gln Leu Pro Pro Lys
                240                 245                 250

cgg gat gag aca gca ctg caa gaa gag gag gag cta cag ctg gcg ctg          821
Arg Asp Glu Thr Ala Leu Gln Glu Glu Glu Glu Leu Gln Leu Ala Leu
            255                 260                 265

gcc cta tca cag tca gag gct gag gag aag gaa agg atg aga cag aag          869
Ala Leu Ser Gln Ser Glu Ala Glu Glu Lys Glu Arg Met Arg Gln Lys
        270                 275                 280

tca aca tac aca gcg cat cca aag tca gag cct gcg ccc ttg gct tcc 917
Ser Thr Tyr Thr Ala His Pro Lys Ser Glu Pro Ala Pro Leu Ala Ser
    285                 290                 295

tct gca ccc cca gct ggt agc ctg tac tcc tcg cct gtg aac tca tca 965
Ser Ala Pro Pro Ala Gly Ser Leu Tyr Ser Ser Pro Val Asn Ser Ser
300                 305                 310                 315

gca cct ctg gct gag gac atc gac cct gag ctt gca aga tac ctc aac 1013
Ala Pro Leu Ala Glu Asp Ile Asp Pro Glu Leu Ala Arg Tyr Leu Asn
                320                 325                 330

cgg aac tac tgg gag aag aaa cag gaa gaa gct cgg aag agc ccc aca 1061
Arg Asn Tyr Trp Glu Lys Lys Gln Glu Glu Ala Arg Lys Ser Pro Thr
            335                 340                 345

cca tct gca cct gtg ccc ctg acc gag cca gct gcc cag ccc gga gaa 1109
Pro Ser Ala Pro Val Pro Leu Thr Glu Pro Ala Ala Gln Pro Gly Glu
        350                 355                 360

gga cat aca gcc ccc aac agc atg gta gag gcc cct ctt cca gag aca 1157
Gly His Thr Ala Pro Asn Ser Met Val Glu Ala Pro Leu Pro Glu Thr
    365                 370                 375

gac tct cag ccc ata act tcc tgc agt ggc ccc ttt agt gag cag tac 1205
Asp Ser Gln Pro Ile Thr Ser Cys Ser Gly Pro Phe Ser Glu Gln Tyr
380                 385                 390                 395

cag aac gcg gag tcg gag gag agc cac gag cag ttc ctc aag gcc ctg 1253
Gln Asn Ala Glu Ser Glu Glu Ser His Glu Gln Phe Leu Lys Ala Leu
                400                 405                 410

cag aat gca gtc agc act ttt gtc aac cgc atg aag agc aac cac atg 1301
Gln Asn Ala Val Ser Thr Phe Val Asn Arg Met Lys Ser Asn His Met
            415                 420                 425

agg ggc cgc agt atc acc aat gac tcg gct gtg ctg tcc ctc ttc cag 1349
Arg Gly Arg Ser Ile Thr Asn Asp Ser Ala Val Leu Ser Leu Phe Gln
        430                 435                 440

tcc atc aat agc aca cac cca cag ctg ctc gag ctg ctc aac cgg ctg 1397
Ser Ile Asn Ser Thr His Pro Gln Leu Leu Glu Leu Leu Asn Arg Leu
    445                 450                 455

gat gag cgc agg ctg tac tac gag ggg ctt cag gac aag ctg gca cag 1445
Asp Glu Arg Arg Leu Tyr Tyr Glu Gly Leu Gln Asp Lys Leu Ala Gln
460                 465                 470                 475

ata cgt gat gcc cga ggg gcc ctg agt gcc ctg cgt gaa gag cac agg 1493
Ile Arg Asp Ala Arg Gly Ala Leu Ser Ala Leu Arg Glu Glu His Arg
                480                 485                 490

gag aag ctg cgc cgg gca gct gag gag gcg gag cgt caa cgt cag atc 1541
Glu Lys Leu Arg Arg Ala Ala Glu Glu Ala Glu Arg Gln Arg Gln Ile
            495                 500                 505

cag ctg gca cag aag ctg gag atc atg aga caa aag aag cag gag tat 1589
Gln Leu Ala Gln Lys Leu Glu Ile Met Arg Gln Lys Lys Gln Glu Tyr
        510                 515                 520

ctg gag gtg cag aga cag cta gct atc cag cgt ctg cag gaa cag gag 1637
Leu Glu Val Gln Arg Gln Leu Ala Ile Gln Arg Leu Gln Glu Gln Glu
    525                 530                 535

aag gaa cgg cag atg cgc ctg gag caa cag aag cag act gtc cag atg 1685
Lys Glu Arg Gln Met Arg Leu Glu Gln Gln Lys Gln Thr Val Gln Met
540                 545                 550                 555

cgt gcc cag atg cct gtc ttc ccc ttg cct tat gcc cag ctc cag gct 1733
Arg Ala Gln Met Pro Val Phe Pro Leu Pro Tyr Ala Gln Leu Gln Ala
                560                 565                 570

atg ccc aca gct ggg ggt gta ctc tac cag ccc tca ggc cca acc agc 1781
Met Pro Thr Ala Gly Gly Val Leu Tyr Gln Pro Ser Gly Pro Thr Ser
            575                 580                 585

ttt cct ggc acc ttt agc cca gca ggc tca gta gag ggc tct ccg atg 1829
Phe Pro Gly Thr Phe Ser Pro Ala Gly Ser Val Glu Gly Ser Pro Met
        590                 595                 600

cat ggt gtg tat atg agc cag cca gcc cca gcc act ggc ccc tac ccc 1877
His Gly Val Tyr Met Ser Gln Pro Ala Pro Ala Thr Gly Pro Tyr Pro
    605                 610                 615

agc atg cct ggc acc aca gca gat ccc agc atg gtc agc gcc tac atg 1925
Ser Met Pro Gly Thr Thr Ala Asp Pro Ser Met Val Ser Ala Tyr Met
620                 625                 630                 635

tac cca gca ggt gcc cct ggg gca cag gca gcc cct cag gcc cag gcc 1973
Tyr Pro Ala Gly Ala Pro Gly Ala Gln Ala Ala Pro Gln Ala Gln Ala
                640                 645                 650

ggg ccc acc acc aac cct gcc tac tcc tcg tac cag cct act cca acc 2021
Gly Pro Thr Thr Asn Pro Ala Tyr Ser Ser Tyr Gln Pro Thr Pro Thr
            655                 660                 665

cca ggc tac cag gcc cca cag agc ctc cca gcc atc tcc cag cct cca 2069

Pro Gly Tyr Gln Ala Pro Gln Ser Leu Pro Ala Ile Ser Gln Pro Pro
        670                 675                 680

cag acc agc aac att ggc tac atg ggg agc cag cca atg tcc atg ggc 2117
Gln Thr Ser Asn Ile Gly Tyr Met Gly Ser Gln Pro Met Ser Met Gly
    685                 690                 695

tac cag cca tac aac atg cag aat ctc atg acc acc ctt cca ggc cag 2165
Tyr Gln Pro Tyr Asn Met Gln Asn Leu Met Thr Thr Leu Pro Gly Gln
700                 705                 710                 715

gat gcg tct ctg cca gcc cag cag ccc tac atc aca ggg cag cag ccc 2213
Asp Ala Ser Leu Pro Ala Gln Gln Pro Tyr Ile Thr Gly Gln Gln Pro
                720                 725                 730

atg tac cag cag atg gca ccc agc act ggc cct ccc cag cag cag ccc 2261
Met Tyr Gln Gln Met Ala Pro Ser Thr Gly Pro Pro Gln Gln Gln Pro
            735                 740                 745

cct gtg gcc caa ccg cca cct aca cag gga ccg cca gca cag ggc aat 2309
Pro Val Ala Gln Pro Pro Pro Thr Gln Gly Pro Pro Ala Gln Gly Asn
        750                 755                 760

gag acc cag ctc atc tcc ttc gac tga ccttgagtct ggcgctcacc 2356
Glu Thr Gln Leu Ile Ser Phe Asp
    765                 770

atccagagta acactacagt tctccagaaa ccacttatat gtctaactag ccatttcctc 2416

ccattactgc cctgtagtgt cccttctgtg agcaaggggt gggccttcac ccttggccct 2476

cctccctgtc ctcagtggtc tggctcctgt cactggttcc ctgctttggt cctgatgcag 2536

tccaaccttc ccgggactgg gactctgcag gacaagtagg accttttctg gagaatgccc 2596

agctgtgtcg gggcctgcca gaggtgactg ctgtggggat ggttagcctg gcccagagtc 2656

cgatgtgaac tgtgtggtgt ccaggcatgg ccctggtacc cagaagaacg atgtgaccca 2716

tgcacagcaa ggtagaagtg ttcaggcatc tctgtctccc ctactccttg gatgtcatct 2776

ctccagtgca gaacagtgaa aataaaatgt attcaaaaaa aaaaaaaaaa a 2827

<210> 2
<211> 771
<212> PRT
<213> Rattus norvegicus

<400> 2

Met Gly Arg Gly Ser Gly Thr Phe Glu Arg Leu Leu Asp Lys Ala Thr
1               5                   10                  15

Ser Gln Leu Leu Leu Glu Thr Asp Trp Glu Ser Ile Leu Gln Ile Cys
            20                  25                  30

Asp Leu Ile Arg Gln Gly Asp Thr Gln Ala Lys Tyr Ala Val Asn Ser
        35                  40                  45

Ile Lys Lys Lys Val Asn Asp Lys Asn Pro His Val Ala Leu Tyr Ala
    50                  55                  60

Leu Glu Val Met Glu Ser Val Val Lys Asn Cys Gly Gln Thr Val His
65                  70                  75                  80

Asp Glu Val Ala Asn Lys Gln Thr Met Glu Glu Leu Lys Glu Leu Leu
                85                  90                  95

Lys Arg Gln Val Glu Val Asn Val Arg Asn Lys Ile Leu Tyr Leu Ile
            100                 105                 110

Gln Ala Trp Ala His Ala Phe Arg Asn Glu Pro Lys Tyr Lys Val Val
        115                 120                 125

Gln Asp Thr Tyr Gln Ile Met Lys Val Glu Gly His Val Phe Pro Glu
    130                 135                 140

Phe Lys Glu Ser Asp Ala Met Phe Ala Ala Glu Arg Ala Pro Asp Trp
145                 150                 155                 160

Val Asp Ala Glu Glu Cys His Arg Cys Arg Val Gln Phe Gly Val Val
                165                 170                 175

Thr Arg Lys His His Cys Arg Ala Cys Gly Gln Ile Phe Cys Gly Lys
            180                 185                 190

Cys Ser Ser Lys Tyr Ser Thr Ile Pro Lys Phe Gly Ile Glu Lys Glu
        195                 200                 205

Val Arg Val Cys Glu Pro Cys Tyr Glu Gln Leu Asn Lys Lys Ala Glu
    210                 215                 220

Gly Lys Ala Ala Ser Thr Thr Glu Leu Pro Pro Glu Tyr Leu Thr Ser
225                 230                 235                 240

Pro Leu Ser Gln Gln Ser Gln Leu Pro Pro Lys Arg Asp Glu Thr Ala
                245                 250                 255

Leu Gln Glu Glu Glu Glu Leu Gln Leu Ala Leu Ala Leu Ser Gln Ser
            260                 265                 270

Glu Ala Glu Glu Lys Glu Arg Met Arg Gln Lys Ser Thr Tyr Thr Ala
        275                 280                 285

His Pro Lys Ser Glu Pro Ala Pro Leu Ala Ser Ser Ala Pro Pro Ala
    290                 295                 300

Gly Ser Leu Tyr Ser Ser Pro Val Asn Ser Ser Ala Pro Leu Ala Glu
305                 310                 315                 320

Asp Ile Asp Pro Glu Leu Ala Arg Tyr Leu Asn Arg Asn Tyr Trp Glu
                325                 330                 335

Lys Lys Gln Glu Glu Ala Arg Lys Ser Pro Thr Pro Ser Ala Pro Val
            340                 345                 350

Pro Leu Thr Glu Pro Ala Ala Gln Pro Gly Glu Gly His Thr Ala Pro
        355                 360                 365

Asn Ser Met Val Glu Ala Pro Leu Pro Glu Thr Asp Ser Gln Pro Ile
    370                 375                 380

Thr Ser Cys Ser Gly Pro Phe Ser Glu Gln Tyr Gln Asn Ala Glu Ser
385                 390                 395                 400

Glu Glu Ser His Glu Gln Phe Leu Lys Ala Leu Gln Asn Ala Val Ser
                405                 410                 415

Thr Phe Val Asn Arg Met Lys Ser Asn His Met Arg Gly Arg Ser Ile
            420                 425                 430

Thr Asn Asp Ser Ala Val Leu Ser Leu Phe Gln Ser Ile Asn Ser Thr
        435                 440                 445

His Pro Gln Leu Leu Glu Leu Leu Asn Arg Leu Asp Glu Arg Arg Leu
    450                 455                 460

Tyr Tyr Glu Gly Leu Gln Asp Lys Leu Ala Gln Ile Arg Asp Ala Arg
465                 470                 475                 480

Gly Ala Leu Ser Ala Leu Arg Glu Glu His Arg Glu Lys Leu Arg Arg
                485                 490                 495

Ala Ala Glu Glu Ala Glu Arg Gln Arg Gln Ile Gln Leu Ala Gln Lys
            500                 505                 510

Leu Glu Ile Met Arg Gln Lys Lys Gln Glu Tyr Leu Glu Val Gln Arg
        515                 520                 525

Gln Leu Ala Ile Gln Arg Leu Gln Glu Gln Glu Lys Glu Arg Gln Met
    530                 535                 540

Arg Leu Glu Gln Gln Lys Gln Thr Val Gln Met Arg Ala Gln Met Pro
545                 550                 555                 560

Val Phe Pro Leu Pro Tyr Ala Gln Leu Gln Ala Met Pro Thr Ala Gly
                565                 570                 575

Gly Val Leu Tyr Gln Pro Ser Gly Pro Thr Ser Phe Pro Gly Thr Phe

            580                 585                 590

Ser Pro Ala Gly Ser Val Glu Gly Ser Pro Met His Gly Val Tyr Met
        595                 600                 605

Ser Gln Pro Ala Pro Ala Thr Gly Pro Tyr Pro Ser Met Pro Gly Thr
    610                 615                 620

Thr Ala Asp Pro Ser Met Val Ser Ala Tyr Met Tyr Pro Ala Gly Ala
625                 630                 635                 640

Pro Gly Ala Gln Ala Ala Pro Gln Ala Gln Ala Gly Pro Thr Thr Asn
                645                 650                 655

Pro Ala Tyr Ser Ser Tyr Gln Pro Thr Pro Thr Pro Gly Tyr Gln Ala
            660                 665                 670

Pro Gln Ser Leu Pro Ala Ile Ser Gln Pro Pro Gln Thr Ser Asn Ile
        675                 680                 685

Gly Tyr Met Gly Ser Gln Pro Met Ser Met Gly Tyr Gln Pro Tyr Asn
    690                 695                 700

Met Gln Asn Leu Met Thr Thr Leu Pro Gly Gln Asp Ala Ser Leu Pro
705                 710                 715                 720

Ala Gln Gln Pro Tyr Ile Thr Gly Gln Gln Pro Met Tyr Gln Gln Met
                725                 730                 735

Ala Pro Ser Thr Gly Pro Pro Gln Gln Gln Pro Pro Val Ala Gln Pro
            740                 745                 750

Pro Pro Thr Gln Gly Pro Pro Ala Gln Gly Asn Glu Thr Gln Leu Ile
        755                 760                 765

Ser Phe Asp
    770

<210> 3
<211> 2906
<212> DNA
<213> Homo sapiens

<220>
<221> CDS
<222> (76)..(2409)

<400> 3
gaagcggaag tcgggggccg caccagctcg tagcagggaa gcgcccgcgg cgtcgggttt 60

gggctggagg tcgcc atg ggg cga ggc agc ggc acc ttc gag cgt ctc cta 111
Met Gly Arg Gly Ser Gly Thr Phe Glu Arg Leu Leu
1               5                   10

gac aag gcg acc agc cag ctc ctg ttg gag aca gat tgg gag tcc att 159
Asp Lys Ala Thr Ser Gln Leu Leu Leu Glu Thr Asp Trp Glu Ser Ile
        15                  20                  25

ttg cag atc tgc gac ctg atc cgc caa ggg gac aca caa gca aaa tat 207
Leu Gln Ile Cys Asp Leu Ile Arg Gln Gly Asp Thr Gln Ala Lys Tyr
    30                  35                  40

gct gtg aat tcc atc aag aag aaa gtc aac gac aag aac cca cac gtc 255
Ala Val Asn Ser Ile Lys Lys Lys Val Asn Asp Lys Asn Pro His Val
45                  50                  55                  60

gcc ttg tat gcc ctg gag gtc atg gaa tct gtg gta aag aac tgt ggc 303
Ala Leu Tyr Ala Leu Glu Val Met Glu Ser Val Val Lys Asn Cys Gly
                65                  70                  75

cag aca gtt cat gat gag gtg gcc aac aag cag acc atg gag gag ctg 351
Gln Thr Val His Asp Glu Val Ala Asn Lys Gln Thr Met Glu Glu Leu
            80                  85                  90

aag gac ctg ctg aag aga caa gtg gag gta aac gtc cgt aac aag atc 399
Lys Asp Leu Leu Lys Arg Gln Val Glu Val Asn Val Arg Asn Lys Ile
        95                  100                 105

ctg tac ctg atc cag gcc tgg gcg cat gcc ttc cgg aac gag ccc aag 447
Leu Tyr Leu Ile Gln Ala Trp Ala His Ala Phe Arg Asn Glu Pro Lys
    110                 115                 120

tac aag gtg gtc cag gac acc tac cag atc atg aag gtg gag ggg cac 495
Tyr Lys Val Val Gln Asp Thr Tyr Gln Ile Met Lys Val Glu Gly His

125                 130                 135                 140

gtc ttt cca gaa ttc aaa gag agc gat gcc atg ttt gct gcc gag aga          543
Val Phe Pro Glu Phe Lys Glu Ser Asp Ala Met Phe Ala Ala Glu Arg
                145                 150                 155

gcc cca gac tgg gtg gac gct gag gaa tgc cac cgc tgc agg gtg cag          591
Ala Pro Asp Trp Val Asp Ala Glu Glu Cys His Arg Cys Arg Val Gln
            160                 165                 170

ttc ggg gtg atg acc cgt aag cac cac tgc cgg gcg tgt ggg cag ata          639
Phe Gly Val Met Thr Arg Lys His His Cys Arg Ala Cys Gly Gln Ile
        175                 180                 185

ttc tgt gga aag tgt tct tcc aag tac tcc acc atc ccc aag ttt ggc          687
Phe Cys Gly Lys Cys Ser Ser Lys Tyr Ser Thr Ile Pro Lys Phe Gly
    190                 195                 200

atc gag aag gag gtg cgc gtg tgt gag ccc tgc tac gag cag ctg aac          735
Ile Glu Lys Glu Val Arg Val Cys Glu Pro Cys Tyr Glu Gln Leu Asn
205                 210                 215                 220

agg aaa gcg gag gga aag gcc act tcc acc act gag ctg ccc ccc gag          783
Arg Lys Ala Glu Gly Lys Ala Thr Ser Thr Thr Glu Leu Pro Pro Glu
                225                 230                 235

tac ctg acc agc ccc ctg tct cag cag tcc cag ctg ccc ccc aag agg          831
Tyr Leu Thr Ser Pro Leu Ser Gln Gln Ser Gln Leu Pro Pro Lys Arg
            240                 245                 250

gac gag acg gcc ctg cag gag gag gag gag ctg cag ctg gcc ctg gcg          879
Asp Glu Thr Ala Leu Gln Glu Glu Glu Glu Leu Gln Leu Ala Leu Ala
        255                 260                 265

ctg tca cag tca gag gcg gag gag aag gag agg ctg aga cag aag tcc          927
Leu Ser Gln Ser Glu Ala Glu Glu Lys Glu Arg Leu Arg Gln Lys Ser
    270                 275                 280

acg tac act tcg tac ccc aag gcg gag ccc atg ccc tcg gcc tcc tca          975
Thr Tyr Thr Ser Tyr Pro Lys Ala Glu Pro Met Pro Ser Ala Ser Ser
285                 290                 295                 300

gcg ccc ccc gcc agc agc ctg tac tct tca cct gtg aac tcg tcg gcg          1023
Ala Pro Pro Ala Ser Ser Leu Tyr Ser Ser Pro Val Asn Ser Ser Ala
                305                 310                 315

cct ctg gct gag gac atc gac cct gag ctc gca cgg tat ctc aac cgg          1071
Pro Leu Ala Glu Asp Ile Asp Pro Glu Leu Ala Arg Tyr Leu Asn Arg
            320                 325                 330

aac tac tgg gag aag aag cag gag gag gct cgc aag agc ccc acg cca 1119
Asn Tyr Trp Glu Lys Lys Gln Glu Glu Ala Arg Lys Ser Pro Thr Pro
        335                 340                 345

tct gcg ccc gtg ccc ctg acg gag ccg gct gca cag cct ggg gaa ggg 1167
Ser Ala Pro Val Pro Leu Thr Glu Pro Ala Ala Gln Pro Gly Glu Gly
    350                 355                 360

cac gca gcc ccc acc aac gtg gtg gag aac ccc ctc ccg gag aca gac 1215
His Ala Ala Pro Thr Asn Val Val Glu Asn Pro Leu Pro Glu Thr Asp
365                 370                 375                 380

tct cag ccc att cct ccc tct ggt ggc ccc ttt agt gag cca cag ttc 1263
Ser Gln Pro Ile Pro Pro Ser Gly Gly Pro Phe Ser Glu Pro Gln Phe
                385                 390                 395

cac aat ggc gag tct gag gag agc cac gag cag ttc ctg aag gcg ctg 1311
His Asn Gly Glu Ser Glu Glu Ser His Glu Gln Phe Leu Lys Ala Leu
            400                 405                 410

cag aac gcc gtc acc acc ttc gtg aac cgc atg aag agt aac cac atg 1359
Gln Asn Ala Val Thr Thr Phe Val Asn Arg Met Lys Ser Asn His Met
        415                 420                 425

cgg ggc cgc agc atc acc aat gac tcg gcc gtg ctc tca ctc ttc cag 1407
Arg Gly Arg Ser Ile Thr Asn Asp Ser Ala Val Leu Ser Leu Phe Gln
    430                 435                 440

tcc atc aac ggc atg cac ccg cag ctg ctg gag ctg ctc aac cag ctg 1455
Ser Ile Asn Gly Met His Pro Gln Leu Leu Glu Leu Leu Asn Gln Leu
445                 450                 455                 460

gac gag cgc agg ctg tac tat gag ggg ctg cag gac aag ctg gca cag 1503
Asp Glu Arg Arg Leu Tyr Tyr Glu Gly Leu Gln Asp Lys Leu Ala Gln
                465                 470                 475

atc cgc gat gcc cgg ggg gcg ctg agt gcc ctg cgc gaa gag cac cgg 1551
Ile Arg Asp Ala Arg Gly Ala Leu Ser Ala Leu Arg Glu Glu His Arg
            480                 485                 490

gag aag ctt cgc cgg gca gcc gag gag gca gag cgc cag cgc cag atc 1599
Glu Lys Leu Arg Arg Ala Ala Glu Glu Ala Glu Arg Gln Arg Gln Ile
        495                 500                 505

cag ctg gcc cag aag ctg gag ata atg cgg cag aag aag cag gag tac 1647
Gln Leu Ala Gln Lys Leu Glu Ile Met Arg Gln Lys Lys Gln Glu Tyr
    510                 515                 520

ctg gag gtg cag agg cag ctg gcc atc cag cgc ctg cag gag cag gag 1695
Leu Glu Val Gln Arg Gln Leu Ala Ile Gln Arg Leu Gln Glu Gln Glu
525                 530                 535                 540

aag gag cgg cag atg cgg ctg gag cag cag aag cag acg gtc cag atg 1743
Lys Glu Arg Gln Met Arg Leu Glu Gln Gln Lys Gln Thr Val Gln Met
                545                 550                 555

cgc gcg cag atg ccc gcc ttc ccc ctg ccc tac gcc cag ctc cag gcc 1791
Arg Ala Gln Met Pro Ala Phe Pro Leu Pro Tyr Ala Gln Leu Gln Ala
            560                 565                 570

atg ccc gca gcc gga ggt gtg ctc tac cag ccc tcg gga cca gcc agc 1839
Met Pro Ala Ala Gly Gly Val Leu Tyr Gln Pro Ser Gly Pro Ala Ser
        575                 580                 585

ttc ccc agc acc ttc agc cct gcc ggc tcg gtg gag ggc tcc cca atg 1887
Phe Pro Ser Thr Phe Ser Pro Ala Gly Ser Val Glu Gly Ser Pro Met
    590                 595                 600

cac ggc gtg tac atg agc cag ccg gcc cct gcc gct ggc ccc tac ccc 1935
His Gly Val Tyr Met Ser Gln Pro Ala Pro Ala Ala Gly Pro Tyr Pro
605                 610                 615                 620

agc atg ccc agc act gcg gct gat ccc agc atg gtg agt gcc tac atg 1983
Ser Met Pro Ser Thr Ala Ala Asp Pro Ser Met Val Ser Ala Tyr Met
                625                 630                 635

tac cca gca ggg gcc act ggg gcg cag gcg gcc ccc cag gcc cag gcc 2031
Tyr Pro Ala Gly Ala Thr Gly Ala Gln Ala Ala Pro Gln Ala Gln Ala
            640                 645                 650

gga ccc acc gcc agc ccc gct tac tca tcc tac cag cct act ccc aca 2079
Gly Pro Thr Ala Ser Pro Ala Tyr Ser Ser Tyr Gln Pro Thr Pro Thr
        655                 660                 665

gcg ggc tac cag aac gtg gcc tcc cag gcc cca cag agc ctc ccg gcc 2127
Ala Gly Tyr Gln Asn Val Ala Ser Gln Ala Pro Gln Ser Leu Pro Ala
    670                 675                 680

atc tct cag cct ccg cag tcc agc acc atg ggc tac atg ggg agc cag 2175
Ile Ser Gln Pro Pro Gln Ser Ser Thr Met Gly Tyr Met Gly Ser Gln
685                 690                 695                 700

tca gtc tcc atg ggc tac cag cct tac aac atg cag aat ctc atg acc 2223
Ser Val Ser Met Gly Tyr Gln Pro Tyr Asn Met Gln Asn Leu Met Thr
                705                 710                 715

acc ctc cca agc cag gat gcg tct ctg cca ccc cag cag ccc tac atc 2271

Thr Leu Pro Ser Gln Asp Ala Ser Leu Pro Pro Gln Gln Pro Tyr Ile
        720                 725                 730

gcg ggg cag cag ccc atg tac cag cag atg gca ccc tct ggc ggt ccc      2319
Ala Gly Gln Gln Pro Met Tyr Gln Gln Met Ala Pro Ser Gly Gly Pro
    735                 740                 745

ccc cag cag cag ccc ccc gtg gcc cag caa ccg cag gca cag ggg ccg      2367
Pro Gln Gln Gln Pro Pro Val Ala Gln Gln Pro Gln Ala Gln Gly Pro
750                 755                 760

ccg gca cag ggc agc gag gcc cag ctc att tca ttc gac tga              2409
Pro Ala Gln Gly Ser Glu Ala Gln Leu Ile Ser Phe Asp
765                 770                 775

cccaggccat gctcacgtcc ggagtaacac tacatacagt tcacctgaaa cgcctcgtct    2469

ctaactgccg tcgtcctgcc tccctgtcct ctactgccgg tagtgtccct tctctgcgag    2529

tgaggggggg ccttcacccc aagcccacct cccttgtcct cagcctactg cagtccctga    2589

gttagtctct gctttctttc cccagggctg ggccatgggg agggaaggac tttctcccag    2649

gggaagcccc cagccctgtg ggtcatggtc tgtgagaggt ggcaggaatg gggaccctca     2709

ccccccaagc agcctgtgcc ctctggccgc actgtgagct ggctgtggtg tctgggtgtg    2769

gcctggggct ccctctgcag gggcctctct cggcagccac agccaagggt ggaggcttca     2829

ggtctccagc ttctctgctt ctcagctgcc atctccagtg ccccagaatg gtacagcgat    2889

aataaaatgt atttcag                                                   2906

<210> 4
<211> 777
<212> PRT
<213> Homo sapiens

<400> 4

Met Gly Arg Gly Ser Gly Thr Phe Glu Arg Leu Leu Asp Lys Ala Thr
1               5                   10                  15

Ser Gln Leu Leu Leu Glu Thr Asp Trp Glu Ser Ile Leu Gln Ile Cys
            20                  25                  30

Asp Leu Ile Arg Gln Gly Asp Thr Gln Ala Lys Tyr Ala Val Asn Ser
        35                  40                  45

Ile Lys Lys Lys Val Asn Asp Lys Asn Pro His Val Ala Leu Tyr Ala
    50                  55                  60

Leu Glu Val Met Glu Ser Val Val Lys Asn Cys Gly Gln Thr Val His
65                  70                  75                  80

Asp Glu Val Ala Asn Lys Gln Thr Met Glu Glu Leu Lys Asp Leu Leu
                85                  90                  95

Lys Arg Gln Val Glu Val Asn Val Arg Asn Lys Ile Leu Tyr Leu Ile
            100                 105                 110

Gln Ala Trp Ala His Ala Phe Arg Asn Glu Pro Lys Tyr Lys Val Val
        115                 120                 125

Gln Asp Thr Tyr Gln Ile Met Lys Val Glu Gly His Val Phe Pro Glu
    130                 135                 140

Phe Lys Glu Ser Asp Ala Met Phe Ala Ala Glu Arg Ala Pro Asp Trp
145                 150                 155                 160

Val Asp Ala Glu Glu Cys His Arg Cys Arg Val Gln Phe Gly Val Met
                165                 170                 175

Thr Arg Lys His His Cys Arg Ala Cys Gly Gln Ile Phe Cys Gly Lys
            180                 185                 190

Cys Ser Ser Lys Tyr Ser Thr Ile Pro Lys Phe Gly Ile Glu Lys Glu
        195                 200                 205

Val Arg Val Cys Glu Pro Cys Tyr Glu Gln Leu Asn Arg Lys Ala Glu
    210                 215                 220

Gly Lys Ala Thr Ser Thr Thr Glu Leu Pro Pro Glu Tyr Leu Thr Ser

225                 230                 235                 240

Pro Leu Ser Gln Gln Ser Gln Leu Pro Pro Lys Arg Asp Glu Thr Ala
                245                 250                 255

Leu Gln Glu Glu Glu Glu Leu Gln Leu Ala Leu Ala Leu Ser Gln Ser
            260                 265                 270

Glu Ala Glu Glu Lys Glu Arg Leu Arg Gln Lys Ser Thr Tyr Thr Ser
        275                 280                 285

Tyr Pro Lys Ala Glu Pro Met Pro Ser Ala Ser Ser Ala Pro Pro Ala
    290                 295                 300

Ser Ser Leu Tyr Ser Ser Pro Val Asn Ser Ser Ala Pro Leu Ala Glu
305                 310                 315                 320

Asp Ile Asp Pro Glu Leu Ala Arg Tyr Leu Asn Arg Asn Tyr Trp Glu
                325                 330                 335

Lys Lys Gln Glu Glu Ala Arg Lys Ser Pro Thr Pro Ser Ala Pro Val
            340                 345                 350

Pro Leu Thr Glu Pro Ala Ala Gln Pro Gly Glu Gly His Ala Ala Pro
        355                 360                 365

Thr Asn Val Val Glu Asn Pro Leu Pro Glu Thr Asp Ser Gln Pro Ile
    370                 375                 380

Pro Pro Ser Gly Gly Pro Phe Ser Glu Pro Gln Phe His Asn Gly Glu
385                 390                 395                 400

Ser Glu Glu Ser His Glu Gln Phe Leu Lys Ala Leu Gln Asn Ala Val
                405                 410                 415

Thr Thr Phe Val Asn Arg Met Lys Ser Asn His Met Arg Gly Arg Ser
            420                 425                 430

Ile Thr Asn Asp Ser Ala Val Leu Ser Leu Phe Gln Ser Ile Asn Gly
        435                 440                 445

Met His Pro Gln Leu Leu Glu Leu Leu Asn Gln Leu Asp Glu Arg Arg
    450                 455                 460

Leu Tyr Tyr Glu Gly Leu Gln Asp Lys Leu Ala Gln Ile Arg Asp Ala
465                 470                 475                 480

Arg Gly Ala Leu Ser Ala Leu Arg Glu Glu His Arg Glu Lys Leu Arg
                485                 490                 495

Arg Ala Ala Glu Glu Ala Glu Arg Gln Arg Gln Ile Gln Leu Ala Gln
            500                 505                 510

Lys Leu Glu Ile Met Arg Gln Lys Lys Gln Glu Tyr Leu Glu Val Gln
        515                 520                 525

Arg Gln Leu Ala Ile Gln Arg Leu Gln Glu Gln Glu Lys Glu Arg Gln
    530                 535                 540

Met Arg Leu Glu Gln Gln Lys Gln Thr Val Gln Met Arg Ala Gln Met
545                 550                 555                 560

Pro Ala Phe Pro Leu Pro Tyr Ala Gln Leu Gln Ala Met Pro Ala Ala
                565                 570                 575

Gly Gly Val Leu Tyr Gln Pro Ser Gly Pro Ala Ser Phe Pro Ser Thr
            580                 585                 590

Phe Ser Pro Ala Gly Ser Val Glu Gly Ser Pro Met His Gly Val Tyr
        595                 600                 605

Met Ser Gln Pro Ala Pro Ala Ala Gly Pro Tyr Pro Ser Met Pro Ser
    610                 615                 620

Thr Ala Ala Asp Pro Ser Met Val Ser Ala Tyr Met Tyr Pro Ala Gly
625                 630                 635                 640

Ala Thr Gly Ala Gln Ala Ala Pro Gln Ala Gln Ala Gly Pro Thr Ala
                645                 650                 655

Ser Pro Ala Tyr Ser Ser Tyr Gln Pro Thr Pro Thr Ala Gly Tyr Gln
            660                 665                 670

Asn Val Ala Ser Gln Ala Pro Gln Ser Leu Pro Ala Ile Ser Gln Pro
        675                 680                 685

Pro Gln Ser Ser Thr Met Gly Tyr Met Gly Ser Gln Ser Val Ser Met
    690                 695                 700

Gly Tyr Gln Pro Tyr Asn Met Gln Asn Leu Met Thr Thr Leu Pro Ser
705                 710                 715                 720

Gln Asp Ala Ser Leu Pro Pro Gln Gln Pro Tyr Ile Ala Gly Gln Gln
                725                 730                 735

Pro Met Tyr Gln Gln Met Ala Pro Ser Gly Gly Pro Pro Gln Gln Gln
            740                 745                 750

Pro Pro Val Ala Gln Gln Pro Gln Ala Gln Gly Pro Pro Ala Gln Gly
        755                 760                 765

Ser Glu Ala Gln Leu Ile Ser Phe Asp
    770                 775

<210>  5
<211>  2458
<212>  DNA
<213>  Mus musculus

<220>
<221>  CDS

<222>  (43)..(2370)

<400>  5
aaagagggag cgactgcagc gtcggtccgg agtggggtcg cc atg ggg cga ggc      54
                                               Met Gly Arg Gly
                                               1

agc ggc acc ttc gag cgt ctc cta gac aaa gcc acc agc cag ctt ctg      102
Ser Gly Thr Phe Glu Arg Leu Leu Asp Lys Ala Thr Ser Gln Leu Leu
5                   10                  15                  20

ttg gag aca gac tgg gag tcc att cta cag atc tgc gac ctg atc cgt      150
Leu Glu Thr Asp Trp Glu Ser Ile Leu Gln Ile Cys Asp Leu Ile Arg
                25                  30                  35

cag ggg gac aca caa gca aaa tat gct gta aac tcc atc aag aag aag      198
Gln Gly Asp Thr Gln Ala Lys Tyr Ala Val Asn Ser Ile Lys Lys Lys
            40                  45                  50

gtt aat gat aag aac cca cac gtg gct ttg tat gct ctg gag gtg atg      246
Val Asn Asp Lys Asn Pro His Val Ala Leu Tyr Ala Leu Glu Val Met
        55                  60                  65

gag tct gtg gta aag aac tgt ggc cag aca gtc cat gat gaa gtg gcc      294
Glu Ser Val Val Lys Asn Cys Gly Gln Thr Val His Asp Glu Val Ala
    70                  75                  80

aac aaa cag acc atg gaa gaa ctg aag gag ctg ctg aag agg caa gtg      342
Asn Lys Gln Thr Met Glu Glu Leu Lys Glu Leu Leu Lys Arg Gln Val
85                  90                  95                  100

gaa gtt aat gtt cgg aac aag atc ttg tac ctg atc cag gcc tgg gca      390
Glu Val Asn Val Arg Asn Lys Ile Leu Tyr Leu Ile Gln Ala Trp Ala
                105                 110                 115

cat gcc ttc cgg aac gaa ccc aag tac aag gtg gtc cag gac aca tac      438
His Ala Phe Arg Asn Glu Pro Lys Tyr Lys Val Val Gln Asp Thr Tyr
            120                 125                 130

cag atc atg aag gta gaa gga cat gtc ttc cct gaa ttt aag gaa agt      486
Gln Ile Met Lys Val Glu Gly His Val Phe Pro Glu Phe Lys Glu Ser
        135                 140                 145

gat gcc atg ttc gct gct gaa aga gcc cct gac tgg gtg gat gct gag      534
Asp Ala Met Phe Ala Ala Glu Arg Ala Pro Asp Trp Val Asp Ala Glu
    150                 155                 160

gaa tgc cat cgg tgc aga gta cag ttt ggg gtg gtg acc cgc aag cat      582
Glu Cys His Arg Cys Arg Val Gln Phe Gly Val Val Thr Arg Lys His

165                 170                 175                 180

cac tgc cga gca tgt ggg cag atc ttc tgt ggc aag tgc tcc tcc aag          630
His Cys Arg Ala Cys Gly Gln Ile Phe Cys Gly Lys Cys Ser Ser Lys
                185                 190                 195

tac tcc acc atc ccc aag ttc ggc att gag aag gag gtg cgc gtg tgt          678
Tyr Ser Thr Ile Pro Lys Phe Gly Ile Glu Lys Glu Val Arg Val Cys
            200                 205                 210

gag ccc tgc tat gag cag ctg aac aag aag gca gaa ggg aag gct tcc          726
Glu Pro Cys Tyr Glu Gln Leu Asn Lys Lys Ala Glu Gly Lys Ala Ser
        215                 220                 225

tct acc act gag ctg ccc cca gag tac ctg acc agc ccc ctg tca cag          774
Ser Thr Thr Glu Leu Pro Pro Glu Tyr Leu Thr Ser Pro Leu Ser Gln
    230                 235                 240

cag tct cag ctg ccc cca aag cgg gat gag aca gcc ctg cag gaa gag          822
Gln Ser Gln Leu Pro Pro Lys Arg Asp Glu Thr Ala Leu Gln Glu Glu
245                 250                 255                 260

gag gag cta cag ctg gct ctg gcc cta tca cag tca gag gct gag gag          870
Glu Glu Leu Gln Leu Ala Leu Ala Leu Ser Gln Ser Glu Ala Glu Glu
                265                 270                 275

aaa gag agg atg aga cag aaa aca aca tat aca gca cat cca aag gca          918
Lys Glu Arg Met Arg Gln Lys Thr Thr Tyr Thr Ala His Pro Lys Ala
            280                 285                 290

gag ccc acg ccc ttg gct tcc tct gcg ccc cca gct ggc agc ctg tat          966
Glu Pro Thr Pro Leu Ala Ser Ser Ala Pro Pro Ala Gly Ser Leu Tyr
        295                 300                 305

tcc tcg cct gtg aac tca tca gca cct ctg gct gag gac atc gac cct          1014
Ser Ser Pro Val Asn Ser Ser Ala Pro Leu Ala Glu Asp Ile Asp Pro
    310                 315                 320

gag ctt gca aga tac ctc aac cgg aac tac tgg gag aag aaa cag gag          1062
Glu Leu Ala Arg Tyr Leu Asn Arg Asn Tyr Trp Glu Lys Lys Gln Glu
325                 330                 335                 340

gaa gca cgg aag agc ccc aca cca tct gca cct gtg ccc ctg aca gag          1110
Glu Ala Arg Lys Ser Pro Thr Pro Ser Ala Pro Val Pro Leu Thr Glu
                345                 350                 355

cct gct gcc cag cct gga gaa gga cat aca gcc ccc aac agc atg gca          1158
Pro Ala Ala Gln Pro Gly Glu Gly His Thr Ala Pro Asn Ser Met Ala
            360                 365                 370

gag gct cct ctt cca gag aca gac tct cag ccc ata act ccc tgc agc 1206
Glu Ala Pro Leu Pro Glu Thr Asp Ser Gln Pro Ile Thr Pro Cys Ser
        375                 380                 385

ggc ccc ttt agt gag tac cag aat ggg gag tcg gag gag agc cat gag 1254
Gly Pro Phe Ser Glu Tyr Gln Asn Gly Glu Ser Glu Glu Ser His Glu
    390                 395                 400

cag ttc ctc aag gcc ctg cag aat gcc gtg agc act ttt gtc aac cgc 1302
Gln Phe Leu Lys Ala Leu Gln Asn Ala Val Ser Thr Phe Val Asn Arg
405                 410                 415                 420

atg aag agc aac cac atg cgg gga cgc agc atc acc aac gac tcg gct 1350
Met Lys Ser Asn His Met Arg Gly Arg Ser Ile Thr Asn Asp Ser Ala
                425                 430                 435

gtg ctg tca ctc ttc cag tcc atc aac acc atg cac ccg cag ctg ctc 1398
Val Leu Ser Leu Phe Gln Ser Ile Asn Thr Met His Pro Gln Leu Leu
            440                 445                 450

gag ctg ctc aac cag ctg gat gag cgc agg ctg tac tac gag ggc ctt 1446
Glu Leu Leu Asn Gln Leu Asp Glu Arg Arg Leu Tyr Tyr Glu Gly Leu
        455                 460                 465

cag gac aag ctg gca cag ata cgt gac gcc cga ggg gct ctg agc gcc 1494
Gln Asp Lys Leu Ala Gln Ile Arg Asp Ala Arg Gly Ala Leu Ser Ala
    470                 475                 480

ctg cgt gaa gaa cac agg gag aag ctg cgc cgg gca gct gag gag gct 1542
Leu Arg Glu Glu His Arg Glu Lys Leu Arg Arg Ala Ala Glu Glu Ala
485                 490                 495                 500

gag cgt caa cgc cag atc cag ctg gca cag aaa ctg gag atc atg aga 1590
Glu Arg Gln Arg Gln Ile Gln Leu Ala Gln Lys Leu Glu Ile Met Arg
                505                 510                 515

cag aag aag cag gag tac ctg gag gtg cag aga cag cta gct atc cag 1638
Gln Lys Lys Gln Glu Tyr Leu Glu Val Gln Arg Gln Leu Ala Ile Gln
            520                 525                 530

cgc ctg cag gaa cag gag aag gaa cgg cag atg cgt ctg gag caa cag 1686
Arg Leu Gln Glu Gln Glu Lys Glu Arg Gln Met Arg Leu Glu Gln Gln
        535                 540                 545

aag cag act gtc cag atg cgc gcc cag atg cct gcc ttt ccc ttg cct 1734
Lys Gln Thr Val Gln Met Arg Ala Gln Met Pro Ala Phe Pro Leu Pro
    550                 555                 560

tat gcc cag ctc cag gct atg ccc acg gct ggg ggt gta ctc tac cag 1782
Tyr Ala Gln Leu Gln Ala Met Pro Thr Ala Gly Gly Val Leu Tyr Gln
565                 570                 575                 580

ccc tca ggc cca acc agc ttc cct gcc acc ttc agc cca gca ggc tca 1830
Pro Ser Gly Pro Thr Ser Phe Pro Ala Thr Phe Ser Pro Ala Gly Ser
                585                 590                 595

gta gag ggc tct ccg atg cat ggt gtg tat atg agc cag cca gcc cca 1878
Val Glu Gly Ser Pro Met His Gly Val Tyr Met Ser Gln Pro Ala Pro
            600                 605                 610

gcc act ggc ccc tac ccc agc atg cct ggc aca aca gca gat ccc agc 1926
Ala Thr Gly Pro Tyr Pro Ser Met Pro Gly Thr Thr Ala Asp Pro Ser
        615                 620                 625

atg gtc agc gcc tac atg tac cca aca ggt gcc cct ggg gca cag gca 1974
Met Val Ser Ala Tyr Met Tyr Pro Thr Gly Ala Pro Gly Ala Gln Ala
    630                 635                 640

gcc cct cag gcc cag gcc ggg ccc acc acc agt cct gcc tac tcc tcc 2022
Ala Pro Gln Ala Gln Ala Gly Pro Thr Thr Ser Pro Ala Tyr Ser Ser
645                 650                 655                 660

tac cag ccc aca cca acc cca ggc tac cag agc gtg gct tct cag gcc 2070
Tyr Gln Pro Thr Pro Thr Pro Gly Tyr Gln Ser Val Ala Ser Gln Ala
                665                 670                 675

cca cag agc ctc cca gcc atc tca cag cct cca cag acc agc aac ata 2118
Pro Gln Ser Leu Pro Ala Ile Ser Gln Pro Pro Gln Thr Ser Asn Ile
            680                 685                 690

ggc tac atg ggg agc cag cca atg tcc atg ggc tac cag ccg tac aat 2166
Gly Tyr Met Gly Ser Gln Pro Met Ser Met Gly Tyr Gln Pro Tyr Asn
        695                 700                 705

atg cag aat ctc atg acc gcc ctt ccc ggg cag gat gcg tct ctg cca 2214
Met Gln Asn Leu Met Thr Ala Leu Pro Gly Gln Asp Ala Ser Leu Pro
    710                 715                 720

gcc cag cag ccc tac atc cca ggg cag cag ccc ctg tac cag cag atg 2262
Ala Gln Gln Pro Tyr Ile Pro Gly Gln Gln Pro Leu Tyr Gln Gln Met
725                 730                 735                 740

gcc ccc agc acc ggc cct ccc cag cag caa ccc cct gtg gcc cag cca 2310
Ala Pro Ser Thr Gly Pro Pro Gln Gln Gln Pro Pro Val Ala Gln Pro
                745                 750                 755

gcg cct aca cag gga ccg cca gca cag ggc agt gag gcc cag ctc atc 2358

Ala Pro Thr Gln Gly Pro Pro Ala Gln Gly Ser Glu Ala Gln Leu Ile
        760                 765                 770

tcc ttt gac tga ccttgagtct ggcgctcacc atgcagagta acactacagt        2410
Ser Phe Asp
    775

tcaccagaaa ccacttatat gtctaactag ccattcctcg cggccgct               2458

<210> 6
<211> 775
<212> PRT
<213> Mus musculus

<400> 6

Met Gly Arg Gly Ser Gly Thr Phe Glu Arg Leu Leu Asp Lys Ala Thr
1               5                   10                  15

Ser Gln Leu Leu Leu Glu Thr Asp Trp Glu Ser Ile Leu Gln Ile Cys
            20                  25                  30

Asp Leu Ile Arg Gln Gly Asp Thr Gln Ala Lys Tyr Ala Val Asn Ser
        35                  40                  45

Ile Lys Lys Lys Val Asn Asp Lys Asn Pro His Val Ala Leu Tyr Ala
    50                  55                  60

Leu Glu Val Met Glu Ser Val Val Lys Asn Cys Gly Gln Thr Val His
65                  70                  75                  80

Asp Glu Val Ala Asn Lys Gln Thr Met Glu Glu Leu Lys Glu Leu Leu
                85                  90                  95

Lys Arg Gln Val Glu Val Asn Val Arg Asn Lys Ile Leu Tyr Leu Ile
            100                 105                 110

Gln Ala Trp Ala His Ala Phe Arg Asn Glu Pro Lys Tyr Lys Val Val
        115                 120                 125

Gln Asp Thr Tyr Gln Ile Met Lys Val Glu Gly His Val Phe Pro Glu
    130                 135                 140

Phe Lys Glu Ser Asp Ala Met Phe Ala Ala Glu Arg Ala Pro Asp Trp
145                 150                 155                 160

Val Asp Ala Glu Glu Cys His Arg Cys Arg Val Gln Phe Gly Val Val
                165                 170                 175

Thr Arg Lys His His Cys Arg Ala Cys Gly Gln Ile Phe Cys Gly Lys
            180                 185                 190

Cys Ser Ser Lys Tyr Ser Thr Ile Pro Lys Phe Gly Ile Glu Lys Glu
        195                 200                 205

Val Arg Val Cys Glu Pro Cys Tyr Glu Gln Leu Asn Lys Lys Ala Glu
    210                 215                 220

Gly Lys Ala Ser Ser Thr Thr Glu Leu Pro Pro Glu Tyr Leu Thr Ser
225                 230                 235                 240

Pro Leu Ser Gln Gln Ser Gln Leu Pro Pro Lys Arg Asp Glu Thr Ala
                245                 250                 255

Leu Gln Glu Glu Glu Glu Leu Gln Leu Ala Leu Ala Leu Ser Gln Ser
            260                 265                 270

Glu Ala Glu Glu Lys Glu Arg Met Arg Gln Lys Thr Thr Tyr Thr Ala
        275                 280                 285

His Pro Lys Ala Glu Pro Thr Pro Leu Ala Ser Ser Ala Pro Pro Ala
    290                 295                 300

Gly Ser Leu Tyr Ser Ser Pro Val Asn Ser Ser Ala Pro Leu Ala Glu
305                 310                 315                 320

Asp Ile Asp Pro Glu Leu Ala Arg Tyr Leu Asn Arg Asn Tyr Trp Glu

                325                 330                 335

Lys Lys Gln Glu Glu Ala Arg Lys Ser Pro Thr Pro Ser Ala Pro Val
            340                 345                 350

Pro Leu Thr Glu Pro Ala Ala Gln Pro Gly Glu Gly His Thr Ala Pro
        355                 360                 365

Asn Ser Met Ala Glu Ala Pro Leu Pro Glu Thr Asp Ser Gln Pro Ile
    370                 375                 380

Thr Pro Cys Ser Gly Pro Phe Ser Glu Tyr Gln Asn Gly Glu Ser Glu
385                 390                 395                 400

Glu Ser His Glu Gln Phe Leu Lys Ala Leu Gln Asn Ala Val Ser Thr
                405                 410                 415

Phe Val Asn Arg Met Lys Ser Asn His Met Arg Gly Arg Ser Ile Thr
            420                 425                 430

Asn Asp Ser Ala Val Leu Ser Leu Phe Gln Ser Ile Asn Thr Met His
        435                 440                 445

Pro Gln Leu Leu Glu Leu Leu Asn Gln Leu Asp Glu Arg Arg Leu Tyr
    450                 455                 460

Tyr Glu Gly Leu Gln Asp Lys Leu Ala Gln Ile Arg Asp Ala Arg Gly
465                 470                 475                 480

Ala Leu Ser Ala Leu Arg Glu Glu His Arg Glu Lys Leu Arg Arg Ala
                485                 490                 495

Ala Glu Glu Ala Glu Arg Gln Arg Gln Ile Gln Leu Ala Gln Lys Leu
            500                 505                 510

Glu Ile Met Arg Gln Lys Lys Gln Glu Tyr Leu Glu Val Gln Arg Gln
        515                 520                 525

Leu Ala Ile Gln Arg Leu Gln Glu Gln Glu Lys Glu Arg Gln Met Arg
    530                 535                 540

Leu Glu Gln Gln Lys Gln Thr Val Gln Met Arg Ala Gln Met Pro Ala
545                 550                 555                 560

Phe Pro Leu Pro Tyr Ala Gln Leu Gln Ala Met Pro Thr Ala Gly Gly
                565                 570                 575

Val Leu Tyr Gln Pro Ser Gly Pro Thr Ser Phe Pro Ala Thr Phe Ser
            580                 585                 590

Pro Ala Gly Ser Val Glu Gly Ser Pro Met His Gly Val Tyr Met Ser
        595                 600                 605

Gln Pro Ala Pro Ala Thr Gly Pro Tyr Pro Ser Met Pro Gly Thr Thr
    610                 615                 620

Ala Asp Pro Ser Met Val Ser Ala Tyr Met Tyr Pro Thr Gly Ala Pro
625                 630                 635                 640

Gly Ala Gln Ala Ala Pro Gln Ala Gln Ala Gly Pro Thr Thr Ser Pro
                645                 650                 655

Ala Tyr Ser Ser Tyr Gln Pro Thr Pro Thr Pro Gly Tyr Gln Ser Val
            660                 665                 670

Ala Ser Gln Ala Pro Gln Ser Leu Pro Ala Ile Ser Gln Pro Pro Gln
        675                 680                 685

Thr Ser Asn Ile Gly Tyr Met Gly Ser Gln Pro Met Ser Met Gly Tyr
    690                 695                 700

Gln Pro Tyr Asn Met Gln Asn Leu Met Thr Ala Leu Pro Gly Gln Asp
705                 710                 715                 720

Ala Ser Leu Pro Ala Gln Gln Pro Tyr Ile Pro Gly Gln Gln Pro Leu
                725                 730                 735

Tyr Gln Gln Met Ala Pro Ser Thr Gly Pro Pro Gln Gln Gln Pro Pro
            740                 745                 750

Val Ala Gln Pro Ala Pro Thr Gln Gly Pro Pro Ala Gln Gly Ser Glu
        755                 760                 765

Ala Gln Leu Ile Ser Phe Asp
    770                 775

<210> 7
<211> 522
<212> DNA
<213> Rattus norvegicus

<220>
<221> CDS
<222> (1)..(522)

<400> 7
ggc ccc ttt agt gag cag tac cag aac gcg gag tcg gag gag agc cac 48
Gly Pro Phe Ser Glu Gln Tyr Gln Asn Ala Glu Ser Glu Glu Ser His
1               5                   10                  15

gag cag ttc ctc aag gcc ctg cag aat gca gtc agc act ttt gtc aac 96
Glu Gln Phe Leu Lys Ala Leu Gln Asn Ala Val Ser Thr Phe Val Asn
            20                  25                  30

cgc atg aag agc aac cac atg agg ggc cgc agt atc acc aat gac tcg 144
Arg Met Lys Ser Asn His Met Arg Gly Arg Ser Ile Thr Asn Asp Ser
        35                  40                  45

gct gtg ctg tcc ctc ttc cag tcc atc aat agc aca cac cca cag ctg 192
Ala Val Leu Ser Leu Phe Gln Ser Ile Asn Ser Thr His Pro Gln Leu
    50                  55                  60

ctc gag ctg ctc aac cgg ctg gat gag cgc agg ctg tac tac gag ggg 240
Leu Glu Leu Leu Asn Arg Leu Asp Glu Arg Arg Leu Tyr Tyr Glu Gly
65                  70                  75                  80

ctt cag gac aag ctg gca cag ata cgt gat gcc cga ggg gcc ctg agt 288

Leu Gln Asp Lys Leu Ala Gln Ile Arg Asp Ala Arg Gly Ala Leu Ser
                85                  90                  95

gcc ctg cgt gaa gag cac agg gag aag ctg cgc cgg gca gct gag gag      336
Ala Leu Arg Glu Glu His Arg Glu Lys Leu Arg Arg Ala Ala Glu Glu
            100                 105                 110

gcg gag cgt caa cgt cag atc cag ctg gca cag aag ctg gag atc atg      384
Ala Glu Arg Gln Arg Gln Ile Gln Leu Ala Gln Lys Leu Glu Ile Met
        115                 120                 125

aga caa aag aag cag gag tat ctg gag gtg cag aga cag cta gct atc      432
Arg Gln Lys Lys Gln Glu Tyr Leu Glu Val Gln Arg Gln Leu Ala Ile
    130                 135                 140

cag cgt ctg cag gaa cag gag aag gaa cgg cag atg cgc ctg gag caa      480
Gln Arg Leu Gln Glu Gln Glu Lys Glu Arg Gln Met Arg Leu Glu Gln
145                 150                 155                 160

cag aag cag act gtc cag atg cgt gcc cag atg cct gtc ttc              522
Gln Lys Gln Thr Val Gln Met Arg Ala Gln Met Pro Val Phe
                165                 170

<210>  8
<211>  174
<212>  PRT
<213>  Rattus norvegicus

<400>  8

Gly Pro Phe Ser Glu Gln Tyr Gln Asn Ala Glu Ser Glu Glu Ser His
1               5                   10                  15

Glu Gln Phe Leu Lys Ala Leu Gln Asn Ala Val Ser Thr Phe Val Asn
            20                  25                  30

Arg Met Lys Ser Asn His Met Arg Gly Arg Ser Ile Thr Asn Asp Ser
        35                  40                  45

Ala Val Leu Ser Leu Phe Gln Ser Ile Asn Ser Thr His Pro Gln Leu
    50                  55                  60

Leu Glu Leu Leu Asn Arg Leu Asp Glu Arg Arg Leu Tyr Tyr Glu Gly
65                  70                  75                  80

Leu Gln Asp Lys Leu Ala Gln Ile Arg Asp Ala Arg Gly Ala Leu Ser
                85                  90                  95

Ala Leu Arg Glu Glu His Arg Glu Lys Leu Arg Arg Ala Ala Glu Glu
            100                 105                 110

Ala Glu Arg Gln Arg Gln Ile Gln Leu Ala Gln Lys Leu Glu Ile Met
        115                 120                 125

Arg Gln Lys Lys Gln Glu Tyr Leu Glu Val Gln Arg Gln Leu Ala Ile
    130                 135                 140

Gln Arg Leu Gln Glu Gln Glu Lys Glu Arg Gln Met Arg Leu Glu Gln
145                 150                 155                 160

Gln Lys Gln Thr Val Gln Met Arg Ala Gln Met Pro Val Phe
                165                 170

<210>  9
<211>  519
<212>  DNA
<213>  Homo sapiens

<220>
<221>  CDS
<222>  (1)..(519)

<400>  9
ttt agt gag cca cag ttc cac aat ggc gag tct gag gag agc cac gag          48
Phe Ser Glu Pro Gln Phe His Asn Gly Glu Ser Glu Glu Ser His Glu
1               5                   10                  15

cag ttc ctg aag gcg ctg cag aac gcc gtc acc acc ttc gtg aac cgc          96
Gln Phe Leu Lys Ala Leu Gln Asn Ala Val Thr Thr Phe Val Asn Arg
            20                  25                  30

atg aag agt aac cac atg cgg ggc cgc agc atc acc aat gac tcg gcc          144
Met Lys Ser Asn His Met Arg Gly Arg Ser Ile Thr Asn Asp Ser Ala
        35                  40                  45

gtg ctc tca ctc ttc cag tcc atc aac ggc atg cac ccg cag ctg ctg          192
Val Leu Ser Leu Phe Gln Ser Ile Asn Gly Met His Pro Gln Leu Leu
    50                  55                  60

gag ctg ctc aac cag ctg gac gag cgc agg ctg tac tat gag ggg ctg          240
Glu Leu Leu Asn Gln Leu Asp Glu Arg Arg Leu Tyr Tyr Glu Gly Leu
65                  70                  75                  80

cag gac aag ctg gca cag atc cgc gat gcc cgg ggg gcg ctg agt gcc          288
Gln Asp Lys Leu Ala Gln Ile Arg Asp Ala Arg Gly Ala Leu Ser Ala
                85                  90                  95

ctg cgc gaa gag cac cgg gag aag ctt cgc cgg gca gcc gag gag gca          336
Leu Arg Glu Glu His Arg Glu Lys Leu Arg Arg Ala Ala Glu Glu Ala
            100                 105                 110

gag cgc cag cgc cag atc cag ctg gcc cag aag ctg gag ata atg cgg          384
Glu Arg Gln Arg Gln Ile Gln Leu Ala Gln Lys Leu Glu Ile Met Arg
        115                 120                 125

cag aag aag cag gag tac ctg gag gtg cag agg cag ctg gcc atc cag          432
Gln Lys Lys Gln Glu Tyr Leu Glu Val Gln Arg Gln Leu Ala Ile Gln
    130                 135                 140

cgc ctg cag gag cag gag aag gag cgg cag atg cgg ctg gag cag cag          480
Arg Leu Gln Glu Gln Glu Lys Glu Arg Gln Met Arg Leu Glu Gln Gln
145                 150                 155                 160

aag cag acg gtc cag atg cgc gcg cag atg ccc gcc ttc                      519
Lys Gln Thr Val Gln Met Arg Ala Gln Met Pro Ala Phe
                165                 170

<210>  10
<211>  173
<212>  PRT
<213>  Homo sapiens

<400>  10

Phe Ser Glu Pro Gln Phe His Asn Gly Glu Ser Glu Glu Ser His Glu
1               5                   10                  15

Gln Phe Leu Lys Ala Leu Gln Asn Ala Val Thr Thr Phe Val Asn Arg
            20                  25                  30

Met Lys Ser Asn His Met Arg Gly Arg Ser Ile Thr Asn Asp Ser Ala

35                  40                  45

Val Leu Ser Leu Phe Gln Ser Ile Asn Gly Met His Pro Gln Leu Leu
    50                  55                  60

Glu Leu Leu Asn Gln Leu Asp Glu Arg Arg Leu Tyr Tyr Glu Gly Leu
65                  70                  75                  80

Gln Asp Lys Leu Ala Gln Ile Arg Asp Ala Arg Gly Ala Leu Ser Ala
                85                  90                  95

Leu Arg Glu Glu His Arg Glu Lys Leu Arg Arg Ala Ala Glu Glu Ala
            100                 105                 110

Glu Arg Gln Arg Gln Ile Gln Leu Ala Gln Lys Leu Glu Ile Met Arg
        115                 120                 125

Gln Lys Lys Gln Glu Tyr Leu Glu Val Gln Arg Gln Leu Ala Ile Gln
    130                 135                 140

Arg Leu Gln Glu Gln Glu Lys Glu Arg Gln Met Arg Leu Glu Gln Gln
145                 150                 155                 160

Lys Gln Thr Val Gln Met Arg Ala Gln Met Pro Ala Phe
                165                 170

<210>  11
<211>  513
<212>  DNA
<213>  Mus musculus

<220>
<221>  CDS
<222>  (1)..(513)

<400>  11
ggc ccc ttt agt gag tac cag aat ggg gag tcg gag gag agc cat gag      48
Gly Pro Phe Ser Glu Tyr Gln Asn Gly Glu Ser Glu Glu Ser His Glu
1               5                   10                  15

cag ttc ctc aag gcc ctg cag aat gcc gtg agc act ttt gtc aac cgc     96
Gln Phe Leu Lys Ala Leu Gln Asn Ala Val Ser Thr Phe Val Asn Arg
            20                  25                  30

atg aag agc aac cac atg cgg gga cgc agc atc acc aac gac tcg gct     144
Met Lys Ser Asn His Met Arg Gly Arg Ser Ile Thr Asn Asp Ser Ala
        35                  40                  45

gtg ctg tca ctc ttc cag tcc atc aac acc atg cac ccg cag ctg ctc     192
Val Leu Ser Leu Phe Gln Ser Ile Asn Thr Met His Pro Gln Leu Leu
    50                  55                  60

gag ctg ctc aac cag ctg gat gag cgc agg ctg tac tac gag ggc ctt     240
Glu Leu Leu Asn Gln Leu Asp Glu Arg Arg Leu Tyr Tyr Glu Gly Leu
65                  70                  75                  80

cag gac aag ctg gca cag ata cgt gac gcc cga ggg gct ctg agc gcc     288
Gln Asp Lys Leu Ala Gln Ile Arg Asp Ala Arg Gly Ala Leu Ser Ala
                85                  90                  95

ctg cgt gaa gaa cac agg gag aag ctg cgc cgg gca gct gag gag gct     336
Leu Arg Glu Glu His Arg Glu Lys Leu Arg Arg Ala Ala Glu Glu Ala
            100                 105                 110

gag cgt caa cgc cag atc cag ctg gca cag aaa ctg gag atc atg aga     384
Glu Arg Gln Arg Gln Ile Gln Leu Ala Gln Lys Leu Glu Ile Met Arg
        115                 120                 125

cag aag aag cag gag tac ctg gag gtg cag aga cag cta gct atc cag     432
Gln Lys Lys Gln Glu Tyr Leu Glu Val Gln Arg Gln Leu Ala Ile Gln
    130                 135                 140

cgc ctg cag gaa cag gag aag gaa cgg cag atg cgt ctg gag caa cag     480
Arg Leu Gln Glu Gln Glu Lys Glu Arg Gln Met Arg Leu Glu Gln Gln
145                 150                 155                 160

aag cag act gtc cag atg cgc gcc cag atg cct                         513
Lys Gln Thr Val Gln Met Arg Ala Gln Met Pro
                165                 170

<210>  12
<211>  171
<212>  PRT
<213>  Mus musculus

<400>  12

Gly Pro Phe Ser Glu Tyr Gln Asn Gly Glu Ser Glu Glu Ser His Glu
1               5                   10                  15

Gln Phe Leu Lys Ala Leu Gln Asn Ala Val Ser Thr Phe Val Asn Arg
            20                  25                  30

Met Lys Ser Asn His Met Arg Gly Arg Ser Ile Thr Asn Asp Ser Ala
        35                  40                  45

Val Leu Ser Leu Phe Gln Ser Ile Asn Thr Met His Pro Gln Leu Leu
    50                  55                  60

Glu Leu Leu Asn Gln Leu Asp Glu Arg Arg Leu Tyr Tyr Glu Gly Leu
65                  70                  75                  80

Gln Asp Lys Leu Ala Gln Ile Arg Asp Ala Arg Gly Ala Leu Ser Ala
                85                  90                  95

Leu Arg Glu Glu His Arg Glu Lys Leu Arg Arg Ala Ala Glu Glu Ala
            100                 105                 110

Glu Arg Gln Arg Gln Ile Gln Leu Ala Gln Lys Leu Glu Ile Met Arg
        115                 120                 125

Gln Lys Lys Gln Glu Tyr Leu Glu Val Gln Arg Gln Leu Ala Ile Gln
    130                 135                 140

Arg Leu Gln Glu Gln Glu Lys Glu Arg Gln Met Arg Leu Glu Gln Gln
145                 150                 155                 160

Lys Gln Thr Val Gln Met Arg Ala Gln Met Pro
                165                 170

<210>  13
<211>  56
<212>  DNA
<213>  Artificial

<220>
<223>  synthetic nucleotide

<400>  13
ttgaattcat ggactacaag gacgacgatg acaagatggg gcgaggcagc ggcacc          56

<210>  14
<211>  56
<212>  DNA
<213>  Artificial

<220>
<223>  synthetic nucleotide

<400>  14
ttgaattcat ggactacaag gacgacgatg acaagccccc agagtacctg accagc          56

<210>  15
<211>  56
<212>  DNA
<213>  Artificial

<220>
<223>  synthetic nucleotide

<400>  15
ttgaattcat ggactacaag gacgacgatg acaagtttag tgagcagtac cagaac          56

<210>  16
<211>  56
<212>  DNA
<213>  Artificial

<220>
<223>  synthetic nucleotide

<400>  16
ttgaattcat ggactacaag gacgacgatg acaagccctt gccttatgcc cagctc          56

<210>  17
<211>  37
<212>  DNA
<213>  Artificial

<220>

<223>  synthetic nucleotide

<400>  17
atagtttatg cggccgctaa gtggtagagg cagcttt                                37

<210>  18
<211>  38
<212>  DNA
<213>  Artificial

<220>
<223>  synthetic nucleotide

<400>  18
atagtttatg cggccgctca ggaagttatg ggctgaga                               38

<210>  19
<211>  39
<212>  DNA
<213>  Artificial

<220>
<223>  synthetic nucleotide

<400>  19
atagtttatg cggccgctag gcacgcatct ggacagtct                              39

<210>  20
<211>  41
<212>  DNA
<213>  Artificial

<220>
<223>  synthetic nucleotide

<400>  20
atagtttatg cggccgctca gtcgaaggag atgagctggg t                           41

<210>  21
<211>  31
<212>  DNA
<213>  Artificial

<220>
<223>  synthetic nucleotide

<400> 21
atatagatct ccgggagtcc attctacaga t 31

<210> 22
<211> 26
<212> DNA
<213> Artificial

<220>
<223> synthetic nucleotide

<400> 22
ggtacctcga gtaaaaaggg agtcca 26

<210> 23
<211> 55
<212> DNA
<213> Artificial

<220>
<223> synthetic nucleotide

<400> 23
ccgggagtcc attctacaga tttcaagaga atctgtagaa tggactccct tttta 55

<210> 24
<211> 28
<212> DNA
<213> Artificial

<220>
<223> synthetic nucleotide

<400> 24
atatagatct ccgttaatga taagaacc 28

<210> 25
<211> 28
<212> DNA
<213> Artificial

<220>
<223> synthetic nucleotide

<400> 25
ggtacctcga gcttaaaaag ttaatgat                                          28


<210> 26
<211> 55
<212> DNA
<213> Artificial

<220>
<223> synthetic nucleotide

<400> 26
ccgttaatga taagaaccca cttcaagaga gtgggttctt atcattaact tttta            55


<210> 27
<211> 29
<212> DNA
<213> Artificial

<220>
<223> synthetic nucleotide

<400> 27
atatagatct ccgtctgtgg taaagaact                                         29


<210> 28
<211> 28
<212> DNA
<213> Artificial

<220>
<223> synthetic nucleotide

<400> 28
ggtacctcga gcttaaaaag tctgtggt                                          28


<210> 29
<211> 55
<212> DNA
<213> Artificial

<220>
<223> synthetic nucleotide

<400> 29

ccgtctgtgg taaagaactg tttcaagaga acagttcttt accacagact tttta 55

<210> 30
<211> 25
<212> RNA
<213> Artificial

<220>
<223> synthetic nucleotide

<400> 30
accuccacgu cucugucgau cgaua 25

<210> 31
<211> 25
<212> RNA
<213> Artificial

<220>
<223> synthetic nucleotide

<400> 31
uaucgaucga cagagacgug gaggu 25

<210> 32
<211> 25
<212> RNA
<213> Artificial

<220>
<223> synthetic nucleotide

<400> 32
ggacgauacu cgucgacuug uucuu 25

<210> 33
<211> 25
<212> RNA
<213> Artificial

<220>
<223> synthetic nucleotide

<400> 33
aagaacaagu cgacgaguau cgucc 25

<210> 34
<211> 25
<212> RNA
<213> Artificial

<220>
<223> synthetic nucleotide

<400> 34
uagaauggac ucccagucug acucc 25


<210> 35
<211> 25
<212> RNA
<213> Artificial

<220>
<223> synthetic nucleotide

<400> 35
ggagucagac ugggagucca uucua 25


<210> 36
<211> 25
<212> RNA
<213> Artificial

<220>
<223> synthetic nucleotide

<400> 36
uagcaauagg cacuccgacu guucc 25


<210> 37
<211> 25
<212> RNA
<213> Artificial

<220>
<223> synthetic nucleotide

<400> 37
ggaacagucg gagugccuau ugcua 25

<210>  38
<211>  25
<212>  RNA
<213>  Artificial

<220>
<223>  synthetic nucleotide

<400>  38
aacagaagcu ggcugguggc uuugu                                          25


<210>  39
<211>  25
<212>  RNA
<213>  Artificial

<220>
<223>  synthetic nucleotide

<400>  39
acaaagccac cagccagcuu cuguu                                          25


<210>  40
<211>  25
<212>  RNA
<213>  Artificial

<220>
<223>  synthetic nucleotide

<400>  40
aucauggacu gucuggccac aguuc                                          25


<210>  41
<211>  25
<212>  RNA
<213>  Artificial

<220>
<223>  synthetic nucleotide

<400>  41
gaacuguggc cagacagucc augau                                          25

<210> 42
<211> 25
<212> RNA
<213> Artificial

<220>
<223> synthetic nucleotide

<400> 42
uuucuucucc caguaguucc gguug 25


<210> 43
<211> 25
<212> RNA
<213> Artificial

<220>
<223> synthetic nucleotide

<400> 43
caaccggaac uacugggaga agaaa 25


<210> 44
<211> 20
<212> PRT
<213> Artificial

<220>
<223> synthetic peptide

<400> 44

Met Lys Ser Asn His Met Arg Gly Arg Ser Ile Thr Asn Asp Ser Ala
1               5                   10                  15


Val Leu Ser Leu
            20


<210> 45
<211> 20
<212> PRT
<213> Artificial

<220>
<223> synthetic peptide

<400>  45

Ile Thr Asn Asp Ser Ala Val Leu Ser Leu Phe Gln Ser Ile Asn Thr
1               5                   10                  15

Met His Pro Gln
            20

<210>  46
<211>  20
<212>  PRT
<213>  Artificial

<220>
<223>  synthetic peptide

<400>  46

Phe Gln Ser Ile Asn Thr Met His Pro Gln Leu Leu Glu Leu Leu Asn
1               5                   10                  15

Gln Leu Asp Glu
            20

<210>  47
<211>  20
<212>  PRT
<213>  Artificial

<220>
<223>  synthetic peptide

<400>  47

Leu Leu Glu Leu Leu Asn Gln Leu Asp Glu Arg Arg Leu Tyr Tyr Glu
1               5                   10                  15

Gly Leu Gln Asp
            20

<210>  48
<211>  20

<212>  PRT
<213>  Artificial

<220>
<223>  synthetic peptide

<400>  48

Arg Arg Leu Tyr Tyr Glu Gly Leu Gln Asp Lys Leu Ala Gln Ile Arg
1               5                   10                  15

Asp Ala Arg Gly
            20

<210>  49
<211>  20
<212>  PRT
<213>  Artificial

<220>
<223>  synthetic peptide

<400>  49

Lys Leu Ala Gln Ile Arg Asp Ala Arg Gly Ala Leu Ser Ala Leu Arg
1               5                   10                  15

Glu Glu His Arg
            20

<210>  50
<211>  20
<212>  PRT
<213>  Artificial

<220>
<223>  synthetic peptide

<400>  50

Ala Leu Ser Ala Leu Arg Glu Glu His Arg Glu Lys Leu Arg Arg Ala
1               5                   10                  15

Ala Glu Glu Ala

            20

<210> 51
<211> 20
<212> PRT
<213> Artificial

<220>
<223> synthetic peptide

<400> 51

Glu Lys Leu Arg Arg Ala Ala Glu Glu Ala Glu Arg Gln Arg Gln Ile
1               5                   10                  15

Gln Leu Ala Gln
            20

<210> 52
<211> 20
<212> PRT
<213> Artificial

<220>
<223> synthetic peptide

<400> 52

Glu Arg Gln Arg Gln Ile Gln Leu Ala Gln Lys Leu Glu Ile Met Arg
1               5                   10                  15

Gln Lys Lys Gln
            20

<210> 53
<211> 20
<212> PRT
<213> Artificial

<220>
<223> synthetic peptide

<400> 53

Lys Leu Glu Ile Met Arg Gln Lys Lys Gln Glu Tyr Leu Glu Val Gln
1               5                   10                  15

Arg Gln Leu Ala
            20

<210>  54
<211>  20
<212>  PRT
<213>  Artificial

<220>
<223>  synthetic peptide

<400>  54

Glu Tyr Leu Glu Val Gln Arg Gln Leu Ala Ile Gln Arg Leu Gln Glu
1               5                   10                  15

Gln Glu Lys Glu
            20

<210>  55
<211>  20
<212>  PRT
<213>  Artificial

<220>
<223>  synthetic peptide

<400>  55

Ile Gln Arg Leu Gln Glu Gln Glu Lys Glu Arg Gln Met Arg Leu Glu
1               5                   10                  15

Gln Gln Lys Gln
            20

<210>  56
<211>  20
<212>  PRT
<213>  Artificial

<220>
<223>  synthetic peptide

<400>  56

Arg Gln Met Arg Leu Glu Gln Gln Lys Gln Thr Val Gln Met Arg Ala
1               5                   10                  15

Gln Met Pro Ala
            20

<210>  57
<211>  10
<212>  PRT
<213>  Artificial

<220>
<223>  synthetic peptide

<400>  57

Met Gly Arg Gly Ser Gly Thr Phe Glu Arg
1               5                   10

<210>  58
<211>  10
<212>  PRT
<213>  Artificial

<220>
<223>  synthetic peptide

<400>  58

Phe Gln Ser Ile Asn Thr Met His Pro Gln
1               5                   10

<210>  59
<211>  10
<212>  PRT
<213>  Artificial

<220>
<223>  synthetic peptide

<400> 59

Lys Leu Ala Gln Ile Arg Asp Ala Arg Gly
1               5                   10

<210> 60
<211> 10
<212> PRT
<213> Artificial

<220>
<223> synthetic peptide

<400> 60

Ala Leu Ser Ala Leu Arg Glu Glu His Arg
1               5                   10

<210> 61
<211> 10
<212> PRT
<213> Artificial

<220>
<223> synthetic peptide

<400> 61

Glu Lys Leu Arg Arg Ala Ala Glu Glu Ala
1               5                   10

<210> 62
<211> 10
<212> PRT
<213> Artificial

<220>
<223> synthetic peptide

<400> 62

Glu Arg Gln Arg Gln Ile Gln Leu Ala Gln
1               5                   10

<210> 63

<211>  10
<212>  PRT
<213>  Artificial

<220>
<223>  synthetic peptide

<400>  63

Lys Leu Glu Ile Met Arg Gln Lys Lys Gln
1               5                   10


<210>  64
<211>  10
<212>  PRT
<213>  Artificial

<220>
<223>  synthetic peptide

<400>  64

Glu Tyr Leu Glu Val Gln Arg Gln Leu Ala
1               5                   10


<210>  65
<211>  10
<212>  PRT
<213>  Artificial

<220>
<223>  synthetic peptide

<400>  65

Ile Gln Arg Leu Gln Glu Gln Glu Lys Glu
1               5                   10


<210>  66
<211>  12
<212>  PRT
<213>  Artificial

<220>
<223>  synthetic peptide

<400>  66

Arg Arg Arg Arg Arg Arg Arg Arg Arg Gly Pro Gly
1               5                   10

<210>  67
<211>  22
<212>  PRT
<213>  Artificial

<220>
<223>  synthetic peptide

<400>  67

Arg Arg Arg Arg Arg Arg Arg Arg Arg Gly Pro Gly Glu Tyr Leu Glu
1               5                   10                  15

Val Gln Arg Gln Leu Ala
            20